(19) Europäisches Patentamt
European Patent Office
Office européen des brevets

(11) **EP 1 126 030 A1**

(12) **EUROPEAN PATENT APPLICATION**
published in accordance with Art. 158(3) EPC

(43) Date of publication:
**22.08.2001 Bulletin 2001/34**

(21) Application number: **99951122.3**

(22) Date of filing: **28.10.1999**

(51) Int Cl.7: **C12N 15/53**, C12N 9/02, C12N 1/21, C12N 1/19, C12N 1/15, C12N 5/10, C07K 16/40, C12Q 1/26, C12Q 1/68, G01N 33/50, G01N 33/15, G01N 33/566

(86) International application number:
**PCT/JP99/05983**

(87) International publication number:
**WO 00/26382 (11.05.2000 Gazette 2000/19)**

(84) Designated Contracting States:
**AT BE CH CY DE DK ES FI FR GB GR IE IT LI LU MC NL PT SE**

(30) Priority: **30.10.1998 JP 31042298**

(71) Applicant: **Medical & Biological Laboratories Co., Ltd.**
**Nagayo-shi, Aichi 460-0002 (JP)**

(72) Inventors:
- **TOJI, Shingo, Med. & Biological Lab. Co., Ltd.**
  **Ina-shi, Nagano 396-0002 (JP)**
- **YANO, Minoru, Med. & Biological Lab. Co., Ltd.**
  **Ina-shi, Nagano 396-0002 (JP)**
- **TAMAI, Katsuyuki,**
  **Med. & Biological Lab. Co., Ltd.**
  **Ina-shi, Nagano 396-0002 (JP)**

(74) Representative: **VOSSIUS & PARTNER**
**Siebertstrasse 4**
**81675 München (DE)**

(54) **THIOREDOXIN REDUCTASE II**

(57) An XIAP-biding protein and cDNA encoding the same were provided. This protein having a thioredoxin reductase activity is named thioredoxin reductase II (TxRII) . It is also clarified that TxRII has subfamilies TxRIIα and TxRIIβ by alternative splicing.

EP 1 126 030 A1

## Description

### Technical Field

The present invention relates to a gene encoding a novel protein having a thioredoxin reductase activity and this protein itself. This protein is likely to closely relate to systems, for example, apoptosis, cancerization, or inflammation and expected to be widely applied to a research material for a therapeutic agent and a diagnostic marker.

### Background Art

It is known that viral infection causes apoptosis in cells in a host. This phenomenon is thought to be one of defense mechanisms for removing infected cells from a living body. Against this, viruses furnish an apoptosis inhibitory system for gaining time to proliferate themselves. Namely, an inhibitor of apoptosis protein (IAP) produced by viruses is one of anti-apoptosis proteins which inhibit apoptosis in a host. The presence of homologues for IAP was found in higher animals as well as in viruses. As a human IAP homologue, HIAP1, HIAP2 and XIAP (X-linked Inhibitor of apoptosis protein) have been reported. Among them, HIAP1 and HIAP 2 were clarified to bind to TRAF2 (TNFR associated factor 2) (Cell 83 (7): 1243-52. 1995., Proc Natl Acad Sci USA 94 (19) : 10057-62. 1997) . On the other hand, any factor binding to XIAP has not been identified yet. In order to analyze functions of XIAP involved in the inhibitory mechanisms of apoptosis in humans, its binding factor is necessary to be identified.

On the other hand, the following has been revealed about thioredoxin reductase (abbreviated to TxR, hereafter). Namely, TxR is involved in DNA transcription mechanism and cancer proliferation through the production of thioredoxin. The following is the reported knowledge.

Thioredoxin reductase; TxR (EC 1.6.4.5) is one of pyridine nucleotide-disulfideoxidoreductase families. This family includes glutathion reductase, lipoamide dehydrogenase, tripanothion reductase, mercury ion reductase, and NADPH peroxidase. These proteins form a dimer, and have a disulfide bond at a redox active center. Flavin adenine dinucleotide (abbreviated to FAD) is used as co-enzyme to reduce a substrate using reduced form nicotine amide adenine dinucleotide phosphate (abbreviated to NADPH) . Thioredoxin reductase oxidizes NADPH to $NADP^+$ and converts oxidized form thioredoxin ($-S_2$) which is a substrate to reduced form thioredoxin $(-SH)_2$ (1). Reduced form thioredoxin reduces a disulfide (S-S) bond in a protein and becomes oxidized form thioredoxin itself (2). Thioredoxin is abbreviated to Trx hereafter.

$$NADPH + H^+ + Trx\text{-}S_2 \rightarrow NADP^+ + Trx\text{-}(SH)_2 \qquad (1)$$

$$Trx\text{-}(SH)_2 + protein\text{-}S_2 \rightarrow Trx\text{-}S_2 + protein\text{-}(SH)_2 \qquad (2)$$

Trx is a redox protein, and plays an important role as an electron donor which creates the reduced state *in vivo*. Trx is an electron donor to an enzyme, for example, ribonucleotide reductase, methionine sulfoxide reductase (Annu. Rev. Biochem 54: 237-71, 1985), vitamin K epoxide reductase (Biochem. Biophys. Res. Commun., 155 (3) : 1248-54, 1988) . Moreover, Trx catalyses a holding in a protein, and determines a DNA binding capacity of a transcription factor. The following substances are known as a transcription factor in which a DNA biding capacity is controlled by Trx. NF-KB (J. Biol. Chem. 268 (15): 11380-8. 1993.) (Nucleic Acids Res. 20 (15): 3821-30. 1992)
TFIIIC
BZLF1 (Oncogene 6 (7): 1243-50. 1991.)
Glucocorticoid
p53

In addition, a transcription factor AP-1 is reduced by Ref-1 to have a DNA binding ability, and this Ref-1 is reduced through Trx.

On the other hand, TxR is getting attention as a target for an anticancer agent. For example, secretory type Trx has been reported to have a cytokine-like function and especially reduced form Trx has been reported to be essential for cell proliferation. It is TxR that produces reduced form Trx. Interestingly, in some kind of cancer, concentration of Trx in blood has been reported to increase and TxR protein has also been reported to increase. It has been reported that insertion of mutation in the Trx redox active center and over expression thereof in oncocytes almost completely inhibited proliferation of oncocytes. From such a background, to terminate proliferation of oncocytes, recently inhibitors of TxR have aggressively been screened. Quinone and nitrosourea, which are anticancer agents, and retinoic acid, which terminates cell proliferation and is a differentiation-inducing agent, have the function of inhibiting TxR.

TxR is a protein containing Se (selenium) which is an essential trace element, as Secys (selenocysteine). Interestingly, Secys is the 21st amino acid which can be translated, and has a unique biosynthetic function by which Secys is encoded by the stop codon UGA. Secys has been also reported to have the radiation protective function and an anticancer effect. As a protein containing Secys, glutathione peroxidase (GPx) which reduces and deletes an active oxygen species hydroperoxide (-OOH) , dependently on glutathione and Trx, and type I tetraiodothyronine deiodinase which converts thyroid hormone (thyroxine) precursor T4 into an active form T3, as well as selenoprotein P comprising 10 Secys and selenoprotein W, low molecular weight Secys-containing protein, present in muscles as the proteins which functions have not been well understood, have been reported. The previously reported human TxR has been reported to encode Secys by an amino acid sequence of Cys-Secys-Gly-stop codon (UAA). Absence of the activity of the most understood bovine TxR by treating with carboxypeptidase Y to remove Secys at C-terminus suggested that this C-terminus Secys is reported to be essential for the activity (Zhong, L., E. S. Arn-er, et al. (1998). Rat and calf thioredoxin reductase are homologous to glutathione reductase with a carboxyl-terminal elongation containing a conserved catalytically active penultimate selenocysteine residue. J. Biol Chem 273 (15): 8581-91.). Revealing the structure in a novel selenoprotein contributes studies in selenoproteins.

## Disclosure of the Invention

An objective of the present invention is to isolate an XIAP-biding protein and a DNA encoding the same. In addition, the present invention aims to isolate a novel protein having a TxR activity derived from human, and a DNA encoding the same.
The present inventors searched for an XIAP-binding protein using the yeast two hybrid system. As a result, a gene encoding an XIAP-binding protein has been successfully isolated from a human placenta cDNA library. A protein encoded by this gene was found to have a TxR activity to complete the present invention. Specifically, the present invention relates to the following proteins, DNAs encoding the same, methods for producing the same, and uses thereof.

(1) A protein comprising the amino acid sequence of SEQ ID NO: 2 or 4.
(2) A protein comprising the amino acid sequence of SEQ ID NO: 2 or 4 in which one or more amino acids are replaced, deleted, added, and/or inserted, having homology of 60% or higher to the amino acid sequence of SEQ ID NO: 2 or 4, and having a thioredoxin reductase activity.
(3) A protein having a thioredoxin reductase activity, encoded by a DNA which hybridizes to the DNA comprising the nucleotide sequence of SEQ ID NO: 1 or 3.
(4) A protein comprising the amino acid sequence of SEQ ID NO: 2 or 4 in which one or more amino acids are replaced, deleted, added, and/or inserted and having an XIAP-binding activity.
(5) A protein encoded by a DNA which hybridizes to the DNA comprising the nucleotide sequence of SEQ ID NO: 1 or 3, and having an XIAP-binding activity.
(6) An antibody biding to the protein of any one of (1) to (5).
(7) A cDNA encoding the protein of any one of (1) to (5).
(8) A cDNA comprising a protein coding region of the nucleotide sequence of SEQ ID NO: 1 or 3.
(9) A vector into which the DNA of (7) or (8) has been inserted.
(10) A transformant carrying the vector of (9).
(11) A method for producing the protein of any one of (1) to (5), the method comprising culturing the transformant of (10).
(12) An antisense DNA against all or a part of the cDNA of (7).
(13) An oligonucleotide comprising a strand of at least 15 nucleotides and hybridizing to the cDNA of (7).
(14) A DNA encoding a protein with a thioredoxin reductase activity and comprising the first exon or the second exon, and the third to the nineteenth exons below:

the first exon, SEQ ID NO: 18;
the second exon, SEQ ID NO: 19;
the third exon, SEQ ID NO: 20;
the forth exon, SEQ ID NO: 21;
the fifth exon, SEQ ID NO: 22;
the sixth exon, SEQ ID NO: 23;
the seventh exon, SEQ ID NO: 24;
the eighth exon, SEQ ID NO: 25;
the ninth exon, SEQ ID NO: 26;
the tenth exon, SEQ ID NO: 27;
the eleventh exon, SEQ ID NO: 28;

the twelfth exon, SEQ ID NO: 29;
the thirteenth exon, SEQ ID NO: 30;
the fourteenth exon, SEQ ID NO: 31;
the fifteenth exon, SEQ ID NO: 32;
the sixteenth exon, SEQ ID NO: 33;
the seventeenth exon, SEQ ID NO: 34;
the eighteenth exon, SEQ ID NO: 35; and
the nineteenth exon, SEQ ID NO: 36.

(15) The DNA of (14), described by SEQ ID NO: 37.
(16) A DNA hybridizing to the nucleotide sequence of any one of SEQ ID NOs: 18 to 36 or a part thereof, which can hybridize to human chromosome 22q11.2.
(17) A DNA which can hybridize to all or a part of a portion of the nucleotide sequence of SEQ ID NO: 37, the portion non-overlapping with the nucleotide sequences of SEQ ID NOs: 18 to 36.
(18) A method for screening a compound having an activity of inhibiting a binding of XIAP with the binding factor, the method comprising the steps of:

(a) contacting simultaneously a candidate substance as a subject for screening, and XIAP with the protein of (2), or
(a)' contacting a candidate substance as a subject for screening with XIAP, and then, further contacting with the protein of (2),
(b) determining the amount of the protein of (2) which binds and/or does not bind to XIAP, and
(c) selecting a compound which inhibits binding of XIAP with the protein of (2).

(19) A method for screening a compound having an activity of promoting or inhibiting an enzyme activity of thioredoxin reductase II, the method comprising the steps of:

(a) contacting a candidate substance as a subject for screening with the protein of any one of (1) to (3),
(b) observing the change in a thioredoxin reductase activity of the protein of any one of (1) to (3) , and
(c) selecting a compound which promotes or inhibits an enzyme activity of thioredoxin reductase II.

SEQ ID NOs : 2 and 4 show amino acid sequences for a novel protein TxRIIα and protein TxRIIβ, respectively, which have been obtained by the present inventors, and SEQ ID NOs: 1 and 3, respectively, show nucleotide sequences of cDNA encoding the same. In the following specification, TxRIIs is used as a term simultaneously containing both TxRIIα and TxRIIβ. These amino acid sequences were deduced based on novel genes structures of which were determined by screening based on a human placenta cDNA library by applying the two hybrid system. The two hybrid method is for confirming interaction among proteins with high sensitivity. The principle is the method for screening a combination of interacting proteins using the expression of a marker gene as an index, as described in Examples. The present inventors applied this method for searching for a substance having an avidity to XIAP to discover a novel factor and reveal the structure.

A location of a gene encoding TxRIIs provided by the present invention was confirmed to be 22q11.2 on chromosomes. Both TxRIIs are present in 70 kbp in this region, by separating into 19 exons. The genes were mapped on chromosomes by database searching, and the presence of genes for proteins having TxR activity in this location was not known at all. TxRIIα and TxRIIβ were determined to be alternative splicing forms of TxRII because they comprised the identical structure in the second and the following exons. Specifically, the first exon of TxRIIα is Exon 1 below (SEQ ID NO: 18), and the first exon of TxRIIβ is Exon 2 below (SEQ ID NO: 19). The second and the following exons of the both, from Exon 3 (SEQ ID NO: 20) to Exon 19 (SEQ ID NO: 36), are identical.

Interestingly, causative genes of, for example, Di George syndrome, and neurofibromatosis, are mapped close to these TxRII genes, and the possibility of involvement of TxRIIs discovered by us in some inherited disease can not be denied. More importantly, the exon 1 of TxRIIα is overlapped with a promoter region of catechol-o-methyltransferase (EC 2.1.1.6, abbreviated to COMT, hereafter) . COMT was also mapped at 22q11.1 11.2 on chromosomes and the direction of transcription was reverse against the TxR II. Namely, it was suggested that, when transcribed, TxR IIα possibly inhibited the expression of COMT by acting on mRNA of COMT in an antisense manner. This may be a cause of schizophrenia and Parkinson's disease. These facts suggest that transcription of COMT can be efficiently inhibited by overexpressing the sense strand DNA of exon 1 in TxRIIα or administering an antisense oligonucleotide or a sense nucleic acid analogue.

Information on the locations of the exons and introns in the genomic DNA provided by the present invention is essential for studying the relationship between these diseases and genetic abnormalities, and may provide a probe for diagnosing these genetic abnormality. Table 1 shows the location for each exon in genome. Each number indicating a location described below is the number when 5' end of the genomic nucleotide sequence in SEQ ID NO: 37 is 1. Based on these information, for example, a DNA hybridizing specifically to each exon can be used as a primer for amplifying an intron part. In contrast, a DNA hybridizing to an intron region except for each exon in the nucleotide sequences of SEQ ID NO: 37 can be used for amplifying an exon by PCR. These primers are essential tools for detecting abnormality in exons and introns. Because inherited diseases may result not only from abnormality in a protein coding region, but also from the abnormality in an intron, leading to the event in which splicing does not occur correctly. Therefore, these kinds of primers are useful for revealing the inherited diseases. In addition, a DNA which can hybridize to an exon is useful as a probe. Especially, a DNA specifically hybridizing to chromosome 22q11.2 among these DNA is useful as a probe for cloning the genomic DNA of SEQ ID NO: 37 by the present invention. Specifically, by screening a human genomic library as a source with these probes, the genomic DNA of SEQ ID NO: 37 can be isolated. In the case of using as a probe or a primer, the oligonucleotide based on the present invention comprises at least 15 nucleotides to achieve hybridization under stringent conditions, preferably of 15 to 200 nucleotides, and more preferably of 25 to 100 nucleotides.

Table 1

| | | The structure of a splicing part | | SEQ ID NO: |
|---|---|---|---|---|
| Exon | Nucleotide No. | 3 side | 5' side | |
| Exon 1 | 1-103 | agcag/GTA | | 18 |
| Exon 2 | 9247-9446 | ccaag/GTG | CAG/caggtc | 19 |
| Exon 3 | 10706-10774 | ggagg/GTA | CAG/ccgccc | 20 |
| Exon 4 | 22205-22261 | ccaag/GTA | CAG/gcacc | 21 |
| Exon 5 | 22800-22944 | gactg/GTA | CAG/gagga | 22 |
| Exon 6 | 23587-23661 | gacag/GTA | CAG/aaaag | 23 |
| Exon 7 | 25961-26039 | aagag/GTG | CAG/attct | 24 |
| Exon 8 | 26529-26591 | cgcac/GTG | CAG/atcga | 25 |
| Exon 9 | 30358-30428 | aaaac/GTA | CAG/gttgg | 26 |
| Exon 10 | 43016-43035 | cagct/GTA | CAG/atgtg | 27 |
| Exon 11 | 43954-44045 | accag/GTA | CAG/caaat | 28 |
| Exon 12 | 46503-46677 | catag/GTA | CAG/gtcga | 29 |
| Exon 13 | 58623-58759 | tggag/GTA | AAG/gggcg | 30 |
| Exon 14 | 61367-61462 | acaat/GTG | CAG/gttct | 31 |
| Exon 15 | 61813-61905 | ttgag/GTG | CAG/gtcta | 32 |
| Exon 16 | 63647-63718 | taaag/GTG | CAG/atggt | 33 |
| Exon 17 | 63897-63994 | atcaa/GTA | CAG/gtgtg | 34 |
| Exon 18 | 64850-65044 | cccag/GTA | CAG/gatgg | 35 |
| Exon 19 | 66277-66566 | - | | 36 |

The amino acid sequence of SEQ ID NO: 2 showed 55% homology to the known human TxR by searching database, and 38% homology to human glutathione reductase. Especially, in a redox active center, a FAD-binding region, a NADPH-binding region, and a selenocysteine active center, homology was completely conserved. Figure 1 shows alignment of amino acid sequences for TxRIIα and the known TxR. The present inventors named the protein comprising the amino acid sequence of SEQ ID NO: 2 TxRIIα, based on these data. Because an avidity with XIAP is not seen in the known human TxR, the protein of the present invention is novel. Homology between these two amino acid sequences does not reach 60%. Therefore, these two are different proteins, and human TxR does not predict structures and functions of TxRIIα or TxRIIβ.

It has been reported that human thioredoxin reductase reported in 1995 contains a sequence of AUUUA in the untranslated region present at 3' side (abbreviated to 3' UTR hereafter). This AUUUA is considered to contribute instability of mRNA and it has been reported that mRNA is rapidly decomposed by the presence of this sequence in 3' UTR. This kind of sequences has been also reported in cytokines and protooncogenes, and it has been known that these proteins increase at once by a stimulus and disappear. These facts suggest that the previously reported human thioredoxin reductase is transiently transcribed and translated by some stimulus and decomposed immediately after that, and that, thus, the effect is limited to a very temporary one. In contrast, this kind of sequences is not present in 3' UTR of TxRIIs of the present invention, and TxRIIs are considered to be constantly involved in controlling redox *in*

*vivo*. Therefore, inhibitors and promoters for TxRIIs are likely to be completely different from the reported inhibitors of TxR in terms of specificity, inhibitory effects, and as therapeutic agents. Therefore, the knowledge regarding TxRIIs, revealed by the present invention, has an important meaning in the development of drugs involved in redox control *in vivo*.

The proteins of the present invention contain not only those disclosed in SEQ ID NOs: 2 and 4, but also mutants having the physiological activity at the same level. Specifically, the present invention contains the protein comprising the amino acid sequence of SEQ ID NO: 2 or 4, or a protein having an XIAP-biding activity and comprising the amino acid sequence in which one or more amino acids are replaced, deleted, added, and/or inserted. Alternatively the proteins of the present invention contain the protein comprising the amino acid sequence of SEQ ID NO: 2 or 4, or all proteins comprising the amino acid sequences in which one or more amino acids are replaced, deleted, added and/or inserted, and desirably having 60% or higher homology as a whole to the above amino acid sequence and a TxR activity.

As understood from the amino acid sequence of SEQ ID NO: 2 or 4, TxRIIs of the present invention are a selenoprotein containing selenocysteine in a molecule. On the other hand, the previously reported human TxR has been reported to encode Secys by an amino sequence of Cys-Secys-Gly-stop codon (UAA) . Moreover, in bovine TxR, this Cys-Secys-Gly at C terminus is a region essential for the activity expression of TxR. Therefore, in human TxRIIs by the present invention, this region is considered to have an important meaning in the TxR activity expression.

A method for adding mutation in an amino acid sequence while maintaining a physiological activity is known. For example, as a method for preparing a mutant using the random mutation, the chemical mutagenesis method (Myers RM, et al. Methods Enzymol., 1987; 155: 501-527) is known. In this method, a random mutation is introduced into a single-stranded DNA of a target gene by adding a nucleotide modification reagent. Then, a double-stranded DNA is synthesized by using appropriate primers with the obtained single stand DNA as a template by PCR and cloned. A target mutant can be obtained by selecting a clone which provides an expression product having an desired activity from a library of mutants. On the other hand, as a method for preparing a mutant by determining a target nucleotide, a method for introducing the mutation by conducting PCR with a target gene as a template using mutation oligonucleotide primers is known (Ito W. et al., Gene 1991 June 15; 102 (1) : 67-70). Mutation in an amino acid sequence occurs not only by an artificial manipulation but also in the natural condition. A mutant of the present invention

More specifically, a method according to the method shown as an inhibitor assay of Example 7-5) can be presented. If a diluted series of a candidate compound is used as a sample and the decreased absorbance is observed dependently on the diluted series, the candidate compound is judged to have a binding inhibitory activity. Alternatively, a combinatorial chemistry can be applied. Specifically, a library of candidate compounds is prepared, and the proteins of the present invention are added thereto with XIAP to monitor XIAP to be bound to the candidate compound to screen an antagonistic inhibitory substance for TxRIIs. On the other hand, a compound which blocks the biding of TxRIIs to XIAP can be screened by using TxRIIs which bind to a candidate compound as an index.

In the screening method by the present invention, any proteins can be used as the above-described protein of the present invention as long as it comprises a biding activity domain with XIAP. Specifically, a protein is not necessarily the complete molecule of the amino acid sequence of SEQ ID NO: 2 or 4. In order to observe a binding of candidate compound or the protein of the present invention, these proteins are modified with an observable molecule. As an observable molecule, for example, radioactive isotopes, fluorescent substances, luminescent substances, and enzymatic active substances can be used. In the case of applying the above combinatorial chemistry, an immobilized library of candidate compounds on a solid phase is useful as isolation of reaction solution, washing, and the following measurement of labels are easily manipulated.

These methods can be used, not only for screening mutants in the present invention, but also, for screening compounds which inhibits the binding of XIAP and the protein of the present invention in general. Because a compound screened by this method can control signal transduction system in which XIAP is involved, the proteins provided by the present invention, an antibody thereof, an analogue thereof and such can be expected to have effects of inhibiting cancer, inducing cell death in virus infected cells through promotion of apoptosis, etc.

In addition, a method for screening a compound having an includes such a naturally occurring mutant as long as it maintains the TxR activity or the XIAP-binding activity.

As a method for confirming the TxR activity, the following two methods are known for example (Holmgren, A. et al. Methods Enzymol. 252: 199). First, using an appropriate SH indicator, such as 5, 5'-dithiobis (2-nitrobenzoic acid) (DTNB) , 5-mercapto-2-nitrobenzoic acid (TNB) produced by the TxR activity is measured with absorbance at 412 nm by a thiol. This reaction is shown as follows:

$$\text{DTNB} + \text{NADPH} + \text{H}^+ \rightarrow 2\text{TNB} + \text{NADP}^+$$

The other index for the TxR activity is a method called an insulin assay in which an enzyme activity is monitored by tracing a change created by the cleavage of the SS-bond of insulin by reduced form Trx resulted from the TxR activity. As a change which is an index, the decrease of absorbance at 340 nm by oxidation of co-enzyme NADPH, and absorbance at 412 nm of a thiol group resulted from reduction of insulin are used. Production process of reduced form Trx by TxR is as described above.

On the other hand, an XIAP-biding activity functionally equivalent to the binding activity in the natural form TxRIIα or β comprising the amino acid sequence of SEQ ID NO: 2 or 4 can be used. As a method for screening functionally equivalent substances, specifically, for example, the following methods can be used. Specifically, the method is a method for screening a compound having an activity of inhibiting the biding of XIAP with the binding substance and comprising the following processes (a) or (a)', (b), and (c) :

(a) contacting simultaneously a candidate substance as a subject for screening, and XIAP with the protein of the present invention, or (a)' contacting a candidate substance as a subject for screening with XIAP, and then, further contacting with the protein of the present invention,
(b) determining the amount of the protein of the present invention which binds and/or does not bind to XIAP, and
(c) selecting a compound which inhibits binding of XIAP and the protein of the present invention. activity of promoting or inhibiting the enzyme activity can be provided by using TxRIIs of the present invention. This method comprises the steps of:

(a) contacting a candidate substance as a subject for screening with TxRIIs,
(b) observing the change in the TxR activity of TxRIIs, and
(c) selecting a compound which promotes or inhibits the TxR activity in TxRIIs.

TxRIIs to be used for this screening are not necessarily a complete molecule, and a fragment maintaining an enzyme activity of TxRIIs can be used. The TxR activity can be measured based on a method such as the above method. Because the structure of TxRIIs is different from the known TxRI, a compound which affects one activity does not necessarily affect the other. Therefore, a method for screening a substance which affects an enzyme activity of TxRIIs is an essential technique for identifying inhibitors and activators specific to the enzyme activity of TxRIIs or obtaining a compound which affects TxRI in the same manner as in TxRIIs.

As TxR controls redox *in vivo,* an inhibitor for TxRIIs which can be obtained based on the screening method by the present invention can be expected to be used as an anticancer drug, or a therapeutic agent for autoimmune disorders. For example, an organic gold compound used as a general therapeutic agent for rheumatism, an autoimmune disorder, is considered to have a high inhibitory activity on selenoproteins, especially on TxR. Thus, a compound having an inhibitory effect on TxRII can be expected to have a similar pharmacological activity (Stephan Gromer et al., J. Biol. Chem. Vol. 273, No. 32, 20096-20101, 1998). Moreover, if a pharmacological activity through the inhibition of TxR activity is expected, the method for screening a compound which affects an activity of TxRII, provided by the present invention, is useful because it is necessary to select a compound effective not only to a known TxR but also TxRII.

The proteins of the present invention can be obtained by extracting and purifying from cells expressing TxRIIα or β. Selecting cells which highly express a target protein is advantageous. Because the nucleotide sequence of DNA encoding the target protein is provided, the method for screening cell lines which highly express the target gene by using a probe based on this sequence is routinely conducted by a person skilled in the art. As shown in Examples, TxRIIs by the present invention are expressed in many cultured cells, these cultured cells can be used as a material. A method for purifying a target protein by combining various extraction methods and protein purification methods from cell culture can be selected by a person skilled in the art from experiences. Specifically, various purification methods, for example, gel filtration, ion exchange chromatography, reversed phase chromatography, immuno affinity chromatography, can be used.

Apart from the purification from these natural materials, the proteins of the present invention can be obtained by the genetic engineering technique. For example, an expression vector is constructed by inserting a translation region to an appropriate vector based on the nucleotide sequence of SEQ ID NO: 1 or 3. Then this expression vector is transfected to an appropriate host to express the target TxRII as a recombinant.

In addition, the present invention provides cDNAs encoding the above proteins of the invention. The DNA comprising the nucleotide sequence of SEQ ID NO: 1 or 3 disclosed in the present invention is novel. Regarding cDNA of the present invention, the target gene can be obtained by screening a cDNA library using a probe designed based on, for example, the nucleotide sequence of SEQ ID NO: 1 or 3. Alternatively, the gene of the present invention can be obtained by synthesizing primers based on the nucleotide sequence of SEQ ID NO: 1 or 3, and conducting PCR using a cDNA library as a template. Probes and primers can be designed and prepared based on the nucleotide sequences of cDNA of the present invention by the methods known to a person skilled in the art. In primers for PCR, sequences close to 5' end and 3' end of a fragment to be amplified are selected. Addition of a restriction enzyme recognition site to 5' side of the primers is convenient for insertion into a vector. Both nucleotide sequences of SEQ ID NOs: 1 and 3 comprise the length of about 2 kbp. A whole region of such a length can be amplified by conducting PCR once using a pair of primers and cDNA as a template. A target gene can be sensitively detected by confirming an amplification product to be obtained by electrophoresis. An expression vector can be constructed by inserting the amplification product into a vector. A commercially available library used in Examples contains a full length cDNA of TxRIIs by the present invention. Therefore, by conducting PCR using this as a template, the cDNA of the present invention can be easily obtained. Alternatively the cDNA of the present invention can be obtained by conducting RT-PCR based on mRNA in each cell line which shown the expression of TxRIIs. An element of 3'UTR is as important as CDS at the construction of the expression vector for active form of TxRIIs, based on the nucleotide sequences of SEQ ID NO: 1 or 3. Among 3' UTR, the portions corresponding to 1780-1909 of SEQ ID NO: 1 and 1883-2012 of SEQ ID NO: 3 (SEQ ID NO: 5, 130 bp) constitute a common nucleotide sequence. This part is essential for expressing a complete form of TxRIIs containing selenocysteine. UGA which is a stop codon in general, is translated to selenocysteine by the stem loop structure composed of a part corresponding to this region in mRNA. As previously described, selenocysteine is considered to be an amino acid essential for an enzyme activity of TxR. Therefore, into the expression vector of the present invention, an insert should be inserted in the form containing this region. If the TxR activity is not expected to TxRIIs of the present invention, this region is not essential. For example, in the case of aiming the expression of a domain peptide of TxRIIs composed only of a specific region, a target protein can be obtained by inserting only the nucleotide sequence encoding a necessary amino acid sequence in the form able to express. To the domain peptide obtained in this manner, an enzyme activity of TxR can not be expected. However, for example, the domain peptide can be used as an immunogen for preparing an antibody which recognizes TxRIIs by the present invention. Alternatively, a mutant with a binding-activity with XIAP based on the present invention can be prepared by selecting a region serving the biding with XIAP.

The DNA of the present invention contains not only the DNA constituted by the nucleotide sequence of SEQ ID NOs: 1 and 3, but also mutants thereof. Mutants of the DNA based on the present invention are mainly classified to the following two. Specifically, first, a DNA comprising a nucleotide sequence encoding all proteins comprising mutation in the above amino acid sequence by the present invention is the DNA mutant based on the present invention. More specifically, a DNA encoding all mutants comprising mutation in the amino acid sequence within the range of maintaining an activity as TxRIIs are contained in the DNA of the present invention, regardless of being able to hybridizing to SEQ ID NO: 1 or 3 or not. Because several sequences correspond to codons for one amino acid in general (degeneracy), theoretically an astronomical number can be expected for a nucleotide sequences of DNA encoding a given amino acid sequence. From this reason, the DNA nucleotide sequences of the present invention must be identified regardless of complementarity to a specific sequence.

Second, a DNA which can hybridize to SEQ ID NO: 1 or 3, and encodes a protein having an activity as TxRIIs is included in the DNA of the present invention. Many of sequences which can hybridize to a specific sequence under stringent conditions are thought to have an activity similar to the protein encoded by the specific sequence. A specific example of hybridization conditions is 5xSSC, at 25°C in the absence of formamide, preferably, 6xSSC, at 25°C with 40% formamide, and more preferably, 5xSSC, at 40°C with 50% formamide. An example of washing after hybridization is 2xSSC at 37°C, preferably 1xSSC at 55°C, and more preferably 1xSSC at 60°C.

The nucleotide sequence of DNA of the present invention including mutants can be used for various uses based on the known technologies. Based on the cDNA nucleotide sequence identified in the present invention, an oligonucleotide which specifically hybridizes to this nucleotide sequence can be obtained. An oligonucleotide of the present invention is composed of at least 15 nucleotides in order to archive hybridization under stringent conditions, preferably of 15-200 nucleotides, and more preferably 25-100 nucleotides. Such a nucleotide can be used as a probe and a primer. Based on a given sequence, a person skilled in the art routinely designs a probe specifically hybridizing to the sequence. A nucleotide sequence archiving a specific hybridization is not necessarily completely complementary on a target nucleotide sequence. Variation of sequences is acceptable as long as it can archive the necessary specificity under stringent conditions. An oligonucleotide comprising a determined nucleotide sequence can be obtained by the chemical synthesis. The oligonucleotide can be used for hybridization assays in various formats by adding an appropriate label to the oligonucleotide. In the case of using as a primer, multiple regions can be set depending on a synthesis principle for a complementary strand. For example, as a primer for PCR, a region determining both 5' and 3' sides in the segment which is an object of the synthesis is selected. The oligonucleotide of the present invention can be applied to various complementary strand synthesis reaction, for example, not only basic PCR, but also RT-PCR with RNA as a template, nested PCR which enables a sensitive detection by nesting a amplification region, cDNA synthesis, etc.

For example, as a primer for amplifying cDNA of TxRIIs, or for amplifying 3'UTR, the following nucleotide sequences can be presented. By using a primer for amplifying cDNA of TxRIIs described below, TxRIIs of the present invention can be distinguished from a known TxR and cDNA of the latter can be specifically amplified.

Forward primer for TxRIIα (SEQ ID NO: 13) :
5'-ACGATGGCGGCAATGGCGGTG-3'

Forward primer for TxRIIβ (SEQ ID NO: 14):
5'-ACCATGGAGGACCAAGCAGGT-3'
Reverse primer for TxRIIs (SEQ ID NO: 15):
5'-TTACCCTCAGCAGCCTGTCAC-3'
Forward primer for 3'UTR (SEQ ID NO: 16) :
5'-GCGCCATCCCTGCAGGCCAGG-3'
Reverse primer for 3'UTR (SEQ ID NO: 17) :
5'-CACACTTCAGAAAAAGTACCC-3'

The oligonucleotide based on the present invention can be used as an antisense DNA which inhibits the expression of TxRIIs. There are more than one factors as an inhibitory effect of an antisense nucleic acid on the expression of a target gene (Hirashima and Inoue: "Shin-seikagaku Jikken Koza (New Biochemistry Experiment) 2 Nucleic Acid IV Replication and Expression of a gene", Edited by Japanese Biochemistry Society, Tokyo-Kagakudojin, pp. 319-347, 1993). The expression of a target gene can be inhibited by any of the effects. In one embodiment, the translation of the gene is effectively inhibited by designing an antisense sequence complementary to non-translation region close to 5' end of mRNA in the gene. A sequence complementary to a coding region or a non-translation region at 3' side, however, can be used. A DNA including an antisense sequence of not only a translation region of a gene but also a non-translation region is included in the antisense DNA used in the present invention. An antisense DNA to be used is ligated downstream of an appropriate promoter and preferably a sequence containing a transcription termination signal is ligated to 3' side thereof. The DNA prepared in this manner can be transfected into cells in which the expression should be inhibited by a known method. A sequence of an antisense DNA is preferably complementary to an endogenous TxRIIs gene contained in cells to be transformed (or a homologous gene) or a part thereof, but is not necessarily completely complementary as long as it is able to effectively inhibit the expression of the gene. A transcribed RNA has preferably 90% complementarity, and the most preferably 95% complementarity on the transcription product of a target gene. To effectively inhibit the expression of the target gene using an antisense sequence, the length of an antisense DNA is at least 15 or more nucleotides, preferably 100 or more nucleotides, and more preferably 500 or more nucleotides. Ordinarily, the length of an antisense RNA to be used is shorter than 5 kb, and preferably shorter than 2.5 kb. The expression of an endogenous gene can be inhibited by using a DNA encoding a ribozyme.

The present invention provides an antibody which recognizes the protein based on the present invention. An antibody of the present invention can be prepared by immunizing the protein obtained in the above manner or a fragment thereof through a known method. In immunization, adjuvant, such as FCA, is mixed with an immunogen and subcutaneously immunized to an animal to be immunized by an appropriate immunization schedule. High immune stimulation can be expected by selecting an animal to be immunized, in which the structure of TxR is as different from that of human as possible. An antibody can be prepared not only as a polyclonal antibody purified from serum of the immunized animal, but also as a monoclonal antibody which can be obtained by cloning antibody-producing cells. The method for collecting antibody-producing cells of an immunized animal and establishing cell lines which produce monoclonal antibodies by fusing the cell lines with cultured cell lines enabling cloning is obvious to a person skilled in the art. The antibody obtained in this manner can be used for immunologically detecting and purifying TxR by the present invention.

Moreover a gene in variable region of an antibody contained in antibody-producing cells which recognizes TxRIIs derived from animals of different species can be used for humanization. Specifically, for example, a chimeric antibody which comprises a constant region of a human antibody in the antibody variable region of a mouse can be created by gene recombination. A method for obtaining a so-called humanized antibody in which a hypervariable region is solely inserted into a framework of a human antibody is known. These humanized antibodies can be safely and effectively used in vivo because an immunological reaction is difficult to occur in the case of administering to human.

## Brief Description of the Drawings

Figure 1 shows the alignment of amino acid sequences for TxRIIα of the present invention and the known TxR.

Figure 2 is a photograph showing the result of detecting TxRIIs in each cultured cell line by Western blot method using an antiserum of mouse anti-TxRIIα.

Figure 3 shows the TxR activity measured by the DTNB assay in the TxRIIα recombinants fused with each tag. The vertical and horizontal axes indicate absorbance at 412 nm and reaction time, respectively.

Figure 4 shows the TxR activity measured by insulin assay in the TxRIIα recombinants fused with each tag. The vertical and horizontal axes indicate change of absorbance at 340 nm and reaction time, respectively.

Figure 5 shows effects of the TxR activity inhibitor on the TxR activity of the flag-tag fused TxRIIα protein, measured by the DTNB assay. As a TxR activity inhibitor, 1-chloro-2, 4-dinitrobenzene (CDNB) and 13-cis-retinoic acid are used. The vertical and horizontal axes indicate absorbance at 412 nm and reaction time, respectively.

## Best Mode for Carrying Out the Invention

The present invention is illustrated in detail below based on the Examples.
All techniques used in the present invention followed J. Sambrook, E. F. Fritsch & T. Maniatis (1989) Molecular Cloning, a laboratory manual, second edition, Cold Spring Harbor Laboratory Press.

1. Cloning of XIAP by PCR

1-1) Preparation of primers

The following two primers were synthesized to isolate the full length human XIAP gene by PCR.
· 5' primer (XIAP2486 (32mer))
5'-GCG GGA TCC ATG ACT TTT AAC AGT TTT GAA GG-3'
* 3 bases (GCG) at 5'end are for conveniently conducting the restriction enzyme treatment.
(GGATCC) from the $4^{th}$ to the $9^{th}$ bases at 5' end is a restriction enzyme BamH I site.
· 3' primer (XIAP 2482 (32 mer))
5'-GCG CTC GAG CTA CTA TAG AGT TAG ATT AAG AC-3'
*3 bases (GCG) at 5'end are for conveniently conducting the restriction enzyme treatment.
(CTCGAG) from the $4^{th}$ base to the $9^{th}$ base at 5' end is a restriction enzyme Xho I site.

1-2) PCR

Using the cDNA derived from human T-cell-derived Jurkat cells as a template DNA, the full length human XIAP gene was amplified by PCR. PCR was conducted with GeneAmp PCR System 2400 (PERKINELMER) by the following program.

a) 94°C for 5 min
b) 1 cycle of 94°C for 1 min, 58°C for 3 min, 72°C for 3 min
c) 35 cycles of 94°C for 1 min, 65°C for 1 min, 72°C for 2 min
d) 72°C for 10 min

1-3) Cloning of a PCR product to pAS2-1 vector

i) Purification of a PCR product

The amplified DNA fragment was confirmed by the 1% agarose electrophoresis after PCR. This DNA fragment was treated with restriction enzymes BamH I and Xho I. The DNA fragment treated with restriction enzymes was electrophoresed by the 1% agarose, excised and purified by Glass Matrix method (GeneClean, BIO101).

ii) Preparation of vector

Vector pAS2-1 is a bait vector used in MATCHMAKER Two Hybrid System (a product name) of Clontech, and comprises a multicloning site (MCS) downstream of a sequence encoding GAL4-DNA-BD (a DNA biding domain of GAL4 protein) . A bait in the two hybrid system means a vector in the side which expresses a known protein functioning as a probe for searching unknown binding factors. To match translation frames for GAL4-DNA-BD and the PCR product, this MCS was digested with Nde I at the restriction enzyme Nde I site, blunt-ended by the standard method, and self-ligated to obtain the vector pAS∆NdeI (+2) in which two frames were slipped. The fragment treated with restriction enzymes BamH I and Sal I was purified in the same manner for a PCR product. The purified product and the fragment of vector pAS2-1∆NdeI were ligated. The purified PCR product and pAS2-1∆NdeI were mixed in the molar ratio of 1, and reacted for 1 hour at 16°C with T4 DNA ligase.

iii) Transformation of *E. coli*

A ligation reaction solution was added to *E. coli* strain DH5α made competent by the standard method (Hanahan, D. 1983 Studies on transformation of *Escherichia coli* with plasmids, J. Mol. Biol 166: 557), gently mixed, kept on ice for 30 min, heat-shocked for 90 sec in warm water at 42°C, kept on ice for 2 min again, and cultured with shaking at 37°C for 1 hour in SOC medium. The product was spread on a LB plate containing 50 μg/ml ampicillin and cultured overnight at 37°C.

iv) Collection of DNA by the alkaline-SDS method and confirmation of an insert

Colonies were harvested from the plates and cultured in a LB-ampicillin medium at 37°C overnight. From the cultured *E. coli*, plasmid DNA was collected using the alkaline-SDS method. The collected plasmid DNA was cleaved by an appropriate restriction enzyme and insertion of the target PCR product into the vector was confirmed by the agarose electrophoresis.

v) Confirmation of sequences

The collected DNA was purified by the polyethylene glycol precipitation method and the PCR product in the vector was confirmed by the fluorescence sequencer (PERKINELMER) based on the Sanger method. In this manner, plasmid DNA of pASΔNdeI (+2) -XIAP in which the full length human XIAP gene was inserted into pASΔNdeI (+2) vector was obtained.

2. 2 Hybrid screening

In analyses of intracellular information transduction mechanisms and studies on cellular mechanisms at higher levels, detection of interaction between proteins and identification of known or unknown molecules interacting with a known protein are very important. The two hybrid screening system has been given attention for detecting a interaction between proteins encoded by two genes, or as a method for cloning a molecule interacting a gene product. In this method, each of two gene products is fused to a DNA binding site (GAL4-DNA-BD) and a transcription activation site (GAL4-AD) in a transcription factor, to detect the interaction between two, using a transcription activity as an index. A GAL4-DNA-BD fusion protein and a GAL4-AD fusion protein are simultaneously expressed in a yeast nucleus. When the both interact, HIS3 gene comprising GAL4 promoter upstream and lac Z gene are expected to be transcribed and translated. Specifically, the yeast can grow in the agar medium without histidine only in the presence of the interaction between the both, and β-galactosidase (abbreviated to β-Gal, hereafter) activity can be detected using X-gal as a substrate. The two hybrid screening system is so advantageous that interaction of two gene products can be judged in a yeast nucleus *in vivo* without purifying the proteins. However, a protein transcribed from the GAL4 promoter without showing interaction can not be screened. Therefore, it is very important to confirm that lac Z gene does not express only with the GAL4-DNA-BD fusion protein, namely, there is no β-Gal activity. For the two hybrid screening, the MATCHMAKER two hybrid system method 2 of CLONTECH was used and all experimental methods followed this protocol.

2-1) Purification of a library DNA for pray

Human Placenta MATCHMAKER cDNA Library purchased from CLONTECH was used as a library for screening. This library was prepared by pACT2 vector and contains a MCS downstream of a sequence encoding GAL4-AD (an Activation Domain of the GAL4 protein) and a cDNA fragment was inserted into this MCS. In the two hybrid system method, a library predicted to contain unknown binding factors is called a pray. About 20,000 colonies per an LB-ampicillin plate with a diameter of 150 mm were spread and these 100 plates were cultured at 30°C overnight and bacterial cells were cultured in a LB ampicillin liquid medium at 30°C for 4 hours. Plasmid DNA was collected from the harvested *E. coli* cells by the polyethylene glycol precipitation method and purified.

2-2) Confirmation of expression of a fusion protein and the absence of β-Gal activity

Yeast was transformed by the constructed pASΔNdeI (2+)-XIAP, and expression of XIAP as the GAL4-DNA-BA fusion protein, and an activation of GAL4 promoter solely by the GAL4-DNA-BD fused XIAP (bait) but no-expression of lac Z gene were confirmed.

Yeast Y 190 made competent by the lithium acetate method (Gietz, D., Jean A., Woods, R. A., & Schiestl, R. H. 1992, Improved method for high efficiency transformation of intact yeast cells. Nucleic Acid Res. 20: 1425) was transformed by using plasmid DNA of pASΔNdeI (+2)-XIAP. Colonies obtained by transformation were cultured in the SD/-Trp liquid medium at 30°C for 3 days. After the culture, yeast cells were harvested by centrifugation, and proteins were extracted from yeast by the standard method (Printen, J. A. & Sprague, G. F., Jr. (1994) Protein interactions in the yeast pheromone response pathway: Step 5 interacts with all members of the MAP kinase cascade. Genetics 138: 609-619) , using the urea/SDS protein extraction buffer. After electrophoresis of proteins by SDS-PAGE, the proteins were blotted on the PVDF protein. The expression of the fusion protein of GAL4-DNA-BD and XIAP was confirmed by Western blot using the anti-GAL4 DNA binding domain monoclonal antibody (CLONTECH) and anti-XIAP polyclonal antibody.

A sterile nylon transfer membrane (Hybond-N+, Amersham) was placed on the plate on which yeast transformants in which expression of the fusion protein between GAL4-DNA-BD and XIAP was confirmed were grown. Thus, the colonies were transferred to the membrane. This membrane was immersed in liquid nitrogen for 10 sec, returned to room temperature, placed on a filter paper immersed with the Z-buffer/X-gal solution (100 ml Z-buffer (16.1 g/L $Na_2HPO_4$-$7H_2O$, 5.50 g/L $NaH_2PO_4$-$H_2O$, 0.75 g/L KCl, 0.246 g/L $MgSO_4$-$7H_2O$, adjusted to pH 7.0) , 0.27 ml mercaptoethanol, 1.67 ml X-gal solution (20 mg/ml X-gal in DMFA)) with the surface with colonies up, and kept at 30°C for one hour or longer. As a result, the yeast transformants in which the expression of the fusion protein of GAL4-DNA-BD and XIAP was confirmed did not turn blue. Specifically, sole the fusion protein of GAL4-DNA-BD and XIAP did not activate transcription from the GAL4-promoter, confirming that the two hybrid screening system can be used.

2-3) The primary screening

The yeast transformants in which the expression of the fusion protein of GAL4-DNA-BD and XIAP was confirmed were mass-cultured and were made competent by the lithium acetate method. These were transformed by the previously prepared Human Placenta MATCHMAKER cDNA library. The obtained transformants were streaked on the plates of SD/-Trp/-Leu/-His/+3-AT, and cultured for 7 days at 30°C. By this, only yeast in which bait bound to pray and His3 gene downstream of the GAL4 promoter expressed to become His+ can only grow to form colonies. Independent clones of the library used were 5 X $10^6$ and actually screened ones were 72.5 X $10^7$7, and thus about 5 times were screened. His+ yeasts in this first screening were 82 clones.

2-4) The second screening; β-gal assay

To confirm that in the clones obtained in the first screening, a bait actually bound to a pray to express a gene downstream of the GAL4 promoter, expression of another lac Z gene located downstream of the GAL4 promoter, specifically β-gal activity, was examined. A nylon transfer membrane was placed on the SD/-Trp/-Leu/-His/+3-AT agar plate, and 82 yeast clones which became His+ in the first screening were cultured and grown on this membrane. Clones having His+ and the β-gal activity were obtained by measuring the β-gal activity by colony lift filter assay. By this second screening, 74 colonies having the β-gal activity were obtained.

2-5) Sequencing of a pray

Plasmid DNA was harvested from yeast and transferred to *E. coli*, to examine DNA sequences inserted into the clones obtained by screening.

The yeast clones having His+ and the β-gal activity were scratched from the plates, and cultured on the SD/-Leu medium overnight. Bacterial cells were collected and treated by following the standard method (Kaiser, P. & Auer, B. (1993) Rapid shuttle plasmid preparation from yeast cells by transfer to *E. coli*. Bio Techniques 1.4: 552) to collect yeast plasmid DNA.

*E. coli* HB101 for electroporation, made competent using HEPES-NaOH was electrotransformed with the plasmid DNA collected from yeast. After electroporation, SOC medium warmed at 37°C was added thereto, and the *E. coli* was cultured with shaking at 37°C for 1 hour to recover. The *E. coli* was spread on the -Leu plate (M9 plate containing 50 μg/ml ampicillin, 40 μg/ml proline, 1 mM thiamine hydrochloride, -Leu dropout solution) and cultured at 37°C overnight. *E. coli* HB101 has LeuB mutation. Therefore, among plasmid DNA obtained from yeast, only library vectors encoding LEU2 gene which can complement leuB mutation can transform the *E. coli* HB101 and form colonies on the plate. From grown *E. coli* HB101, plasmid DNA was extracted by the alkaline-SDS method. *E. coli* DH5α was transformed using the harvested plasmid DNA.

Plasmid DNA of pACT2 vector in *E. coli* DH5α was harvested by the alkaline SDS method, and purified by the polyethylene glycol precipitation method. Based on Sanger method, the nucleotide sequences of the genes in the vectors were confirmed by the florescent sequencer. In this manner, a novel gene X19 was obtained.

2-6) Confirmation by re-transformation

After transforming yeast Y190 with the purified plasmid DNA of pACT2-X19, it was confirmed that sole the fusion protein of the GAL4-AD protein and X19 did not cause transcription from the GAL4 promoter by measuring the β-gal activity. By measuring the β-gal activity in Y190 transformed by pASΔNdeI (+2) -XIAP and pACT2-X19, and measuring the β-gal activity in Y190 transformed by pAS-X19 and pACT-XIAP, transcription from the GAL4 promoter, namely, the binding of XIAP and X19 in the yeast nucleus was confirmed.

3. X19 amino acid sequence homology search

Amino acid sequence homology search was conducted using www service (http://www.genome.ad.jp) of Human Genome Analysis Center, Medical Science Institute, The university of Tokyo, and of Supercomputer Laboratory at Institute of Chemistry, Kyoto University to predict the functions of X19 from the amino acid sequence.

3-1) Sequence homology search program BLAST

Using the non-redundant amino acid sequence data base nr-aa, sequences homologous to amino acid sequence of X19 were searched (blastp search). As a result, X19 was a novel gene having 55% homology to human thioredoxin reductase and 38% homology to human glutathione reductase. Moreover, functional regions (a redox active center, a FAD-biding region, a NADPH-binding region, a selenocysteine active center) reported in human thioredoxin reductase were completely conserved in the homologous manner in X19 (Figure 1, SEQ ID NO: 1) . Therefore, we named X19 human thioredoxin reductase II (TxRII).

4. Obtaining the full length TxRII cDNA

4-1) Obtaining a full length cDNA by colony hybridization

From Human Placenta MATCHMAKER cDNA library, a full length TxRII cDNA was obtained by colony hybridization. For screening, a DNA fragment was amplified by PCR from a partial sequence of the sequenced TxRII and used as a probe.

i) Preparation of a membrane for colony hybridization

Human Placenta MATCHMAKER cDNA library was diluted and spread on a LB (ampicillin) plate with a diameter of 150 mm, on which 4 X $10^4$ or more colonies can grow per plate. These 12 plates were prepared and cultured at 30°C overnight. The colonies were transferred to a membrane for hybridization, and the membrane for colony hybridization was prepared by following the standard method.

ii) Preparation of a probe

About 500 bp DNA fragment at N-terminal side was obtained using the following primers by PCR with the cDNA of TxRII as a template. TxRII-sF3 5'-TAT GAT CTC CTG GTG GTC-3'
TxRII-sR2 5'-GTC ATC ACT TGT GAT TCC-3'

The amplified DNA fragment was separated by the 1% agarose gel electrophoresis, and purified by the glass matrix method. From the purified DNA fragment, a [$^{32}$P] labeled probe was prepared using the DNA random labeling kit (rediprime DNA labelling system, Amersham) and [$\alpha$-$^{32}$P] deoxy-CTP (ICN), and purified by spin column (ProbeQuant G-50 Micro Column, Pharmacia).

iii) Hybridization

Hybridization was conducted using a hybridization bottle and a hybridization oven (TAITEC). The membrane crosslinked with DNA was pre-hybridized in hybridization buffer (10% PEG6000, 1.5% SSPE, 7% SDS) at 65°C for 1 hour. The [$^{32}$P] labeled probe was boiled, immediately cooled, and diluted with hybridization buffer warmed at 65°C and the solution used for prehybridization was replaced by the hybridization buffer. Hybridization was conducted at 65°C overnight.

iv) Washing and autoradiography

Hybridization buffer was washed with washing solution of 0.1xSSC, 0.1% SDS, and the level of washing was appropriately confirmed by a survey meter. Washing solution was replaced several times until a count of washing solution was completely absent, and then the membrane was loaded on the film to detect positive colonies by autoradiography.

v) Isolation of positive colonies

Positive colonies were isolated by a Pasteur pipet, diluted by the different dilution ratios, spread on a LB (ampicillin) plate of 100 mm diameter and cultured at 30°C overnight. Hybridization was conduced by the same manner and single positive colony was obtained. From this, plasmid DNA was harvested and the DNA sequence was determined. SEQ ID NO: 1 shows the nucleotide sequence of TxRII α cDNA determined in this manner.

4-2) Obtaining a full length cDNA by PCR cloning

From Human Placenta MATCHMAKER cDNA library used in the two hybrid system, TxRII gene was attempted to obtain by PCR by combining TxRII specific primers and library vector specific primers. Sequences of used primers were set as follows based on the nucleotide sequences of the clones obtained by colony hybridization.

TxRII specific primer 1
5'-ACA GCT TCT GCC ATC TTC CTC-3'
TxRII specific primer 2
5'-AGA AGG TTC CAC GTA GTC CAC-3'
Library vector specific primer
5'-CCA TAC GAT GTT CCA GAT TAC-3'

PCR was conducted by the combination of TxRII specific primer 1 and the library vector specific primer in the following program, using GeneAmp PCR System 2400 (PERKINELMER).

a) 94°C, 5 min
b) 35 cycles of 94°C 30 s, 56°C 30 s, 72°C 1 min and 30 s,
d) 72°C 10 min.

A PCR product was electrophoresed by the 1% agarose gel, excised, and purified to be used as a template for the following PCR. The second PCR was conducted using the combination of the TxRII specific primer 2 and the library vector specific primer by the following program.

a) 94°C, 5 min
b) 35 cycles of 94°C 30 s, 56°C 30 s, 72°C 1 min and 30 s,
d) 72°C 10 min.

The PCR product was electrophoresed by the 1% agarose gel, excised, purified, and cloned by using Topo TA cloning Kit (Invitrogen) and DNA sequence of the PCR product was sequenced. As a result, cDNA containing 5'-non amino acid translation region of about 180 bp was obtained and the first methionine (Met) was judged as the first Met due to the presence of Kozak consensus immediately before the methionine. The sequence at N-terminal side, however, was different from that obtained by the yeast two hybrid method. Because the sequence of the second exon and following sequence in this gene was identical to that in TxRII, the gene was decided to be an alternative splicing form of TxRII. The gene obtained by yeast two hybrid method, and the alternative splicing form were designated TxRIIα and TxRIIβ, respectively. The second exon and the following part in TxRIIβ is identical to that in TxRIIα(SEQ ID NO: 3).

In addition, based on the cDNA nucleotide sequence of TxRIIs, known genomic nucleotide sequences were searched, and the cDNA nucleotide sequence of TxRIIs was mapped on 22q11.2. The genes encoding TxRIIs were present in 70 kbp in this region while separating into 18 exons. The presence of a gene encoding a protein having the binding activity with XIAP or the TxR activity was not predicted in this region.

5. Preparation of anti-TxRII antibody

In order to prepare an antibody against human TxRII proteins, a fusion protein with glutathion-S-transferase (GST) protein was purified as an immunogen, and anti-TxRII mouse antiserum was harvested by immunizing a mouse.

5-1) Expression of the GST-TxRIIα fusion protein

The TxRIIα fragment was re-cloned to pGEX vector (Pharmacia) from pACT2-TxRIIα to construct pGEX-TxRIIα. *E. coli* (DH5α) transformed with this pGEX-TxRIIα was cultured in a LB-ampicillin medium at 37°C overnight. This cultured medium was added to a fresh LB-ampicillin medium at 100X dilution, and cultured at 37°C. When the turbidity of the culture medium reached about 0.6, IPTG (isopropyl-β-D(-)-thiogalactopyranoside) was added thereto at the final concentration of 0.5 mM to express the GST-TxRIIα fusion protein, and cultured at 37°C for further 4 hours. The bacterial cells were harvested by centrifugation after the culture.

The collected bacterial cells were well-suspended in ice-cooled PBS containing 1% Tween -20, and completely crushed by ultrasonication. The crushed solution was centrifuged and the supernatant was passed through a GSH-sepharose 4B column (Pharmacia) to adsorb a GST fusion protein on the column. The column was washed well with WE buffer (10 mM β-mercaptoethanol, 2 mM $MgCl_2$, 20 mM Tris-HCl, (pH7.5)), and the GST-TxRIIα fusion protein was eluted using G buffer (10 mM GSH, 50 mM Tris-HCl, pH 9.6). The eluate was concentrated by 50% glycerol/PBS and the buffer was replaced.

5-2) Immunization of the GST-TxRIIα fusion protein into a mouse, collecting blood, and confirmation of reactivity

The purified GST-TxRIIα fusion protein and Freund's complete adjuvant were emulsified, and intraperitoneally injected into a mouse. This manipulation was repeated once a week for 5 weeks, and blood was collected from the mouse to collect serum containing the anti-TxRII antibody. The immunogen, TxRII overexpressed in mammalian cells, and the reactivity in various cultured cells were confirmed by the Western blotting method using this antiserum.

6. Western blotting method (Figure 2)

Soluble proteins were prepared from cultured cells, and protein concentration was measured by following the standard method (M. M. Bradford, Anal. Biochem. 72, 248, 1976), and SDS-PAGE was conducted with 40 μg of protein per lane. This was immunodetected with anti-TxRII antiserum and the presence of TxRII protein present in each cultured cell line was confirmed. As a result, the expression of TxRII was confirmed in each type of cultured cells. In Figure 2, TxRIIα was the band at around 70 kDa, and TxRIIβ was the band at around 55 kDa. The expression of TxRIIβ was not confirmed in mouse or rat cultured cells. The following 11 cell lines were used as samples.

Raji human Burkitt's lymphoma-derived cell line
Jurkat human T cell acute lymphoblastic leukemia-derived cell line HL60 human acute promyelocytic leukemia-derived cell line
U937 human histiocytic lymphoma-derived cell line
ZR75-1 human epidermic breast cancer-derived cell line
HepG 2 human protopathic hepatoblastoma-derived cell line
HeLa human uterine cervix cancer-derived cell line
A 431 human vulva squamous cell carcinoma-derived cell line
MRC-5 human-derived normal fibroblast cell line
NIH/3T3 mouse fetus-derived normal fibroblast cell line
Rat-1 rat fetus-derived normal fibroblast cell line

7. Purification and activity measurement of the recombinant TxRII α protein

7-1) Preparation of histidine tag fused TxRIIα protein

To pcDNAHis, a mammalian cell expression vector, was sub-cloned a full length TxRIIα gene containing 3'UTR (SEQ ID NO: 1). By transfecting this plasmid DNA to a mammalian cells, TxRIIα protein in which a histidine tag is added at N-terminal side is overexpressed in the cells. The plasmid DNA was transfected to 293T cells by the lipofection method according to the standard method. The cells were harvested 48 hours after the transfection, and the target protein was purified by using the kit for purifying a histidine-tag fusion protein.

7-2) Purification of flag-tag fused TxRIIα protein

To pcDNAFlag, a mammalian cell expression vector, the full length gene of TxRIIα containing 3'UTR was sub-cloned. By transfecting this plasmid DNA into mammalian cells, selenocysteine was inserted into a protein, and only a protein in which flag-tag was added at C-terminal side of TxRIIα can be collected with the anti-Flag antibody affinity column.

According to the standard method, using the lipofection method, the plasmid DNA was transfected to 293T cells. The cells were collected 48 hours after the transfection, and the cell extract solution was passed through the anti-Flag antibody affinity column to collect the flag-tag fused TxRIIα protein using a peptide of Flag.

7-3) Purification of the MYC-tag fused TxRIIα protein

To pCMVmyc, a mammalian cell expression vector, the full length gene of TxRIIα containing 3'UTR was sub-cloned. By transfecting this plasmid DNA into mammalian cells, proteins in which MYC-tag is added at N-terminal side in TxRIIα are overexpressed. By following the standard method, using the lipofection method, the plasmid DNA was transfected to 293T cells. The cells were collected 48 hours after the transfection, Protein A sepharose to which the anti-MYC monoclonal antibody was bound was added to the cell extract solution, and gently stirred at 4°C for 2 hours. By centrifuging, the MYC-tag fused TxRIIα protein binding to protein A sepharose to which the anti-MYC monoclonal antibody bound was precipitated, the supernatant was removed, and the proteins were washed several times with NETN buffer (10 mM Tris-HCl, 1 mM EDTA, 0.5% NP-40, 150 mM NaCl).

7-4) Activity measurement

By following the standard method (Holmgren, A. and Bjornstedt, M. 1995, [21], Thioredoxin and Thioredoxin Reductase Methods in Enzymol 252: 199) , an activity of TxR was measured by the DTNB assay, and the insulin assay.

i) DTNB assay

DTNB assay is a method in which TNB caused by the TxR activity from DTNB is measured by the absorbance of a thiol at 412 nm based on the following reaction formula. The purified tag fused TxRIIα protein (1 to 50 μl) was added to the assay buffer 1 to mess up to 1.0 ml. The absorbance at 412 nm was measured at 25°C for 5 min (Figure 3). DTNB + NADPH + $H^+$ 2TNB + $NADP^+$

Assay buffer 1:

100 mM potassium phosphate pH 7.0, 10 mM EDTA, 0.25 mM NADPH, 0.2 mg/ml bovine serum albumin (BSA), 1% ethanol, 1 mM DTNB

As a result, all TxRIIα purified by three methods was found to have the activity equivalent to that of control TxR derived from *E. coli*. The reason why the activity of histidine and the MYC-tag fused TxRIIα protein is slightly low is considered that TxRIIα in which selenocysteine was not incorporated at C-terminal side was mixed to inhibit the reaction.

ii) Insulin assay

The purified tag-fused TxRIIα protein (1 to 50 μl) was added to the assay buffer 2 and messed up to 1.0 ml. Oxidation of NADPH was measured by decreased absorbance at 340 nm at 30°C for 5 min (Figure 4). The TxR activity reduces Trx and the reduced form Trx further reduces insulin. At this time, the TxR activity can be measured by the amount of NADPH to be oxidized. The amount of oxidized NADPH was calculated by the following calculation formula. ΔA340 x 0.5 / 6.2

Assay buffer 2:

50 mM phosphate buffer pH 7.0, 20 mM EDTA, 80 mM insulin, 0.25 mM NADPH, 16 mM *E. coli* Trx-S2

As a result, all TxRIIα purified by three methods was found to have the activity equivalent to that of control TxR derived from *E. coli*. The reason why the activity of histidine and the MYC-tag fused TxRIIα protein is slightly low is considered that TxRIIα in which selenocysteine was not incorporated at C-terminal side was mixed to inhibit the reaction.

7-5) Inhibitor assay

To compare an enzyme activity of the TxRIIs by the present invention, obtained as a recombinant, and an activity of the natural TxR, an effect of an inhibitor was observed. As an inhibitor for the TxR activity, 1-chloro-2, 4-dinitrobenzene (CDNB) and 13-cis-retinoic acid was used. For confirming the TxR activity, the DTNB assay was used.

The diluted series of the inhibitors was prepared with 0.2 ml of HE buffer (100 mM HEPES buffer pH 7.2, 5 mM EDTA) . The tag-fused TxRIIαprotein was prepared at 3 μM and 0.2 ml thereof was added thereto, then 0.2 ml of HE buffer containing 3 mM NADPH and 30 mM DTNB was added thereto. The reaction system of this solution is composed of 100 mM HEPES buffer pH 7.2, 5 mM EDTA, 1 μM flag-tag fused TxRIIα protein, 1 mM NADPH, and 10 mM DTNB. The amount of reduced insulin was measured by absorbance of a thiol at 412 nm at 25°C for 5min. Figure 5 shows the result.

As a result, the activity of the purified flag-tag fused TxRIIα protein was clarified to be effectively inhibited by CDNB and 13-cis retinoic acid, as previously reported in the references of TxR I. The TxRIIα of the present invention was predicted to express an enzyme activity by the same mechanism as in the known TxR.

## Industrial Applicability

Higher animal's TxR was first purified as an enzyme in the 1990's, and the amino acid sequence thereof was reported in 1995. TxR in higher animals was given attention due to the difference in the size and substrate specificity of the proteins from the homologues in lower animals reported previously. The presence of TxRIIS in human, however, was not predicted, and thus the structure and activity of TxRIIs revealed in the present invention is very meaningful. The following is the importance of the present invention in detail.

The present invention provides an important information in screening of anticancer agents. It has been mentioned that TxR is given attention as a target for an anticancer agent. The importance of the present invention is large because it revealed that there are more than one species of molecules for the target. Specifically, to provide more certain therapeutic effects, an approach for comprehensively controlling the TxR activity including TxRIIs of the present invention is needed. This kind of approach can be possible first by the knowledge of the present invention.

In a cDNA provided by the present invention, there is 3'UTR constituting the stem loop structure essential for translating selenocysteine present close to C-terminus of TxRIIs. This nucleotide sequence supports the expression of the region containing selenocysteine essential for the expression of the TxR activity. The 3'UTR clarified in the present invention is composed of only 130 bp, and the fact that selenocysteine can be translated by such a short sequence is a novel knowledge. Moreover, considering the present invention from the aspect that the XIAP-binding protein was isolated, the protein of the present invention may bind to XIAP serving the control of apoptosis and, thus, may control the functions. The present invention provides a novel technique for promoting apoptosis, through this possibility. Promotion of apoptosis induces the death of abnormal cells, for example, cancer and virus-infected cells, leading to the treatment of the diseases.

SEQUENCE LISTING

<110> Medical & Biological Laboratories Co.,Ltd.

<120> Thioredoxin reductase II

<130> M3-007PCT

<140>
<141>

<150> JP 1998-310422
<151> 1998-10-30

<160> 37

<170> PatentIn Ver. 2.0

<210> 1
<211> 1959
<212> DNA
<213> Homo sapiens

<220>
<221> CDS
<222> (10)..(1572)

<220>
<221> misc_structure
<222> (1567)..(1569)

<220>
<221> misc_structure

<222> (1664)..(1666)

<223> tga is transrated to selenosysteine, shown by Xaa.

<400> 1

```
atggcggca atg gcg gtg gcg ctg cgg gga tta gga ggg cgc ttc cgg tgg 51
          Met Ala Val Ala Leu Arg Gly Leu Gly Gly Arg Phe Arg Trp
          1               5                   10

cgg acg cag gcc gtg gcg ggc ggg gtg cgg ggc gcg gcg cgg ggc gca   99
Arg Thr Gln Ala Val Ala Gly Gly Val Arg Gly Ala Ala Arg Gly Ala
15                  20                  25                  30

gca gca ggt cag cgg gac tat gat ctc ctg gtg gtc ggc ggg gga tct  147
Ala Ala Gly Gln Arg Asp Tyr Asp Leu Leu Val Val Gly Gly Gly Ser
                35                  40                  45

ggt ggc ctg gct tgt gcc aag gag gcc gcc cag ctg gga agg aag gtg  195
Gly Gly Leu Ala Cys Ala Lys Glu Ala Ala Gln Leu Gly Arg Lys Val
            50                  55                  60

gcc gtg gtg gac tac gtg gaa cct tct ccc caa ggc acc cgg tgg ggc  243
Ala Val Val Asp Tyr Val Glu Pro Ser Pro Gln Gly Thr Arg Trp Gly
        65                  70                  75

ctc ggc ggc acc tgc gtc aac gtg ggc tgc atc ccc aag aag ctg atg  291
Leu Gly Gly Thr Cys Val Asn Val Gly Cys Ile Pro Lys Lys Leu Met
    80                  85                  90

cac cag gcg gca ctg ctg gga ggc ctg atc caa gat gcc ccc aac tat  339
His Gln Ala Ala Leu Leu Gly Gly Leu Ile Gln Asp Ala Pro Asn Tyr
95                  100                 105                 110

ggc tgg gag gtg gcc cag ccc gtg ccg cat gac tgg agg aag atg gca  387
Gly Trp Glu Val Ala Gln Pro Val Pro His Asp Trp Arg Lys Met Ala
                115                 120                 125
```

gaa gct gtt caa aat cac gtg aaa tcc ttg aac tgg ggc cac cgt gtc 435
Glu Ala Val Gln Asn His Val Lys Ser Leu Asn Trp Gly His Arg Val
130 135 140

cag ctt cag gac aga aaa gtc aag tac ttt aac atc aaa gcc agc ttt 483
Gln Leu Gln Asp Arg Lys Val Lys Tyr Phe Asn Ile Lys Ala Ser Phe
145 150 155

gtt gac gag cac acg gtt tgc ggc gtt gcc aaa ggt ggg aaa gag att 531
Val Asp Glu His Thr Val Cys Gly Val Ala Lys Gly Gly Lys Glu Ile
160 165 170

ctg ctg tca gcc gat cac atc atc att gct act gga ggg cgg ccg aga 579
Leu Leu Ser Ala Asp His Ile Ile Ile Ala Thr Gly Gly Arg Pro Arg
175 180 185 190

tac ccc acg cac atc gaa ggt gcc ttg gaa tat gga atc aca agt gat 627
Tyr Pro Thr His Ile Glu Gly Ala Leu Glu Tyr Gly Ile Thr Ser Asp
195 200 205

gac atc ttc tgg ctg aag gaa tcc cct gga aaa acg ttg gtg gtc ggg 675
Asp Ile Phe Trp Leu Lys Glu Ser Pro Gly Lys Thr Leu Val Val Gly
210 215 220

gcc agc tat gtg gcc ctg gag tgt gct ggc ttc ctc acc ggg att ggg 723
Ala Ser Tyr Val Ala Leu Glu Cys Ala Gly Phe Leu Thr Gly Ile Gly
225 230 235

ctg gac acc acc atc atg atg cgc agc atc ccc ctc cgc ggc ttc gac 771
Leu Asp Thr Thr Ile Met Met Arg Ser Ile Pro Leu Arg Gly Phe Asp
240 245 250

cag caa atg tcc tcc atg gtc ata gag cac atg gca tct cat ggc acc 819
Gln Gln Met Ser Ser Met Val Ile Glu His Met Ala Ser His Gly Thr
255 260 265 270

```
cgg ttc ctg agg ggc tgt gcc ccc tcg cgg gtc agg agg ctc cct gat    867
Arg Phe Leu Arg Gly Cys Ala Pro Ser Arg Val Arg Arg Leu Pro Asp
                275                 280                 285

ggc cag ctg cag gtc acc tgg gag gac agc acc acc ggc aag gag gac    915
Gly Gln Leu Gln Val Thr Trp Glu Asp Ser Thr Thr Gly Lys Glu Asp
            290                 295                 300

acg ggc acc ttt gac acc gtc ctg tgg gcc ata ggt cga gtc cca gac    963
Thr Gly Thr Phe Asp Thr Val Leu Trp Ala Ile Gly Arg Val Pro Asp
        305                 310                 315

acc aga agt ctg aat ttg gag aag gct ggg gta gat act agc ccc gac    1011
Thr Arg Ser Leu Asn Leu Glu Lys Ala Gly Val Asp Thr Ser Pro Asp
    320                 325                 330

act cag aag atc ctg gtg gac tcc cgg gaa gcc acc tct gtg ccc cac    1059
Thr Gln Lys Ile Leu Val Asp Ser Arg Glu Ala Thr Ser Val Pro His
335                 340                 345                 350

atc tac gcc att ggt gac gtg gtg gag ggg cgg cct gag ctg aca ccc    1107
Ile Tyr Ala Ile Gly Asp Val Val Glu Gly Arg Pro Glu Leu Thr Pro
                355                 360                 365

aca gcg atc atg gcc ggg agg ctc ctg gtg cag cgg ctc ttc ggc ggg    1155
Thr Ala Ile Met Ala Gly Arg Leu Leu Val Gln Arg Leu Phe Gly Gly
            370                 375                 380

tcc tca gat ctg atg gac tac gac aat gtt ccc acg acc gtc ttc acc    1203
Ser Ser Asp Leu Met Asp Tyr Asp Asn Val Pro Thr Thr Val Phe Thr
        385                 390                 395

cca ctg gag tat ggc tgt gtg ggg ctg tcc gag gag gag gca gtg gct    1251
Pro Leu Glu Tyr Gly Cys Val Gly Leu Ser Glu Glu Glu Ala Val Ala
    400                 405                 410
```

cgc cac ggg cag gag cat gtt gag gtc tat cac gcc cat tat aaa cca 1299
Arg His Gly Gln Glu His Val Glu Val Tyr His Ala His Tyr Lys Pro
415 420 425 430

ctg gag ttc acg gtg gct gga cga gat gca tcc cag tgt tat gta aag 1347
Leu Glu Phe Thr Val Ala Gly Arg Asp Ala Ser Gln Cys Tyr Val Lys
435 440 445

atg gtg tgc ctg agg gag ccc cca cag ctg gtg ctg ggc ctg cat ttc 1395
Met Val Cys Leu Arg Glu Pro Pro Gln Leu Val Leu Gly Leu His Phe
450 455 460

ctt ggc ccc aac gca ggc gaa gtt act caa gga ttt gct ctg ggg atc 1443
Leu Gly Pro Asn Ala Gly Glu Val Thr Gln Gly Phe Ala Leu Gly Ile
465 470 475

aag tgt ggg gct tcc tat gcg cag gtg atg cgg acc gtg ggt atc cat 1491
Lys Cys Gly Ala Ser Tyr Ala Gln Val Met Arg Thr Val Gly Ile His
480 485 490

ccc aca tgc tct gag gag gta gtc aag ctg cgc atc tcc aag cgc tca 1539
Pro Thr Cys Ser Glu Glu Val Val Lys Leu Arg Ile Ser Lys Arg Ser
495 500 505 510

ggc ctg gac ccc acg gtg aca ggc tgc tga ggg taagcgccat ccctgcaggc 1592
Gly Leu Asp Pro Thr Val Thr Gly Cys Xaa Gly
515 520

cagggcacac ggtgcgcccg ccgccagctc ctcggaggcc agacccagga tggctgcagg 1652

ccaggtttgg ggggcctcaa ccctctcctg gagcgcctgt gagatggtca gcgtggagcg 1712

caagtgctgg acgggtggcc cgtgtgcccc acagggatgg ctcaggggac tgtccacctc 1772

acccctgcac ctttcagcct ttgccgccgg gcaccccccc caggctcctg gtgccggatg 1832

atgacgacct gggtggaaac ctaccctgtg ggcacccatg tccgagcccc ctggcatttc 1892

tgcaatgcaa ataaagaggg tactttttct gaagtgtgta aaaaaaaaaa aaaaaaaaaa 1952

aaaaaaa 1959

<210> 2
<211> 521
<212> PRT
<213> Homo sapiens
<223> Xaa(520) means selenosysteine.

<400> 2
Met Ala Val Ala Leu Arg Gly Leu Gly Gly Arg Phe Arg Trp Arg Thr
1 5 10 15

Gln Ala Val Ala Gly Gly Val Arg Gly Ala Ala Arg Gly Ala Ala Ala
20 25 30

Gly Gln Arg Asp Tyr Asp Leu Leu Val Val Gly Gly Gly Ser Gly Gly
35 40 45

Leu Ala Cys Ala Lys Glu Ala Ala Gln Leu Gly Arg Lys Val Ala Val
50 55 60

Val Asp Tyr Val Glu Pro Ser Pro Gln Gly Thr Arg Trp Gly Leu Gly
65 70 75 80

Gly Thr Cys Val Asn Val Gly Cys Ile Pro Lys Lys Leu Met His Gln
85 90 95

Ala Ala Leu Leu Gly Gly Leu Ile Gln Asp Ala Pro Asn Tyr Gly Trp
100 105 110

Glu Val Ala Gln Pro Val Pro His Asp Trp Arg Lys Met Ala Glu Ala

115 120 125

Val Gln Asn His Val Lys Ser Leu Asn Trp Gly His Arg Val Gln Leu
130 135 140

Gln Asp Arg Lys Val Lys Tyr Phe Asn Ile Lys Ala Ser Phe Val Asp
145 150 155 160

Glu His Thr Val Cys Gly Val Ala Lys Gly Gly Lys Glu Ile Leu Leu
165 170 175

Ser Ala Asp His Ile Ile Ile Ala Thr Gly Gly Arg Pro Arg Tyr Pro
180 185 190

Thr His Ile Glu Gly Ala Leu Glu Tyr Gly Ile Thr Ser Asp Asp Ile
195 200 205

Phe Trp Leu Lys Glu Ser Pro Gly Lys Thr Leu Val Val Gly Ala Ser
210 215 220

Tyr Val Ala Leu Glu Cys Ala Gly Phe Leu Thr Gly Ile Gly Leu Asp
225 230 235 240

Thr Thr Ile Met Met Arg Ser Ile Pro Leu Arg Gly Phe Asp Gln Gln
245 250 255

Met Ser Ser Met Val Ile Glu His Met Ala Ser His Gly Thr Arg Phe
260 265 270

Leu Arg Gly Cys Ala Pro Ser Arg Val Arg Arg Leu Pro Asp Gly Gln
275 280 285

Leu Gln Val Thr Trp Glu Asp Ser Thr Thr Gly Lys Glu Asp Thr Gly
290 295 300

Thr Phe Asp Thr Val Leu Trp Ala Ile Gly Arg Val Pro Asp Thr Arg

305 310 315 320

Ser Leu Asn Leu Glu Lys Ala Gly Val Asp Thr Ser Pro Asp Thr Gln
325 330 335

Lys Ile Leu Val Asp Ser Arg Glu Ala Thr Ser Val Pro His Ile Tyr
340 345 350

Ala Ile Gly Asp Val Val Glu Gly Arg Pro Glu Leu Thr Pro Thr Ala
355 360 365

Ile Met Ala Gly Arg Leu Leu Val Gln Arg Leu Phe Gly Gly Ser Ser
370 375 380

Asp Leu Met Asp Tyr Asp Asn Val Pro Thr Thr Val Phe Thr Pro Leu
385 390 395 400

Glu Tyr Gly Cys Val Gly Leu Ser Glu Glu Glu Ala Val Ala Arg His
405 410 415

Gly Gln Glu His Val Glu Val Tyr His Ala His Tyr Lys Pro Leu Glu
420 425 430

Phe Thr Val Ala Gly Arg Asp Ala Ser Gln Cys Tyr Val Lys Met Val
435 440 445

Cys Leu Arg Glu Pro Pro Gln Leu Val Leu Gly Leu His Phe Leu Gly
450 455 460

Pro Asn Ala Gly Glu Val Thr Gln Gly Phe Ala Leu Gly Ile Lys Cys
465 470 475 480

Gly Ala Ser Tyr Ala Gln Val Met Arg Thr Val Gly Ile His Pro Thr
485 490 495

Cys Ser Glu Glu Val Val Lys Leu Arg Ile Ser Lys Arg Ser Gly Leu

500 505 510

Asp Pro Thr Val Thr Gly Cys Xaa Gly
515 520

<210> 3
<211> 2056
<212> DNA
<213> Homo sapiens

<220>
<221> CDS
<222> (188)..(1669)
<223> tga(1664)..(1666) is transrated to selenosysteine, shown by Xaa.

<400> 3
gtcccggacc tcaggcccag ttcagtgtac ttcccctctc tacttcctcc ctccagtccc 60

ttctccatcc ctccctttt tggctgcccc ttgcctgcct tcctcgccag tagcttgcag 120

agtagacacg atgacacctt ttgcaggcta aaaaggctga gagtggcact atgtgcagtg 180

agccacc atg gag gac caa gca ggt cag cgg gac tat gat ctc ctg gtg 229
Met Glu Asp Gln Ala Gly Gln Arg Asp Tyr Asp Leu Leu Val
1 5 10

gtc ggc ggg gga tct ggt ggc ctg gct tgt gcc aag gag gcc gcc cag 277
Val Gly Gly Gly Ser Gly Gly Leu Ala Cys Ala Lys Glu Ala Ala Gln
15 20 25 30

ctg gga agg aag gtg gcc gtg gtg gac tac gtg gaa cct tct ccc caa 325
Leu Gly Arg Lys Val Ala Val Val Asp Tyr Val Glu Pro Ser Pro Gln
35 40 45

ggc acc cgg tgg ggc ctc ggc ggc acc tgc gtc aac gtg ggc tgc atc 373

Gly Thr Arg Trp Gly Leu Gly Gly Thr Cys Val Asn Val Gly Cys Ile
50 55 60

ccc aag aag ctg atg cac cag gcg gca ctg ctg gga ggc ctg atc caa 421
Pro Lys Lys Leu Met His Gln Ala Ala Leu Leu Gly Gly Leu Ile Gln
65 70 75

gat gcc ccc aac tat ggc tgg gag gtg gcc cag ccc gtg ccg cat gac 469
Asp Ala Pro Asn Tyr Gly Trp Glu Val Ala Gln Pro Val Pro His Asp
80 85 90

tgg agg aag atg gca gaa gct gtt caa aat cac gtg aaa tcc ttg aac 517
Trp Arg Lys Met Ala Glu Ala Val Gln Asn His Val Lys Ser Leu Asn
95 100 105 110

tgg ggc cac cgt gtc cag ctt cag gac aga aaa gtc aag tac ttt aac 565
Trp Gly His Arg Val Gln Leu Gln Asp Arg Lys Val Lys Tyr Phe Asn
115 120 125

atc aaa gcc agc ttt gtt gac gag cac acg gtt tgc ggc gtt gcc aaa 613
Ile Lys Ala Ser Phe Val Asp Glu His Thr Val Cys Gly Val Ala Lys
130 135 140

ggt ggg aaa gag att ctg ctg tca gcc gat cac atc atc att gct act 661
Gly Gly Lys Glu Ile Leu Leu Ser Ala Asp His Ile Ile Ile Ala Thr
145 150 155

gga ggg cgg ccg aga tac ccc acg cac atc gaa ggt gcc ttg gaa tat 709
Gly Gly Arg Pro Arg Tyr Pro Thr His Ile Glu Gly Ala Leu Glu Tyr
160 165 170

gga atc aca agt gat gac atc ttc tgg ctg aag gaa tcc cct gga aaa 757
Gly Ile Thr Ser Asp Asp Ile Phe Trp Leu Lys Glu Ser Pro Gly Lys
175 180 185 190

acg ttg gtg gtc ggg gcc agc tat gtg gcc ctg gag tgt gct ggc ttc 805

Thr Leu Val Val Gly Ala Ser Tyr Val Ala Leu Glu Cys Ala Gly Phe
195 200 205

ctc acc ggg att ggg ctg gac acc acc atc atg atg cgc agc atc ccc 853
Leu Thr Gly Ile Gly Leu Asp Thr Thr Ile Met Met Arg Ser Ile Pro
210 215 220

ctc cgc ggc ttc gac cag caa atg tcc tcc atg gtc ata gag cac atg 901
Leu Arg Gly Phe Asp Gln Gln Met Ser Ser Met Val Ile Glu His Met
225 230 235

gca tct cat ggc acc cgg ttc ctg agg ggc tgt gcc ccc tcg cgg gtc 949
Ala Ser His Gly Thr Arg Phe Leu Arg Gly Cys Ala Pro Ser Arg Val
240 245 250

agg agg ctc cct gat ggc cag ctg cag gtc acc tgg gag gac agc acc 997
Arg Arg Leu Pro Asp Gly Gln Leu Gln Val Thr Trp Glu Asp Ser Thr
255 260 265 270

acc ggc aag gag gac acg ggc acc ttt gac acc gtc ctg tgg gcc ata 1045
Thr Gly Lys Glu Asp Thr Gly Thr Phe Asp Thr Val Leu Trp Ala Ile
275 280 285

ggt cga gtc cca gac acc aga agt ctg aat ttg gag aag gct ggg gta 1093
Gly Arg Val Pro Asp Thr Arg Ser Leu Asn Leu Glu Lys Ala Gly Val
290 295 300

gat act agc ccc gac act cag aag atc ctg gtg gac tcc cgg gaa gcc 1141
Asp Thr Ser Pro Asp Thr Gln Lys Ile Leu Val Asp Ser Arg Glu Ala
305 310 315

acc tct gtg ccc cac atc tac gcc att ggt gac gtg gtg gag ggg cgg 1189
Thr Ser Val Pro His Ile Tyr Ala Ile Gly Asp Val Val Glu Gly Arg
320 325 330

cct gag ctg aca ccc aca gcg atc atg gcc ggg agg ctc ctg gtg cag 1237

```
Pro Glu Leu Thr Pro Thr Ala Ile Met Ala Gly Arg Leu Leu Val Gln
335                 340                 345               · 350

cgg ctc ttc ggc ggg tcc tca gat ctg atg gac tac gac aat gtt ccc    1285
Arg Leu Phe Gly Gly Ser Ser Asp Leu Met Asp Tyr Asp Asn Val Pro
                355                 360                 365

acg acc gtc ttc acc cca ctg gag tat ggc tgt gtg ggg ctg tcc gag    1333
Thr Thr Val Phe Thr Pro Leu Glu Tyr Gly Cys Val Gly Leu Ser Glu
            370                 375                 380

gag gag gca gtg gct cgc cac ggg cag gag cat gtt gag gtc tat cac    1381
Glu Glu Ala Val Ala Arg His Gly Gln Glu His Val Glu Val Tyr His
        385                 390                 395

gcc cat tat aaa cca ctg gag ttc acg gtg gct gga cga gat gca tcc    1429
Ala His Tyr Lys Pro Leu Glu Phe Thr Val Ala Gly Arg Asp Ala Ser
    400                 405                 410

cag tgt tat gta aag atg gtg tgc ctg agg gag ccc cca cag ctg gtg    1477
Gln Cys Tyr Val Lys Met Val Cys Leu Arg Glu Pro Pro Gln Leu Val
415                 420                 425                 430

ctg ggc ctg cat ttc ctt ggc ccc aac gca ggc gaa gtt act caa gga    1525
Leu Gly Leu His Phe Leu Gly Pro Asn Ala Gly Glu Val Thr Gln Gly
                435                 440                 445

ttt gct ctg ggg atc aag tgt ggg gct tcc tat gcg cag gtg atg cgg    1573
Phe Ala Leu Gly Ile Lys Cys Gly Ala Ser Tyr Ala Gln Val Met Arg
            450                 455                 460

acc gtg ggt atc cat ccc aca tgc tct gag gag gta gtc aag ctg cgc    1621
Thr Val Gly Ile His Pro Thr Cys Ser Glu Glu Val Val Lys Leu Arg
        465                 470                 475

atc tcc aag cgc tca ggc ctg gac ccc acg gtg aca ggc tgc tga ggg    1669
```

Ile Ser Lys Arg Ser Gly Leu Asp Pro Thr Val Thr Gly Cys Xaa Gly
480 485 490

taagcgccat ccctgcaggc cagggcacac ggtgcgcccg ccgccagctc ctcggaggcc 1729

agacccagga tggctgcagg ccaggtttgg ggggcctcaa ccctctcctg gagcgcctgt 1789

gagatggtca gcgtggagcg caagtgctgg acgggtggcc cgtgtgcccc acagggatgg 1849

ctcaggggac tgtccacctc acccctgcac ctttcagcct ttgccgccgg gcaccccccc 1909

caggctcctg gtgccggatg atgacgacct gggtggaaac ctaccctgtg ggcacccatg 1969

tccgagcccc ctggcatttc tgcaatgcaa ataaagaggg tactttttct gaagtgtgta 2029

aaaaaaaaaa aaaaaaaaaa aaaaaaa 2056

<210> 4
<211> 492
<212> PRT
<213> Homo sapiens
<223> Xaa(493) means selenosysteine.

<400> 4
Met Glu Asp Gln Ala Gly Gln Arg Asp Tyr Asp Leu Leu Val Val Gly
1 5 10 15

Gly Gly Ser Gly Gly Leu Ala Cys Ala Lys Glu Ala Ala Gln Leu Gly
20 25 30

Arg Lys Val Ala Val Val Asp Tyr Val Glu Pro Ser Pro Gln Gly Thr
35 40 45

Arg Trp Gly Leu Gly Gly Thr Cys Val Asn Val Gly Cys Ile Pro Lys
50 55 60

Lys Leu Met His Gln Ala Ala Leu Leu Gly Gly Leu Ile Gln Asp Ala
65 70 75 80

Pro Asn Tyr Gly Trp Glu Val Ala Gln Pro Val Pro His Asp Trp Arg
85 90 95

Lys Met Ala Glu Ala Val Gln Asn His Val Lys Ser Leu Asn Trp Gly
100 105 110

His Arg Val Gln Leu Gln Asp Arg Lys Val Lys Tyr Phe Asn Ile Lys
115 120 125

Ala Ser Phe Val Asp Glu His Thr Val Cys Gly Val Ala Lys Gly Gly
130 135 140

Lys Glu Ile Leu Leu Ser Ala Asp His Ile Ile Ile Ala Thr Gly Gly
145 150 155 160

Arg Pro Arg Tyr Pro Thr His Ile Glu Gly Ala Leu Glu Tyr Gly Ile
165 170 175

Thr Ser Asp Asp Ile Phe Trp Leu Lys Glu Ser Pro Gly Lys Thr Leu
180 185 190

Val Val Gly Ala Ser Tyr Val Ala Leu Glu Cys Ala Gly Phe Leu Thr
195 200 205

Gly Ile Gly Leu Asp Thr Thr Ile Met Met Arg Ser Ile Pro Leu Arg
210 215 220

Gly Phe Asp Gln Gln Met Ser Ser Met Val Ile Glu His Met Ala Ser
225 230 235 240

His Gly Thr Arg Phe Leu Arg Gly Cys Ala Pro Ser Arg Val Arg Arg
245 250 255

Leu Pro Asp Gly Gln Leu Gln Val Thr Trp Glu Asp Ser Thr Thr Gly
260 265 270

Lys Glu Asp Thr Gly Thr Phe Asp Thr Val Leu Trp Ala Ile Gly Arg
275 280 285

Val Pro Asp Thr Arg Ser Leu Asn Leu Glu Lys Ala Gly Val Asp Thr
290 295 300

Ser Pro Asp Thr Gln Lys Ile Leu Val Asp Ser Arg Glu Ala Thr Ser
305 310 315 320

Val Pro His Ile Tyr Ala Ile Gly Asp Val Val Glu Gly Arg Pro Glu
325 330 335

Leu Thr Pro Thr Ala Ile Met Ala Gly Arg Leu Leu Val Gln Arg Leu
340 345 350

Phe Gly Gly Ser Ser Asp Leu Met Asp Tyr Asp Asn Val Pro Thr Thr
355 360 365

Val Phe Thr Pro Leu Glu Tyr Gly Cys Val Gly Leu Ser Glu Glu Glu
370 375 380

Ala Val Ala Arg His Gly Gln Glu His Val Glu Val Tyr His Ala His
385 390 395 400

Tyr Lys Pro Leu Glu Phe Thr Val Ala Gly Arg Asp Ala Ser Gln Cys
405 410 415

Tyr Val Lys Met Val Cys Leu Arg Glu Pro Pro Gln Leu Val Leu Gly
420 425 430

Leu His Phe Leu Gly Pro Asn Ala Gly Glu Val Thr Gln Gly Phe Ala
435 440 445

Leu Gly Ile Lys Cys Gly Ala Ser Tyr Ala Gln Val Met Arg Thr Val
450 455 460

Gly Ile His Pro Thr Cys Ser Glu Glu Val Val Lys Leu Arg Ile Ser
465 470 475 480

Lys Arg Ser Gly Leu Asp Pro Thr Val Thr Gly Cys Xaa Gly
485 490

<210> 5
<211> 130
<212> DNA
<213> Homo sapiens

<400> 5
tcagcctttg ccgccgggca cccccccag gctcctggtg ccggatgatg acgacctggg 60

tggaaaccta ccctgtgggc acccatgtcc gagccccctg gcatttctgc aatgcaaata 120

aagagggtac 130

<210> 6
<211> 32
<212> DNA
<213> Artificial Sequence

<220>
<223> Description of Artificial Sequence:Synthesis

<400> 6
gcgggatcca tgacttttaa cagttttgaa gg 32

<210> 7
<211> 32
<212> DNA
<213> Artificial Sequence

<220>
<223> Description of Artificial Sequence:Synthesis

<400> 7
gcgctcgagc tactatagag ttagattaag ac 32

<210> 8
<211> 18
<212> DNA
<213> Artificial Sequence

<220>
<223> Description of Artificial Sequence:synthesis

<400> 8
tatgatctcc tggtggtc 18

<210> 9
<211> 18
<212> DNA
<213> Artificial Sequence

<220>
<223> Description of Artificial Sequence:Synthesis

<400> 9
gtcatcactt gtgattcc 18

<210> 10
<211> 21
<212> DNA
<213> Artificial Sequence

<220>
<223> Description of Artificial Sequence:Synthesis

<400> 10
acagcttctg ccatcttcct c 21

<210> 11
<211> 21
<212> DNA
<213> Artificial Sequence

<220>
<223> Description of Artificial Sequence:Synthesis

<400> 11
agaaggttcc acgtagtcca c 21

<210> 12
<211> 21
<212> DNA
<213> Artificial Sequence

<220>
<223> Description of Artificial Sequence:Synthesis

<400> 12
ccatacgatg ttccagatta c 21

<210> 13
<211> 21
<212> DNA
<213> Artificial Sequence

<220>
<223> Description of Artificial Sequence:synthesis

<400> 13
acgatggcgg caatggcggt g 21

<210> 14
<211> 21
<212> DNA
<213> Artificial Sequence

<220>
<223> Description of Artificial Sequence:synthesis

<400> 14
accatggagg accaagcagg t 21

<210> 15
<211> 21
<212> DNA
<213> Artificial Sequence

<220>
<223> Description of Artificial Sequence:synthesis

<400> 15
ttaccctcag cagcctgtca c 21

<210> 16
<211> 21
<212> DNA
<213> Artificial Sequence

<220>
<223> Description of Artificial Sequence:synthesis

<400> 16
gcgccatccc tgcaggccag g 21

<210> 17
<211> 21
<212> DNA
<213> Artificial Sequence

<220>
<223> Description of Artificial Sequence:synthesis

<400> 17
cacacttcag aaaaagtacc c 21

<210> 18
<211> 103
<212> DNA
<213> Homo sapiens

<400> 18
atggcggcaa tggcggtggc gctgcgggga ttaggagggc gcttccggtg gcggacgcag 60

gccgtggcgg gcggggtgcg gggcgcggcg cggggcgcag cag 103

<210> 19

<211> 200
<212> DNA
<213> Homo sapiens

<400> 19
gtcccggacc tcaggcccag ttcagtgtac ttcccctctc tacttcctcc ctccagtccc 60

ttctccatcc ctcccttttt tggctgcccc ttgcctgcct tcctcgccag tagcttgcag 120

agtagacacg atgacacctt ttgcaggcta aaaaggctga gagtggcact atgtgcagtg 180

agccaccatg gaggaccaag 200

<210> 20
<211> 69
<212> DNA
<213> Homo sapiens

<400> 20
caggtcagcg ggactatgat ctcctggtgg tcggcggggg atctggtggc ctggcttgtg 60

ccaaggagg 69

<210> 21
<211> 57
<212> DNA
<213> Homo sapiens

<400> 21
ccgcccagct gggaaggaag gtggtggtgg tggactacgt ggaaccttct ccccaag 57

<210> 22
<211> 145

<212> DNA
<213> Homo sapiens

<400> 22
gcacccggtg gggcctcggc ggcacctgcg tcaacgtggg ctgcatcccc aagaagctga 60

tgcaccaggc ggcactgctg ggaggcctga tccaagatgc ccccaactat ggctgggagg 120

tggcccagcc cgtgccgcat gactg 145

<210> 23
<211> 75
<212> DNA
<213> Homo sapiens

<400> 23
gaggaagatg gcagaagctg ttcaaaatca cgtgaaatcc ttgaactggg gccaccgtgt 60

ccagcttcag gacag 75

<210> 24
<211> 79
<212> DNA
<213> Homo sapiens

<400> 24
aaaagtcaag tactttaaca tcaaagccag ctttgttgac gagcacacgg tttgcggcgt 60

tgccaaaggt gggaaagag 79

<210> 25
<211> 63
<212> DNA

<213> Homo sapiens

<400> 25
attctgctgt cagccgatca catcatcatt gctactggag ggcggccgag ataccccacg 60

cac 63

<210> 26
<211> 71
<212> DNA
<213> Homo sapiens

<400> 26
atcgaaggtg ccttggaata tggaatcaca agtgatgaca tcttctggct gaaggaatcc 60

cctggaaaaa c 71

<210> 27
<211> 20
<212> DNA
<213> Homo sapiens

<400> 27
gttggtggtc ggggccagct 20

<210> 28
<211> 92
<212> DNA
<213> Homo sapiens

<400> 28
atgtggccct ggagtgtgct ggcttcctca ccgggattgg gctggacacc accatcatga 60

tgcgcagcat ccccctccgc ggcttcgacc ag 92

<210> 29
<211> 175
<212> DNA
<213> Homo sapiens

<400> 29
caaatgtcct ccatggtcat agagcacatg gcatctcatg gcacccggtt cctgaggggc 60

tgtgccccct cgcgggtcag gaggctccct gatggccagc tgcaggtcac ctgggaggac 120

agcaccaccg gcaaggagga cacgggcacc tttgacaccg tcctgtgggc catag 175

<210> 30
<211> 137
<212> DNA
<213> Homo sapiens

<400> 30
gtcgagtccc agacaccaga agtctgaatt tggagaaggc tggggtagat actagccccg 60

acactcagaa gatcctggtg gactcccggg aagccacctc tgtgccccac atctacgcca 120

ttggtgacgt ggtggag 137

<210> 31
<211> 96
<212> DNA
<213> Homo sapiens

<400> 31
gggcggcctg agctgacacc cacagcgatc atggccggga ggctcctggt gcagcggctc 60

ttcggcgggt cctcagatct gatggactac gacaat 96

<210> 32
<211> 93
<212> DNA
<213> Homo sapiens

<400> 32
gttcccacga ccgtcttcac cccactggag tatggctgtg tggggctgtc cgaggaggag 60

gcagtggctc gccacgggca ggagcatgtt gag 93

<210> 33
<211> 72
<212> DNA
<213> Homo sapiens

<400> 33
gtctatcacg cccattataa accactggag ttcacggtgg ctggacgaga tgcatcccag 60

tgttatgtaa ag 72

<210> 34
<211> 98
<212> DNA
<213> Homo sapiens

<400> 34
atggtgtgcc tgagggagcc cccacagctg gtgctgggcc tgcatttcct tggccccaac 60

gcaggcgaag ttactcaagg atttgctctg gggatcaa 98

<210> 35
<211> 195
<212> DNA
<213> Homo sapiens

<400> 35
gtgtggggct tcctatgcgc aggtgatgcg gaccgtgggt atccatccca catgctctga 60

ggaggtagtc aagctgcgca tctccaagcg ctcaggcctg gaccccacgg tgacaggctg 120

ctgagggtaa gcgccatccc tgcaggccag ggcacacggt gcgcccgccg ccagctcctc 180

ggaggccaga cccag 195

<210> 36
<211> 290
<212> DNA
<213> Homo sapiens

<400> 36
gatggctgca ggccaggttt ggggggcctc aaccctctcc tggagcgcct gtgagatggt 60

cagcgtggag cgcaagtgct ggacgggtgg cccgtgtgcc ccacagggat ggctcagggg 120

actgtccacc tcacccctgc acctttcagc ctttgccgcc gggcaccccc cccaggctcc 180

tggtgccgga tgatgacgac ctgggtggaa acctaccctg tgggcaccca tgtccgagcc 240

ccctggcatt tctgcaatgc aaataaagag ggtacttttt ctgaagtgtg 290

<210> 37
<211> 66566
<212> DNA

<213> Homo sapiens

<400> 37

atggcggcaa tggcggtggc gctgcgggga ttaggagggc gcttccggtg gcggacgcag 60
gccgtggcgg gcggggtgcg gggcgcggcg cggggcgcag caggtaggat ggggtcgggg 120
cgtccccgcg gtaggtgtcc gcgcggccgg ggtgtcctcg tgagggtgtc cgcgcggcgg 180
tggccagggt gtccccgtgg gggtgcccac gcgggggtgt ccacataccg gcctcttggt 240
ctagtcttgc tcaggagtcc gggctgcttc tagccacaag tagccccctt cccaggtggg 300
gaaactgggg ctgggtgcct tgtctaaggt cctgctgtgc tgactgcacc tgtggtctcc 360
cagagctggt atcccagtaa caactacagt tctgaagatg atgatatccc acctccccgag 420
gtcaccaggc accggcccca ctggccagac ttcccaactt ctccccagac ccctagactc 480
tagaggttag aggctgcaca gagcaatggg aggatacaca ctcgtcctcc tggagcccct 540
gaagaacagt taactaaatc aggacaataa tcataactga gcactcgaag cagaggctgg 600
gtgtctggtc actcaggaca gttcaagcct catcctgtag gacagactcc cctagatccg 660
accagagcgc caccatatct gttatgtgtg gccagtttca ttccatgcac gacaacatgg 720
tcccccacca tgcagggggc ccctcgaccc agccccctgg atgcttgtga cagcgagcag 780
ctctccccac aggcagtgag tgtagagggg tgtaaggacg gggtcagggc tccttcccag 840
ggatggcggc tatgggaggc atggtggctg gccctctgcc cgcggtggac tcgggaggga 900
gggctgactg tgtgtgtgaa tgggcagagt tggtgctatg gaggttttgg gggtctccag 960
gacggagggt ggcccaacag agttctggga ggcagtcacc acctcgtggc cttgctgaga 1020

cctggaaccc tcagccaggg cactccatct ttcaaagctt cttggctgca tgcgtcaggt 1080

gggcaagctc aggaaggtta aatgcacccg tgctggcgga gtcccataaa aggggattcg 1140

gcatcaaaag gaggaaaaag gttcaaaggg catttatcat ggggttcaga atcacggatg 1200

tgaggggcgg tagtggggac aacagacaga aaagcttccc cttcccatac tcacagtcca 1260

gacacggcaa tagccaaatt ccaaatttct aggtattctg gactcagaat ggggaatatc 1320

atacgagact tagggggata atgcccttat cttcctattt taagggaaag aacaaactga 1380

accttctatg caaaatagga tgatgatcct ggtcctccca gtaagaaata aaataagtag 1440

tctccaggca ttcctttccg ccagaggagc aactgttttt taaatagccc tttcgtgccc 1500

agtctgttac taaaccatat gagttgtttt tttggggttt tttttttttt ttttttttg 1560

agacagtgtc ttgctctgtc gcccaagctg gagtacagtg gtgcgatctc agctcactgc 1620

aagctccgcc tcccgggttc acgccattct cctgcctcag cctcccgagt agctgggact 1680

acaggcatct gccaccacgc ctggctaatt tttttgtat ttttagtaga gacgggattt 1740

cactgtatta gccaggatgg tctcaatctc ctgacctcat gatccacctg ccttggcctc 1800

ccaaagtgct gggattacag gcatgagcca ctgcgcccag ccgagtcatt ttttaatact 1860

actgcatgtg agttaacaca atcattccca aattgaagtt ttagatgggc cctcaaaatt 1920

tttaggatat ggttttccta caggtttata ttgaaagtat ggggtatctc ctattactcc 1980

tctttttatt tgtcttaaag gagaaaggga gaggccagag accaaatgtc cccatttccc 2040

tatagctaat ctctctggaa gacaagcagc ccagacttga gcttctagat ggatacaacc 2100

aggtgcatgt ccaaggcaca gaggagggta tttataaccc atagtaacat taaatgcagt 2160

gccttctcct ggctgagcgg tgcaacggtc atctgtagtt ccaggcatcc acacactatc 2220

gttagtatag atttctgcag gagcatccat ccaggtgaga ggtcgaataa gtggaggaaa 2280

aggcacataa gcccaataag aataattttg tgtagcaggt aaatcagttt aaggggaaac 2340

tggtgagaca gaaagtgtaa ggaagataat tattaaataa aacctattgt aagtgagatc 2400

cagtgctgaa ggaggaagag aagaacagag ggatgttatt ttcaggctaa tagaaatggt 2460

gagattttta ggttcgtaag gagaaaaaga taattaggag aagtgggatt agttagaggg 2520

gtttacattg ccattaggga ggattgaacc agacccattt tgatttggca tgccagtttc 2580

tgaggagtcg gtacagatct catcaggtat gagggcagtc tctgacgcga acgtctcttc 2640

ctcgtggttt ttattgtcag tattcacacg aagtttaagt ctcctagtgg gcacccagac 2700

gggattgacg atctcctggt aaaacacaag cataccctct tccccacgtt ataattgttc 2760

caggttccca ggtattggtt tgggagtttt tccatgacac tggcttgcct tcgtttaggg 2820

agaatttttt gcctgtataa tggcatttag ctgcagtcag agtattgttt ttaggaacat 2880

ttagaaagct taaacaatgc taaatataat tgggagtggg gagtagttaa attatgcttt 2940

taaaccagcc ttgtcttctt ttacagtaac ttgaagaggt ttagtaattt tttcacgttt 3000

tggaccgaga ccgagtctgg aaacaaaccc catgttttcc attatatgtt gactgggagc 3060

actgtaaaag ttatgtggaa tattaatttc agccccaatt tgtgccagca aatctctgcc 3120

ccgaagatta atggggatgg gcatgatata aggctgaatt gttccctttt gaccatcagg 3180

gccagtgcaa gccaagataa atgtgctctg gtgaacttct tcagcttttc taacaccttc 3240

tagtttcatg ttagtgggat gtttaagcca ggaggaaggc cataaattag aggaaataat 3300

agaaacatca gccccagtat caactaggcc ctcaaccttt tttccttgaa tgtgtatggt 3360

gcaggtaggc cattgtttag aaattacatt aatccaataa gcggcttttt caccgctgga 3420

gcccatccca gggccccatg tcttatctcc tttgtttaaa acaatgttag gtagtaaaag 3480

taattgagca attgactcac tggccggaat ggaaacagga actttggcag acaccataag 3540

tttacataag tttaatctca tcagaggaat cagaattaat gagactagta tgaactatga 3600

tacctttagt atcatgaggt ggatgccctg cctaacacca ggcccaccga accttgaggt 3660

aaagggccag tgacccccat ggggacaatt aaaggcaaag aattaggtag taaatttaga 3720

ggaatggtac tacagagatc gaccgccctg ctgcctactg tggaggtaga caagcattgt 3780

actgagacag aagaagaggc tgggacccat ctggattggc tgtaggtaaa ttttttttgtg 3840

ctgggggctg cgttgggact gcttgacgca aaaacacaac attggtctga gtccgaggtg 3900

tcccatttga tattggggcc tgggactggc cttgcttcct gtttccctgg ttctgtggca 3960

agggattttc atctatatca gactgagtgg caagtacttg cccaatgttt acccttacga 4020

caacgcaggc aaacagtagc aggagcattt ggccgtgttt gttgagccgg cttggccgct 4080

tttaagtttt taacagtgta gtttttttgg gtatgaccaa gttggccaca attatagcag 4140

gctccaagaa aagaatcagt cgagccagtt tggttgccgt gcttcatggc cggtgcccac 4200

agaatagctc tgtgggtctc cgatccaacg ccttcacaag ctttaatata tgcaggcaac 4260

acctcatgat caggtaaatt ttgacgtagg atggaatgca cggccatttt acactcatgg 4320

ttcgcatttt gaaaagctaa catatgaaga agaatacctt gagtgtgctc atcagagaca 4380

gatttttcaa cagcatcttg taatttagct aaaaaatcaa gatataattc attgtgaccc 4440

tgtttaacaa tagtaaaaga aacagaagct tggcctgggg tgcgtaattt atcccaagct 4500

ctcatacaca cctttgttac ttgttccgtg gtgagagcat caaagcctaa atgagcagta 4560

gtatcagagt aattatcgga gcctgtgagc tgagcctgag taattggaat gccatcagcc 4620

cgatttagct gagcctgcag gcgggcctcc tctgaccacc aggtatggaa ttgtaaatgc 4680

tgagatggag ttagaacagc ttttgccaaa aggtcgcagt ctataggaag caaaatgacc 4740

tcggtacaaa gagtctgtaa tacgttttaa catatggaga agtaggaaca tactgagtac 4800

aagcatcctt gaattctttt aaaaaggtaa gattgagcgg cgtatatcga cacacttgta 4860

ccccttgagc attaggaggt tccagcatga ccgaataagc ccacgcctct aatctacttg 4920

tttgttttgg cgtaataagc attgcatgaa agtttcaaga gcaggcacat gagatagagt 4980

cggcatagaa gtgacaggaa aagtatgtat agataagaga aactgagatt gacggggtcg 5040

gactggtata agagtgtgag aaaggggcat tgggggagca gaagaggcag aagcatactg 5100

gtgattactg gcccagtcct gcacaaccag agtggcaggg gtgtccctgc atgaagaagg 5160

gtacagagaa gcaggttgag tggatggcaa agtgaccggt tgagggacca aaatgatagg 5220

agggtgaggg gctgtggtgg atgggggagg gcctgcagaa ttacaggtaa attgtagttt 5280

ggatggctcc ggaggcaaag actgtaaagg tttggagagg gaagagttag catagatatg 5340

gtcctgggct gtctgagttg gggccgcagg agctacaagt gtcggctctt cgtgaaaaga 5400

aataagatca tcagggggtg atgttaagcc aaagtcactg gagttagata ttgaatcctc 5460

agtatcgtca ggtgggggag gagtaggcaa agggaggggc tgagcagata atgaaggccg 5520

agtgggagag gaaagctgag gaagaggtag agggtggcca gattcagaaa actgtggtaa 5580

ctgcagggtg tcacgggatt ggtatgtcat taggatggca cgtaccaagg cccaatcacc 5640

ccaaacagtg acaggaacat aatttcctgt tgggaccagt tctcggaatt ttgcaccaac 5700

atgatcccat agttccacat ctaacgttcc cttttcagga aaccaaggac agtgttcttc 5760

cactgccctg aatagggtga ccatattttc catgggaacc cgaactcccc cattttaaca 5820

ggaatttaat atagcagaga taagcataat gtttagactc tgcgtgaccc atagttaccc 5880

cggagaatac acagacaact caccaatcgt tggggagcca aacaagcatt tctgtggact 5940

ggaccgatga acatttctcc gcacctacca aagggaatcg ggttcccaca tgcacttagg 6000

aaaaagaaaa ccacgttggg cgccagatat tgggggaacc tgcccctaat atttcaacgt 6060

acgttctttc tattttctgt aagtgtcagc cggctgagaa atgaagagaa agagtacaaa 6120

gaggaatttt acagctgggc tgctgggggt gacatcacgt atcggtagga ccatgatgcc 6180

cacctgagcc gcaaaaccag caagttttta ttaaggattt taaaagggga ggggttgtgc 6240

caatagggag taggtcacaa agatcacatg cttcaaaggg caaaaggcag agcaaagatc 6300

acatgcttct gaggaaacag gacaagggca aaatcagaac tcctgataag ggtctatgtt 6360

cagctgtgca catattgtct tgataaacat cttaaacaac ggaaaacatg gtttaagagc 6420

agagaaccag tctgaccaca aatttaccag gacggagttt tttccccacc ctaataagcc 6480

tgagggtact gcaggagacc agggcgtatt tcagtcctta tctcaaccgc ataagacaga 6540

cactcccaga gtggccgttt acagacctcc ccccaggaat gcattccttt tccagggtct 6600

taatagtaat attccttgct aggaaaagaa tttagcgata tctctcctac ttgcacgtcc 6660

atttataagc tctctgcaag aagaaaaata tggctctttt tgcctgaccc cacaggcagt 6720

cagaccttat ggttgtcttc gttccctaaa aatcactgtt attctgttct ttttcaaggt 6780

gcgctgattt catattgttc aaacacacgt tttacaatca atttctacag ttaacacaat 6840

tatcacagtg gtcctgaggt gatgtatatt atcagcttat gaagataaca ggattaagag 6900

ataaagacag gcataagaaa ttataaaagt attacttggg ggcccaggcg cggtggctca 6960

tgcctgtaat cctagcactt tgagaggccg aggcagccat atcacaaggt gaggagatca 7020

agaccatcct ggctaacatg gtgaaaccct gtctctcctg aaagtacaaa aaattagcca 7080

ggtgtggtgg cgggtgcctg tagtcccagc tactcaggag gctgagacag gagaatggtg 7140

tgaacccagg aggcggagct tgcagtgagc tgagattgtg ccactgcacc ccagcctggg 7200

cgacagagag agaatctgcc tcaaaaaaaa aaaaaagaaa aaagtataaa agtattactt 7260

gggaattgat aaatgttcat attgaaatga aatcttcact atttatgttc ctctgccacg 7320

gctccagcca gtccctccat tcggggttcc tgacttcctg caacacaggt gtgagccact 7380

gtacccagac taggggtgca gttttttttt ttttttttt ctgagacaga gtctcactct 7440

gttaaccagg ctggagtgca gtggtgctat catagctcac tgcagccttg aactcctggg 7500

ttcaactcac cctccagcct cagcctccct agtagctggg actgtgggcc ctgcagtttc 7560

tccttttaga gtaggaagac ctgaactgtc ccaggcttgg agtgggtggg cgatgcagcc 7620

cctgaacagg agccagaatg acaacacctg ctgccaggaa agagctctag atagagcagc 7680

catacaggag ggcccctgag gtggcaccct gaggtggcca gcctgcctgt gggtgcacat 7740

tttgggggac ccttccactt gccctcactg gtgcagtgct gcattctctt gggccttgct 7800

atgagctctg ggctcctgct ctttgctggc ctgtaccagg cagtgggttc aaagaggagc 7860

agaaaattaa tggacaatat gtcagaaggc agaggcaaga cagacacttg ctggggccaa 7920

gccctgcagg tggagagggt atgcctggct aaagtgggtg aaaggcaagg ttatgaggtt 7980

ctccaggaca ctggagtgca caggtggtgt gtccccaggt aacgcctgcc acccagccct 8040

tcctcccaca gaacagcatc tgccctaccc acctttgagg tactttgggg tccttccttc 8100

ccagcaggct acccaagccc ttccaagtgc ttaaaggcag atttcctatg cttgcaaacg 8160

actgccctat gccagtgttt atcagcccga gagggctcct gggtgtgcac agggggcgag 8220

caagctgccc aagataagca catccataca gacagctgct caccctgcct gatagcagac 8280

agagggggca cagtgcgagg ctgcagggca ggatgaccta acaagggccc tgctatggca 8340

acaagaagga caggcacctg ccatggaagg tagggacgtt ctgagcaaag cttccggcta 8400

ccaggcagct ggaggagaga gatgcttctc catcagcagg ttcatgctcc ccgggggacc 8460

tggtggcatt ttctccctga ccagcagtcc ttggttctct agacttatat taaagccatt 8520

agaataattt acaacaattt aggcctttcc agagcccttg agttgaatta ggaattgagt 8580

gtgctttgga ctggctgttg gaaccgagtt gtggctctgt cagttcccgc aggtgcgcac 8640

acatctcacg tgcactcaga ggctggctgc caggtgaaag agtgggtggg tttggttgtg 8700

gggcaggctc gctgcaggcc atggcctgag tgcctggatg cagcttccca agctttctca 8760

gctgtgagcc agggctcctc taggctccag ctcttgtatc ctttaggagt gcatgttcta 8820

gacctgtctg tggggcatct gcagggccag ggtgtggaga gacatgacac tccaagtaca 8880

ctctctgcag ccttgcctgc ctaggaaggt ggaggtggct gcaaagataa gtgcagcctt 8940

ctcatggcag acgctaggct ctgatggagg tgttgggcag gttgcccagc cttgtatgac 9000

agaccctgct ctgatcatgg aacctcttgg ccttgtctga agcagcgacc ggctccagat 9060

gctctgggag ggtggtgctt ctcatctggg caggctcgtg tctgcagagg ggctgagggc 9120

actacttgtt ttatacccta gagtcttgtc atcagtcccc accctgccct caccccagca 9180

gactgatgac ttgctattat ttcttccttc cttcagcagg gagttggctg gtgcctggtg 9240

ctgggtgtcc cggacctcag gcccagttca gtgtacttcc cctctctact tcctccctcc 9300

agtcccttct ccatccctcc ctttttggc tgccccttgc ctgccttcct cgccagtagc 9360

ttgcagagta gacacgatga caccttttgc aggctaaaaa ggctgagagt ggcactatgt 9420

gcagtgagcc accatggagg accaaggtga ggcgacacca caaccagccc aaaaggaatt 9480

ccagggatga aacctgagcc caggcagctc tccctgtgcc cagggtggct tccctcctag 9540

ctgactgcag ctgggcacac caagaccctg gctgtgtgca ctggccagct gtgagggaca 9600

ggggctgctt gtgcttttat tctttttttt tttttttttt ttgagacgga gtctcactct 9660

gtctcccagg ctggagtgca gtggcgtggt atccgctcac tgcaagctcc gcctcccggg 9720

ttcacgccat tctcctgcct cagcctcccg agtagctggg actacaggcg cccaccacca 9780

tgcccggcta atttttttgt atttttagta gagacggggt ttcactgtgt tagccaggat 9840

ggtctcgatc tcctaacctt gtgatccacc cgccttggcc tccaaaagtg ctgggattac 9900

aggcgtgagc taccgcccct agcctgtgct tttattcttg ctcacttgtg acggagggca 9960

gccttcacaa ctgaaaggca cgtggacttg agaatgtttt agtccacctt ggtggctcat 10020

gcttgtaatt ctagcacctt gggaggccaa ggtggaagga ttgcttgagg ccaggtgttt 10080

gagaccagcc tgggcaacat agccagaccc catctctaca aaacaaaaaa atttagctgg 10140

acgtggtgat cggtgcttgt agtcccagct attctggagg ctgaggctag aggatcactt 10200

gagcccaaga ggtagaggct gcagtgagct gtgattgtgc cactgtgccc ctgtcgtggg 10260

ggcccttccg ggtctatgtc ccagccctgt gctgaccgtg ttccttctca cctttcatcc 10320

ctcccccaag cagggcagtg gactacaatc ttctgggtga cagagtgaga tcctgtctca 10380

aaaataaata gaataaaaaa gaaaatatct tagttctgtg tctggctgag aacactgggt 10440

gaggtttgag ttcagagtgg ttggtatggt gtgcgtgtga ttttgcaaag atgatcacac 10500

acgcccacgg ccaacctcac caccatatgg cttggtctct ggatttgcac agactatgtg 10560

tatcgagtca ctctttctgc tctgttgtgt ggtgtcttgt cacccctgac catcacagaa 10620

tggagtgtcc aatcctgata aaattgatcc tgtaccacgt ttaaccagac agacagtccg 10680

tcccacctct catcccttct ctcagcaggt cagcgggact atgatctcct ggtggtcggc 10740

gggggatctg gtggcctggc ttgtgccaag gagggtatgt attctgtata cttcgtggca 10800

aggcctcgaa gcttttaggg cccctagaga gggtggtgct gtccagaaaa cgtccatgag 10860

caaaatgcgt tgcctccctg ctggggtcac cccagtggcc tctgtgttgt ggcttgttcc 10920

ctgccactct ccagctgtct gctgtaccca gcggccgccg tggcacttca tggctgaact 10980

gccttcctca gctccagccc tccagctctt ctgctttact cacagtgaca gccccaatcc 11040

gactagtctc agctcccacc actcacaccc tccgtgggct ccccactttc cttcacgtgc 11100

ttctggacag tcctctcctt caccttactg tggctgtcac cctgcctgcc tgctctctgc 11160

ccctgcccgg ctagctgcga ggctggagct gcaccctcct gcttgtccct gtccttgttc 11220

ttagtgctgg tgccttcccc cagggtccat ggtcagttct gctgggtgag tcttgtactt 11280

ggcctggcac ccattgagta aagatgttgg gcgaacgggc tggacccaga ggtgtccaga 11340

gatgacactt tgcagttctc tgctaaccca cgtgatgcac aggccaccag cactttgcca 11400

gactctgtgc cctgctgtag gagttcctgg tctgctctgg ccatgtctac acaatgataa 11460

gagggcagtt gtggtgaatg gggagggaag gggatgagag ggaggtgggg ctgaaatgag 11520

agggagttaa ctgggcactt tggccctgaa gctgtgcttc tctgatgtcc agtattgggc 11580

tctagaagca tacatggtgt aaaagaaatc cactactctt gctctgtggg ggccggggag 11640

tgtgtaggaa cctgggaagt ctcctggcct attggggatc cgcagaggca cccaggatca 11700

gtgccacact gtactctcag aaccactccc agaaacgatg gcgcaggcag ccacgaggcc 11760

tagtgctgtt ccttttagca gaccgggcac ctgggctgtg cgttcactcc catgctggga 11820

gtgaccagct gcagagacct gagtcccctt taatccggaa tgtggacagc tcctgggtat 11880

gtctccgcct ttcatgagag tggcatttcc ctgcagagga tgacgtggtt ttgtggcttt 11940

tttgagatga actgtaccta tcaaaatggg caacctgatc attcttatgt gtgcatgtgt 12000

ccatgcagcc agtaccgcag tcaagattgg cgagtgtgtc ctccaccccg ttagtctgcc 12060

agggctgccg taataaacag actgccataa cagactgggc ggctgaaaca acacatgctt 12120

attgtctctc agttctggag gctagaaatc atccaggtgc gggcagggct gatttcttct 12180

gaggcctctc cgttgggctg tagatggcag ttgtcgtccc tctgtgcatg tctatgtcct 12240

aatctctacc tgtacctgtc ctaatggatc aggtggatgc cctcttattt tgttggcttt 12300

ttattggaga cagggtcttg ttctgcctcc caggctggag tgcagtggcg tgatctcagc 12360

tcactgcaac ctcaaactcc tgggttcaag ccatcctcct gccttagcct cccaaaatgc 12420

tattacaagt gtggccactg tacctgtata tggcctaatt tttttttttt tttttttgag 12480

atggagtctc gctctgttgc ccaggctgga gtgcagtggt gtgatctcag ctcactgcaa 12540

cctctgcctc ccgggttcac gccattctcc tgtctcagcc tcccgagtag ctgggactac 12600

aggcgcccac caccacgccc ggctaatttt ttttattttt agtagagacg gggtttcact 12660

gtgttaacca ggatggtctc aatctcctga cctcatgatc cacccacctc ggcctcccaa 12720

agtgctggga ttacaggcgt gagccaccgc gcccggccta tggcctcatt ttaacttaac 12780

tacctctttt ttttttttgc aagcgagtct tgctttgtca cccaggctgg agtgcagtgg 12840

tgagatcttg gctcactgca acctgtgcct tctgggttca agcgattctc ctgccacagc 12900

ctcctgagta gctaggatta caggcgcctg ccaccacacc tggctaattt ttgtatttta 12960

gtagagacgg ggtttcacca tgtttgtcaa gctggtctcg aacttctggc ctcaagggat 13020

ccgcctgcct cggcctccca aagttctggg attacaggca tgagtcaccg tgtccagcca 13080

acttaattac ctttttgaag accctatctc taaatacagt cacattctga agtgctaggg 13140

tttagggctt ccacataggg attttgaggg gatatggctc agcccataac accccaacat 13200

tttctgaaac cttggcagtt ccttccggct tcccccactt ctgcatccta ggcaaccagg 13260

catgtgctgt ctgtcactat agtttgcatt ttctataatt gcgtataaac ggaatgctgc 13320

tgtatgtcat cttcctcttc tggcttcttt cactcagggt aatgaccttg agactcatct 13380

tcgttggcgc gagtgtcgat ggtttcttgc ttttcattgc tgagtagtgt tctgtttatg 13440

gctgtgccgt ttcgtgtacg tgttcccctg tagctggaca cttgaattgt ttccaccttt 13500

tggccattgt ggacagtatt gctgtgaacg tctgtctgtg tgtttgtgtg gatatatgtt 13560

ttaaattatt ttgggtaagt gcctaaaaat ggaccaactg gatcgtgtgg tatatctctt 13620

atttaggtat tttttcattt cttttagcag cattttgtag tttctttttt actcaagttt 13680

ttttttttta ttaaaaaaaa gaaagatgag ggtctctctg tgttgcccag gctggtctca 13740

aaatcctggg ctçaaatgat cctcccacct tggcctccca aaacgttggg attacaggaa 13800

tgagccacgg tgcctggcct gtggttttca atgtctacgt ctttcacata ttttatcaga 13860

tacatcccta agtgtttcat atttttagaa tagttttatt gagatatacc tcacattacg 13920

tatgcatttg tcccttggta cccgaggacg actgggttca ggaactcccg ctgttagcag 13980

aatccatgga cactgaaatt tgtgcatact ggagtcaggc agttggccct gaagcaccca 14040

cagatacaga gtcagccctc tgtatatata gttttgcatc ctatgaatac tgtttttttc 14100

tttttctttc ttttctttct ttctttcttt tctttttttt tttttttttt ttgagacaag 14160

gtctcatgtg ttgcccaggc tgcagtaaat ggtgcaatct cggctcactg caacctccac 14220

ctcccgggtt caagcgattt tcccacctca gcctcccgag tggctgggac cacaggcgcc 14280

accatgccca gctacttttt gtattttttt ggtggagaag gtgggtttcg ctatgttgcc 14340

caggctggtg ttgaattcct gagctcaagt gatccgtctg cctcagcctc ccaaagtgct 14400

ggattacagg catgagccac catgtccagt ggagtactgt atttccaatc cgagtttggt 14460

tacagatttg gaacttgctg atatggagag ataatatata tatttttttg agaccgagtt 14520

tcgctcttgt cacccaggct ggagtgcaat ggcacaatct cagctcactg cactctccac 14580

ctcctggatt caagcagttc tcttgcctca gcctcccgag tagctgggat tacaggtgcc 14640

tgccaccacg cccagctaat ttttgtattt ttagtagaga cggggtttca ccatgttggc 14700

caggctggtc tcgaactcct gacctcaggt gatccgccca ccttggcctt ccaaagtgtt 14760

gggattacag gtgggagcca ctgtgcccgg tcaagggatt atatttattg aaaaaaaatc 14820

catgtatgag tggacctgtg cagttcaaac ctgtgttatt caagaacatt ttagtttctc 14880

cccaaaatac cctgtaccca ttggcagtca gtctcttccc ccctctcccc agcccctggc 14940

aaccactaat ctacttccta tatccgtgga tgtgtctgtt ctggacattt cagctgtttt 15000

ttggttggtt ggttgtttgt tttcagacag ggtctgactc tgttgcccag gctggggtgc 15060

agtggcacca tctcggctca ctgcaacctc ggcctctcgg gctcaagcaa ttctcccacc 15120

tcagcctccc gagtagctgg gattacaggt gtgcaccacc acatccagct aatttttgt 15180

atttttagta gagacggggt ttcaccatac tggccaggct ggtcttgaac tcttgacctc 15240

tcaagtgttc cacccacctt ggcctcccaa agggttggga ttaacaggtg tgagccgccg 15300

ttgttttgtt ttttgagatg gagtctcgct ctgtcaccct gactggagtg cagtggtgtg 15360

atcttggctc actgcaacct gtgcctcctg ggctcaaatg attctcctgc ctcagcctcc 15420

caagtagctg ggaccacagg cacacaccac tatacccgac taatttttgt acttttagta 15480

gagatggggt ttcaccatgt tggccaggct ggtcttgaac tcttgacctc aggtgatccg 15540

cccacctcgg cctcccagag tgccggtatt acaggcatga gccaccgtgc ctggcctctt 15600

cgcatattct ttttcttttt ctttcttttt tttttttttt gagacggagt ttcgctcttg 15660

ttgcccaggc tggagtacca tggcacaatc ttggctcacc gcaacctctg cctcccaggt 15720

tcaagcaatt ctcctgcctc agcctcctga gtagctggga ttacaggcat gcaccaccac 15780

gcctggctaa ttttgtattt ttagtagaga aagggtttct ccatgttgag gctggtcttg 15840

aactcctgac ctcaggtgat ccacccgcct cggcctccca aagtgctggg attacaggcg 15900

tgagccacca tgcccggccc tgcactttct tgatggtatc ctttgtgcca caaaagtttt 15960

aaattttggt gaagtcctaa tttatctatt tttttccttt gttgcttatg attttggtat 16020

catagctaag aaatccaaga tcacaaagat ttacacctgt gttttcttct aagcatttta 16080

tagttcaagc tcttacagtt aggtctttga tttattttga gttgctttag ggtttatggc 16140

atgtacctta acttgccagt ctacccttaa aaaagcatac atctattcca cccctcctaa 16200

ccttcattct atgttattat atcatgtgtt tactgttaca tgttataaat ccatgataca 16260

tcattattat ttttgtttaa aatatactta aatcgtgtga gaaaatctca catactcacc 16320

cattgtattt ctgtgctctt cattcctttg tgaggatcca catttccatc tggtattttt 16380

cttctacctt aagggcatcc tttagcattt cttgtaatat ggctctgggg gtaatgaatt 16440

ctctcagctt ttgtatgtct gaaaacgtct ttattttgcc ttcttttttg ttgttgttgt 16500

tttgttttgt tttgtttttt gagatggagt cttgctctgt cgcccaggct ggagtgcagt 16560

ggtgcgatct cagctcactg caagctctgc ctcccgggtt tgcgccattc tcctgcctca 16620

gcctcctgag tagctgggac tacaggcgca tgccaccatg cccgtctaat tttttgtgat 16680

tttagtagaa acggggcttc gccgtgttag ccaggatggt ctcgatctcc tgaccttgtg 16740

atccgccccc cttggcctcc caaagtgctg ggattacagg cgtgagccac catgcctggc 16800

cccccgcttt tttttttttt ttcagtactt taaaaatttt gcccctctga cttctggctt 16860

gcattgtttc cagtgagaaa tctgctacta tttttatctt agtgtctctg tagtgtgtct 16920

tggttgcttt taggattttc tcttttcatt ggccttgagt ccctccttct tcccctcaca 16980

tgtggggact tttaattcca tgtatattag gctgcatgaa gcttccccac aacctactga 17040

tgctcttttc attagaaaca tttcttactc tgcgtttcat tttggatagt ttctattcct 17100

atgttttcaa acccaccaat aaaagattct gcaacatctg acctgccatt aatcccgtcc 17160

agtgtatttt tcatctcctg tattgtagtt ttcatctcta caatccagct tgagcctttg 17220

gttatatctt ccatgttgct cctgcactgt ttgaacatgc agaatggcta gtggggcagt 17280

gagctgagga gaagggacag aggggaagct cagctgttgg gtctacgggt atgatggaga 17340

ccatgcagct gaaagtaaac cgtcacccct tctgcttcag tgtgaaaggc caggtgaaga 17400

tgctgcagct gatgaggctg ggccttaggg tgcggggggt ggtggaatct gcttgtgggc 17460

gggagatgtg gctatgtggc tataaaggat gaagatgaac gccctgtttg cttttcagcc 17520

tcgcttggat caaggttaaa aggccggttg tggccttctt ggtggaagaa agagagagat 17580

aaggcactgt cctccccttc ggagggtctg gggatacact aatccatcaa aaccactgag 17640

ggctgggcgt tggtgtgtgc ctgtggtccc agcactttgg gaggctgagc tgggaggatt 17700

gcttgagccc aggaggtcaa ggcgtaagca agctgtgatc gcaccgctgc acaccagtgc 17760

ctgggcaaca aagtgagact ctgtctcttt aaaagaaaaa agaaaaaaaa gccttttttt 17820

aaattaaaaa aaaaatcaag tctacctgaa tggcctgcag ttggacccac aaaccaggta 17880

cccaagttac caggcaaggc agctgcagtt gtaccagtca gaagtccaca agatttgaaa 17940

aaaaaaaaaa aaaaaaaaag cctcaggggt ttcagtgaat gctgtgttaa cttttttttt 18000

tgagacatag tttcactctt gttgcccagg cttgagtgca gtggtgccat ctcggctcac 18060

tgcaacctct gcçtcccggg ttcaagcagt tcttctgcct cagcctcccg agtagctggg 18120

attacaggcg cccaccacca cacctggcta attttgtatt tttagtagag acgagctttc 18180

actactggtc gtgaaactac agttggtcag gctggtctcg aactcctgac ctcaggtgat 18240

ctgcccgcct cggcctccca aaatgctggg attacaggcg tcagttacca cgcccagtct 18300

gcttgttaac ttctataagt cagatgtttg atgtgaccga gttcaaggct gatacagccc 18360

agagccagga caggtcgaga gctgcccatt tttagggcct cagacttcct actgggagca 18420

tcgcagcccc ttctgaacag aggtttggag aaggtgggtc atttggagag aagtgaccta 18480

aactatccta gttgaaggaa tctatccttg aaagtcatca ttgaaggaat tcaaaaagag 18540

aaaacttaag ggaaaggaaa aaaatagttt taaattttaa agagtgggca tgtgggagag 18600

ggggtggaga tagttaagag ttataggaaa acatttccaa ttaatatgag gagaaatttt 18660

atattgggag agttcccaaa attaaatggc cacttgggaa ggcaatacat tcccagacaa 18720

aggcaccagg cataggtgaa cacttggtga ggatgttatg aaggagactc aaacttcagg 18780

taagcaggta gaacaaattg catataatat ccttcctggc cgggcatggt ggctcacgcc 18840

tgtaatccca gcactttggg aggccgaggt gggcggatca tgagatcagg agtttaagac 18900

cagcctgacc aacatggtga aaccccatct ctactaaaaa tacaaaaaaa ttggccgggc 18960

atggtggtgc gcacctgtaa tcccagctat tcaggaggct taggcaggag aatcgcttga 19020

acccaggagg tggaggttgc agtgagccaa gatcacgcca ctgcactcca gcctgggtga 19080

cagagctaga ctccatctca aaaaaagaga aaaaaagatc cttcccacac caaaagtctg 19140

tagctgtgaa aagtagctac catatagtgc aagttgagat gcataagagg tttataaaac 19200

caccctggag tcgaaggaga gagaactttt ggtagggagt aagtaggtaa aaagggaccc 19260

aaaggtctgg gatcttgaac actgaaggag gaacagtgtt ctgtgagaaa ggtttgtttt 19320

taacatgtta atttcttcaa caaaccattt gaatacttaa tgtggtccaa gctctgggcc 19380

aggacccgga acaacaaaga caaaccagga ccaaagcccc tgactgacgg agttctgcag 19440

tttagttacc gagacagaca catgaacaga tagttcatca tatgtgataa ggtggagtta 19500

gggcacacaa gtgaggcatc taagcaagga aataaaaata aaaaagtatg gaatagttag 19560

aatacctgtt ttattgatct tttctgctaa ttctgacatc tgtgtctgtt ccaagtcagt 19620

ctccattgac tgattttttt ttgtcccctt tataggtgta ttttcctgct tctttgtgtg 19680

actggtaatt tttggttgaa tgccagacac ttgaatttac cttgttgggt tctgggcatt 19740

tttgtatttc tagaaatact attattgagc tctgttctgg gatgcagtta ttaagagtca 19800

gtttgatcct tttgggtctt ctgcaaaaga tttgttaggt gggtatggag cagtgcccag 19860

tctagggcta atgattctca actactgggg catgacccaa cgccccagga attatatgct 19920

tttcccagtc cagattctgg gaacaggcac tgtcccctgc cctgtgtggg ctctgtcccc 19980

tccagtcctt ccaggtggtt ctgtctggtc tcaggtagtt tcccaagaca catgcgccga 20040

tcagatctct cagattctct gggcagcccc ctcctcactg tcttctgtgc atgacctctg 20100

gctgcctcag tctcccccag ttctcagccc tctcctgtgc tggattctcc ccatcttccc 20160

cagcctgcag tgctgggtcc tggagactcc ccagggctca ccatgtttgt ttcctctctc 20220

tcaggtcctt tgttacctga catggagtac ctgaaagccc gttgtttcat gcattttacc 20280

tgggttttgg gttgcttcag ggggagggta aatctgggcc ctgctgctcc atcgtggtca 20340

gatgtgggag ttacacactg gcacactggc tttttttttt tttttttttg agacagggtc 20400

ttgctctgtc acctaggctg gagtgcagtg gcacaatctt gactcactgc atcctccacc 20460

tgctaagctc aagccatccc cccaccttag cctcccgagc agctgggacc acaggcgcat 20520

gccaccacac ccagctaatt tttgtatttt ttgtagagac agggttttgc tatattgccc 20580

aggctggtct caaactcctg ggctcaagca atctgcctgc tgcggcctcc caaagtgttg 20640

agattatagg cgtgagccac tgtgccttgt ggacattagc tttttttttt ttttttgag 20700

acagagtctc gctctgtcac ccagtctgga gtacagtggc acaatctcgg ctcactgcaa 20760

gctccgcttc ctgggttcac accgttctcc cgcctcagcc tcccgagtaa ctgggactat 20820

aggcacctgc caccacgccc aactaatttt gtttttgtat tttttagtag agacagggtt 20880

tcaccgtgtt agccaggatg ttctcgatct cctgccctcg tgatctgccc accttggcct 20940

cccaaagtgc tgggattaca ggcatgagcc gctgcaccca gccaagcaaa atggatttta 21000

aaaatgaact cttacaaaac tttgttctaa tcttgctttc taatagccac gaatcaaaca 21060

ctttccttgg cattttaggg ttcatgagat ttgtgtgtgc cttgggaggc agtcaagtcc 21120

aaggcctgaa gtttgctgat gcagtgccca agcccgggca aatggaagct tgaaattagc 21180

cctagctaat gttttttttt tttttctttt ctttcttttt tgagacagag tcttgctctg 21240

tcgcccaggc tggagtacag tggtgtgatc tctgctcact gcagcctctg cctcttgggc 21300

tcaaacgatt ctçttgcctc agcctcccgg gtagctgggg ctacaggctt gcaccaccac 21360

acctggccaa ttttttaatt tttattttta gtggagacac gttttaccat gttggccagg 21420

ctggtcttga actcctgacc acaagtgata caccttcctc agcctcccaa agtgctggga 21480

taacaggtgt gagccaccat gcccggtcct ggctaatggt ttctgagttg gccccagaac 21540

ctgtggccct gggtggccac actgctggct gggagggcaa ctgggtggct ctgcagcggg 21600

aagtgtggtt tggagtcagg tctcggctcc tgctctgggg ctggtccacc ctggggctgc 21660

tgcgtcactt ctcctgagct ttggtgtcct ggtcctgaga gtgaggcagc gaatgcccac 21720

ctagggggct ctggctggga tgggaagggg caagggagag cctctgagtc aggagccatt 21780

cccggtgagt ggctacctgg gggatgccac ccagggatgc ttgggtgggc actgggctta 21840

tggtgaacgt gaacatgcag gggcaggtgg ggctgatgcc agcactgtga ggggtggatg 21900

gtacaaaggc tgcgggccct gagaggtctg agggctcctg cctgtgggca gcgcagctgg 21960

gtgggcagtg ccagaaagag actggaagac tcaatgcaga gccagcaggc agcgcagctc 22020

agcaggccag agcgcagagg cagatcaggg gtccctagca ttgcgcagct cagccactgt 22080

gtgggcagcc gcaggcccag gccctgccca cactcttctg gacctcacaa gccctgagga 22140

gtgcctgttc ttgagtcctg agccagctcc ccatgctcac ctgctgctct ccttatcctt 22200

ccagccgccc agctgggaag gaaggtggtg gtggtggact acgtggaacc ttctccccaa 22260

ggtaggcagc accctatggg agccgggtgt cctggggaag gaggtgggag gtgggcccag 22320

ttcctgtaca gagggcagct gacaccaaag tccttcagcc ctgctgtcca tggtttctgg 22380

acagtgtcct gtttcactca tcattacttt aaagttcttg accattatcg aaaacaactc 22440

tggccaggtg cagttgctca cgcctgtact cacagcactt tgggaagcca aggcaggcct 22500

tgatcacttg agcccaggag ttcgaggcta tagtgagcta tgattgcact actgcactcc 22560

agcctgggta acaagagcaa aactccatct caaataaata aattaataaa tattaaaaag 22620

taaaaacaac tgtttttatt ttcgagtaca gttatagaga gcagattcgt gtggcatgtc 22680

agctggtctg tgttagagtt acaaagcaac tttaaggaat tccaaacact ctagaagaac 22740

agggaagcct ccagcagtca cctgaacata aattcaaatg tgctcttccc acgtcccagg 22800

cacccggtgg ggcctcggcg gcacctgcgt caacgtgggc tgcatcccca agaagctgat 22860

gcaccaggcg gcactgctgg gaggcctgat ccaagatgcc cccaactatg gctgggaggt 22920

ggcccagccc gtgccgcatg actggtaagg atctggcgcc gtggcattcc agtgcttttc 22980

ttctactctt gggtggaaga ggaagaggag gcttatcctc gatgagccct catggggagt 23040

gggccgtagg atgggtttct cagccagggg cgactctgcg ctgtctgcct cagacatttg 23100

ggaatgtctg gagacagttt tggttgtcac tggggaaggg tttgctcccc tttgcgggga 23160

gcccagggat gctacagcca tgcacagcac agccccacca agaacagtgc atctccaagg 23220

ccaggagtgc gggtgggagg ccgcttcagc tgagctcttc tgggaagggg accacgtggc 23280

ccagccacac ccacattggc tcagataggc ctctgcctgc agtgggtagc ctttggggca 23340

cagagcagct gcatctggag agccgtgggt cagagcccct gttttctgtg agtccaaagg 23400

tctgcagccc tgagcctggg acaggcggtt gcacgtaggg atggatgtca cgttttgcca 23460

cctttaaaag cagtcttgtt ttttgatatt tctatgaatg taccatttga atctaatagt 23520

ccatcgtgag gccctgcagc taacacctgt gttgtggatt ttacattttg tttcgtatct 23580

tcacaggagg aagatggcag aagctgttca aaatcacgtg aaatccttga actggggcca 23640

ccgtgtccag cttcaggaca ggtactgaag ctctctccgg gaatgggccg ccctctgggc 23700

cttctcttgg gcgttctgtg cctggacaca cacttactta ctgtgcagag catgctctgg 23760

caggctctgg gggttcatgt cctgctcatg ggtggggatg aggacaagga gcagatggga 23820

gtgactgtag gggaagggga gactgccctt tgctgctgag cagagcctgg aggtgctgcc 23880

aaggaatgag tgaggccaca ttggcagaat ggccagagcc cagggctgca cagggaggag 23940

gcgcttgggg ctggaaggcc tcaggccaga gagcgtggac atagctaggc ctggggctaa 24000

tatgtggtga gggtcatccc agtggcaagc ccccaccccg tgaacccccct tcttactgca 24060

cacctccagc tccttgggtg tgggtgcagg agcttggtgc tttccgctgg agcaaatgtc 24120

cctacttggt caccgttcct gtcagcccct ggggatctcc agcacagagg cctatgctcc 24180

cctggcctac aaccttctcc tggggctcag ctgccagcac agcagatgcc tgaaactggt 24240

accgcctctg gcccagcctt tctcccgggg ctgtggaggg gacagtggtc ccgcagaggt 24300

ctggtggctc tcctcatgca ccatttgctt ggccccaggg cgggtcttcc tggggcttca 24360

cagcaggcag cagttttgtg ctcactaaat ccaggaaagt ggagccagga agccaactgc 24420

ttgccctcca cctggacctc acaagctctc ccctatccag ggagctaagc cacattgtgc 24480

tgtggcattt ctgtgtttct ctgtggggct ctgtatcccc tggtacagtt tcctggggac 24540

agcaggctct gccctccctc ctccctgccc tgggcagctc ctggacgggc accaaacagg 24600

cccagcccac tgcctgctcc ggagccacct gcagaaggag gctggggcgc acctgggctg 24660

tttctgcttt ttcactcttc tgaaaagtgc tgccatgagc attgcccggc tgtgtcccgt 24720

ggcagcttcc tggctgtcga ggtgattgaa gggctcttgc tgtaggaact tcacgcagct 24780

cagacagccc atagaggcac aggcttgcca gtggggagaa ggcaggctca agcaggaggc 24840

agagccttcc cagaaccctt gctgcagcac ggtccttttg tcattagaaa gtgttggtcg 24900

ggtgcagtgg ctcatgcctg taatcctagc actttgggag gctgaggtgg gaggatcact 24960

tgagcccagg agttggagac cagcctgagc aacatagttt tattgctaca tccctacaaa 25020

aaataaaatg aactagccag gtatggttgc ccatgcctat agttccagtt acttgaaaca 25080

aggcttccgt gagctatgat catgccactg cactccagcc tgggtgacag agtgagaaga 25140

tgtctttaaa aaaaaaaaaa agggtggggg ggggtggctc atgcctgtaa tcctagcact 25200

ttgggaggct gaggtgggtg gatcacttga ggtcaggagt tcaagaccag cctggccaac 25260

atggcgaaac cccgtctcta ctaaaaatac aaaaattagc caggcgtggt ggcgcaagcc 25320

tgtaatccca gcttctcagg aggctgaggc aagagctgag gcaagagaat ggcttgaacc 25380

tgggaggtgg agattgcagt gagccaagat cgtgccactg aactccagcc tgggcgacag 25440

agtgagactt tgactcagaa aaaaaaaaaa gaaaagaaag cgtgggtcat ttgtttctgt 25500

gcactatgct cccagccact gttttgccag ccttgtcatg cccgttctct tggtgttacc 25560

acacccctga aatcagaagg tgacaccatc tggtgggcac cacagctccc tgctggaaca 25620

tgtccgggtg atgaggactg tccccaagag aggtccagcc acctcttgct gcacaccagg 25680

gctgtacgtg gcctcttagg accgtgctga gctggcttcc gtccctgctt tgacacctgt 25740

ggttaacatg tccctgggat ccctggggga caggcgaggt gcccccacat cccctccatg 25800

cttctcagca tggttgccgc ttacctcttg gtccatctga gccacagcac caggccctgc 25860

tgggggctgg agctcccttt taccagtgtt ccctattgat ccagttggtg aggtttaatt 25920

tgcagaggaa gtgtttgaaa atcttatctt tatctttcag aaaagtcaag tactttaaca 25980

tcaaagccag ctttgttgac gagcacacgg tttgcggcgt tgccaaaggt gggaaagagg 26040

tgagcatctg acttactcgc gtggctcctt gtggaccctt ctgcagacct tgggcaccaa 26100

ctgcagctgt gtttggcctg ggtgctgttc ttagtaacac gtgctgctgg aatcaaaaag 26160

gtggcttctt ttgaggctgg gcacttgtct ttaacgtgat caaataattt gctgccctgc 26220

tgctcggagt ggcatggcaa cagggttggt gaccacaccc tttttgcagg attttttggg 26280

gatttgaggg tgccttgaag tgcttggagt tagaacatct ccctgtgctt tctgcctgtc 26340

cccctcctgc caggctgatt gttgatggga ttccagctcc atagggcctc tgaactgctg 26400

gccaagggtc cacgctacag ggcaggggcc gtgggaactg ctggccaagg tctgtggtgc 26460

tcaggccctc cggtgggggt gatcaccatg cacctgtctg acccacggct ttctcttttt 26520

ctcctcagat tctgctgtca gccgatcaca tcatcattgc tactggaggg cggccgagat 26580

accccacgca cgtgagtgtc cccagagcat agcgtccctg ctgccgtggc ccattcccgg 26640

cctctttgag ggatacgttt ttacacacgt gcttcccaca gcagcagctt gcacaccctt 26700

tccccctata ctcactatca tcactttctg ctttccaatt ctcttgaatc cacactgctg 26760

aaatgtgggg tccccagtgg cctccacgct gccagatcct caggacagct ctcgttctgc 26820

tctccccctg ccccgctgga tctgtcccct tccacaccag gatcctgctt cctaagtctc 26880

cattgctgat tccccctttt cccttcagcc tcagaatgtt ggaacattca ggatataagc 26940

ctcattcttc atcttcctcc ttcacgtccc ccagtttaaa aaaagtttga aataaaattc 27000

acacgctata aaatttagcc ttttttaggg tacaattcag tggtttttag tacattcaca 27060

gagctgtgcg gccatcagtc accaccatcc attgccagaa ctccttccat catccctgaa 27120

ctgaaactat tcccattaaa ccctactccc cagcgcctcc tcccccagcc cctagaaacc 27180

accacctact ttctgtctct atgaatgtga caacactagg cacctcgtgt aagtgaaatc 27240

gtacatgtaa gtccttctca tgtaactggt acgtgtgtgt cccttagtga ctcgtatgtg 27300

tgtgtccctt agtgactggc ttacttcact gagcataaca tcctccaggt tcacctacat 27360

tgtagtgtgt gtcagtatca ttcctttta tggctagata ctattccatt gtatggatag 27420

accacacttt gtttatctac ttgcttggat aaacacatgt gttatttcca ctttttagct 27480

attatgaata gtgccgctgt gaacatctgc aaagaagttt tttggtggac ttcagttttc 27540

acttctcttg ggttacactt aggagcagaa ctgctgggtc atgtggtaac tttatgttga 27600

atctttcaag gaacgtttca aggaacctca gactgttttc cacaatgact gtgttttaca 27660

ttccttccag tggggtgtaa gggtcatggt ttcttgtttc ttcacgtatc ttgtaatttt 27720

tttattgaaa gctgaacatt tcaaataatt taatgcgata actttggaaa ccagattctc 27780

cctctgcccc aggattctgt tgttacagct gcttatttgg tgacttttcg gaactgactt 27840

tgtagactct tattctttga tgtatgtggc cactgaagtc tttacttggt tagcttagtg 27900

gtcagctaag aactgcatgg agatttccct aaactaagaa ctctcccggt ctttgctgag 27960

ggctctgtgt gcgtttggag gggatgcctt ccacactcaa caggcagcag acagctctgc 28020

cctagccttc acttcctgct tctgcagaga tcaaggtcag ctggaggtga gggttcaggg 28080

cctcgttggt ccttcctgat gtgtgcacag tgctgtgcat gcgcctggcc taggttccca 28140

ggaatatgct ggaacctttc aaagctccag cagacatctc atactttggc ttttcctttg 28200

aagcttttg ggcagtctgt tgttggctct aactgttacc tatcccctca ggcagctgtg 28260

agaagaaaac ctcagacaaa tgcccccaga gaaaggcttt tagccctggc tgagctccgg 28320

tgaagttgga tgaagatgac cctatagttg ctgactctga ctcttctttg tgggggggct 28380

ttgaaagacc ccagctgagt tctgctctct ctgacacatc actgttcagg gctgccgctg 28440

aggtgggagt gggaccagag tgagttaaaa caccctggag ttcccattct cactcagctt 28500

cagctgtttt tcctgacttt aaatgttccc tgtgttgctg caagcctgtg gttaatttcc 28560

ataattctga atctcccagt tcttgccagt tttatcgctg tttttaacag agaggtgaat 28620

aactcctggc ccagtcttgt gggttgtggg ggcagagttg aggaaggggc cccggggtga 28680

ggggttgggg ggcagctgca gcagggaaag caaatgggct gggggtgagc agagaggttt 28740

ggctgagacc agtccccacg ggtctcccag ggaaccgtgt gccgcctgct ccaagcctct 28800

aagtaggctg cagccaagct cttgaagacc acggctctct cggcctggag gccacaccac 28860

ctctgactta gacccagggg cagtccggtg gaggcagagc aaggggaggt tctgccactt 28920

cttggatgtg accccagcct ccacttcgtc ctgtgcttac tgtggaaaca gggaaaggag 28980

cggccccgag gagcacaagc acccccagtc cttagaggcc gtgggacttg tctggccgcc 29040

aggcagccac agcagcctct agagggggca ggttgcttat gcacagaggt ggggctcagg 29100

gctgcgtgac tttgtaggat gacactgtgc agttgttcaa ggcagctgct gcagacaggg 29160

tcccagtgat cccctctcct ggcagctggg ctcatggtgg ttctgcttaa agaaggccac 29220

agccagcttc cagcagccca gcggtcattg ggtttctctg aggccccagg cagagctgca 29280

cgtgcatccg caccagggag cactgcacag gggccctggc ctggccccggc cctgctctgc 29340

acctcacggg cagctgctga cggctctttg ggctcacagg ggatccagca ggcgctcctg 29400

gtccttagga ggcaggcagc tcagggctgc ttcgctctcc ctgccacgcc ctcccagggt 29460

gtctgccaag gcttgcttgc gttttagtcc ctttgattgc cagacctttc ggttttcctt 29520

gagtaaacct gagaaattcc tgactttatt tttttttgcc agttggaagc ataaactgtt 29580

tgagatccgc ttcctccacc agcacatctt gttctcatgg ccgctaaggg gacgttcacc 29640

ctgggcctcc cacctgcttg gccgcccctc ttcaggtgac ctgtatgatt tctgggctca 29700

gagcccaccc gggccagccc tcgagagtgt gaagtccgtc ctggcttcag ccaggtgccc 29760

tcagagctgc ccctcagtcc ctgcccacct cagcctgtgg cacttacccc ctgtgcctcc 29820

tctcctctgg ctggccttgt aggccacccc tgccgcgcag acacacctga gctctgctgg 29880

ccctgatttg ctgacctgtt ctctccccac ttcctctctc tgagtttgga tcccccagaa 29940

cccaggtgtt gctccctggg gctgcatgcc cctgtctgtt tgatgtgtct gtccagaacc 30000

cgggcagact tgaggggtcc cagctgtctg cctgtatggc ccctgcagtt gctgtaccca 30060

cctcaggccc tgtatcctgc tctgaaccag ggtcaagggg agggtcctgg ggaacagagg 30120

ggaaaggtac cctgcgaggg caccgggacc tggagtgcag cagcttagat gcagacaggc 30180

cacctgcagc cccaaagagg ccacagcctg cagacaagga ctggcagcag ggaagccctg 30240

tgcatgtgtg ccctgggaaa gctctgcttg attctgcaaa gctggcatcc tctttaagga 30300

agccctagga caggccaaat ggagctcttg tccaaggggt catttctgtc ttgacagatc 30360

gaaggtgcct tggaatatgg aatcacaagt gatgacatct tctggctgaa ggaatcccct 30420

ggaaaaacgt aaggcctgcg cgtgcttggt ggggtcctct ttttgttcac cagagtgagc 30480

actggaccct tagagcctgt gctggtgctg ggctcctggg gctttctttc cggtttaccc 30540

aaaagaagga aaaaaggcct gttatttgtg gccaggtgtg gtggctcatg cttgttagcc 30600

tagcactttg ggagactgag cgggaggatt gcttgaggcc aggagttcga gatcagcatc 30660

agcctgggca atatagtgag acctctactc tacaaaaatc tgttttggtt tttttttgg 30720

cttttttttt taggtggagt ctcactctgt cgcccagact ggagtgcaat ggcccaatct 30780

tggctcactg caatccctgc ctcccaggtt caagtgattc tcctgcttca gcctccaaag 30840

tagctgaaat tacaggcgcc caccaccaca tctggctaat ttttgtattt ttagtagaaa 30900

tggggtttca ccatcttggt caggctggtc ttgaactcct gacctcaggt gatccgccca 30960

cctcgtcctc ctaaagtgct gggattacaa gcgtgagcca ccacacccag cctacaaaaa 31020

tctgaaaata ttaaccgagc atagtactgt gcacctgtag tcccagctac ttgggaggct 31080

ttaggtggga ggattgcttg agcccaggag gttgaggctg cagtgagctg tgattgcacc 31140

actgcgctca gcctgggaga gtgagacctt gtctcaaaac taaacaataa acctgtgact 31200

tgtgcccctc tgcagggctg ttgatttact ctgtcctgtg gctgccttgg aggtaataac 31260

aataacagga ctgccatata aatggagtca gctgtttgtt atctaggtca gtggttgtca 31320

aatggggttc ccaggagccc tggggttcca gtgggtcccc tcaaggacat cagggagaac 31380

attaaggagg ggagagccag caatccctat cccagcnctc atctaacacc ctcactgtct 31440

tctgatttgt acattgggtt tccctgaaat ctagcaaaga ctccaaaggc ctcagaaaca 31500

acatcttgat tgtggaaggg aggctagcag cgtgcaagtg gctctcactc cccatgggag 31560

caggtgcaga gtcactaagt gcattccgat gcccacagag tgggccatcc cgaggtccca 31620

ggtccaggca gctcctgcct gtggggtgtg gagacttcat gttggcaggg agcagagaga 31680

ccaccagagg gcggcagagg cttggtcaat agcagtctcg agccttcccc aggagaaaat 31740

atcagtggaa aagggtccca cttctgtgtg tgcccgaatg tgcatggtgt gcgtgcagat 31800

gtgtgtggtg tgtgtgtgtg aatggtgtgt gtggtgtgtg tgtgagtggt gtgtgtgtgt 31860

ggtgtgtgag tggtgtgtgt gtgtgaaggg tgtgggtgtg tggtgtgtgt gtgtgtgagt 31920

ggtgtgtgtg agtggtgtgt gtgtggtgtg tgtgtgaagg gtgtgggtgt gtgtggtgtg 31980

tggtgtgtgt gtgagtggtg tgtgtgagtg gtgtgtgtgt ggtgtgtgtg gtgtgtgtgt 32040

gtgaagggtg tgggtgtgtg tggtgtgtgt gtgagtggtg tgtgtgcgtg gtgtgtgtgg 32100

tgtgtgtgag cagtgtgtgt gagtagtgtg tgtgagtggt gtgtgtgtga atggtgtgtg 32160

tatgtgagtg gtgtgtgagt ggtgtgtgtg gtgtgtgtgt gtgagtggtg tgtgtggtgt 32220

gtgtgtgtgg tgtgtgtatg tgagtagtgt gtgagtggtt atgtgtgtgt gtggtgtgtg 32280

tgtgtgagtg gtgtgtgtgt gtgtgagtgg tgtgtcacat gctctgcttg tcattgtttc 32340

acacttcacg gggcatcttt gtgtggggtc aggactggat tcccttgaat ggatgtggta 32400

tagttccttt gacctttctc ttgttaatga gcatctcagt agtttctctt cttcatcatt 32460

atcaatgctg cagtgaaaac cttggaggtg catctgttta ccatcagaag tgcttctgac 32520

acattttccc aattattcat tgtgataaaa cagacatagt ataaaattta ccactttaac 32580

cttttttttt tttttttttg aaacagagtc tcactctgtt gcccaggctg gagtgcagtg 32640

gtgcgatctc ggctcactgc aacctctgcc tcccgggttc aagcgattct cctgtctcag 32700

ccctctgagt agccgggact acaggtgtct gccaccacac cccgctaatt ttttgtgttt 32760

ttagtggaga tggggtttca ccatgttggc caggctggtc tcaaactcct gacctcaggt 32820

gatccacctg ccttggcctt ccaaagtgct gggattacag gggtgagcca ccacgcccag 32880

cccacgttaa ccatttttaa gtatacagtt caatggtatt aaatgtatta ataatgtcgt 32940

gaaaccatca ccactatcca tgtctggaac tcttttcatc catgttgtat ctgcatttcc 33000

ttctttttaa gactgagtaa tattccattg tacaggcaca ccacattttg tttatccatt 33060

catctgtcag tggacaccca agttgcctct gcttcttggc tgttgtgagc agtgctgccg 33120

tgaacatagg tgtgcaaata cctcttgaag acctttcagt tcttttggat gcaaacccag 33180

aagtgggatt gctggatctt atggtcttga ggaacctcca tcctgttccc aacagcgccc 33240

acaccatctt acattctcac cagcagttca ggaggactct gggctcccca catcctcgcc 33300

agtgcatgtt gttttctgtt tttctttctt taatttttat tttttccttt aaactgtttt 33360

cttgatgttt tctgtttttt tgacagtggc catcctagtg gctgtgaggt ggtcttatac 33420

gctttacaag gggagctgcc cttccgtctt aacactttgt gctgacaggc caattccatg 33480

tgtactctgc ctgcttccct gtccttggta actcaggcat cagctttttg ataagacaca 33540

aaacagaaag gagcctctcc tcccataccc ctggcctggg cagtggtcac tgctacccat 33600

ggcgcccaca ctctcctgag agcagtcact gctacccacg gcgcccacac tctcctgaga 33660

gcggtcactg ctacccacgg tgcacccaca ctctcctgag agcagtcact gctacccacg 33720

gcgcccacac tctcctgaga gcggtcactg ctacccacgg cgcccacact ctcctgagaa 33780

cggtcactgc tccccacggc gcccacactc tcctgagagc tgtcactgct acccacggcg 33840

cccacactct cctgagagct gtcactgcta cccatggtgt gcccacactc tcctgagagc 33900

ggtcactgct acccacggcg cccacactct cctgagagcg gtcactgcta cccatggcgc 33960

ccacactctc ctgagagcgg tcactgctac ccacggtgcc cacactctcc tgagagcggt 34020

cactgctacc cacggcgccc acactctcct gagagcggtc gctgctaccc atggtgcgcc 34080

cacactctcc tgagagcggt cactgctacc cacggcgccc acactctcct gagagcggtc 34140

actgctactc atggtgcgcc cacactctcc tgagagcggt cactgctatc cacggcgccc 34200

acactcctga gagcggtcac tgctacccac ggcgcccaca ctctcctgag agcggtcact 34260

gctactcacg gcgcccacac tctcctgaga gcggtcactg ctacccacgg tgcacccaca 34320

ctctcctgag agcggtcact gctacccacg gtgcacccac actctcctga gagcggtcac 34380

tgctacccac ggcgcccaca ctctcctgag agcggtcact gctacccacg gcgcccacac 34440

tctcctgaga gcggtcactg ctacccacgg tgcacccaca ctctcctgag agcggtcact 34500

gctacccacg gcgcccacac tctcctgaga gcagtcactg ctacccacgg tgcgcccaca 34560

ctctcctgag agcggtcact gctacccacg gcgcccacac tctcctgaga gcggtcactg 34620

ctactcacag cgcccacact ctcctgagag cggtcactgc tacttacggt gcgcccacac 34680

tctcctgaga gcggtcactg ctacccacgg tgcccacact ctcctgagag cggtcactgc 34740

tacttacggt gcccacactc tcctgagagc ggtcactgct atccacggcg cccacaccct 34800

cctgagagcg gtcactgcta cccacggcgc ccacactctc ctgagagcgg tcactgctac 34860

tcacggcgcc cacactctcc tgagagcggt cactgctact catggtgcgc ccacactctc 34920

ctgagagcgg tcagtgctac ccatggtgcc cacactctcc tgagagccgt cactgctacc 34980

catggcgccc acactctcct gagagccgtc actgctatcc acggcgccca cactctcctg 35040

agagcggtca ctgctactta cggtgcgccc acactctcct gagagcggtc actgctaccc 35100

acggtgccca cactctcctg agagcggtca ctgctactta cggtgcccac actctcctga 35160

gagccgtcac tgctatccac ggcgcccaca ccctcctgag agcggtcact gctacccacg 35220

gcgcccacac tctcctgaga gtggtcactg ctacccacgg cgcccacact ctcctgagac 35280

tgtgctgagc ttgtgctggt ttccatcgac tgcctgctgc cttccattgg acccactgat 35340

cgtggtttgt ctttggactt ggtgatagta tttcatgcag aaattttaag tttttctgtt 35400

gtcacaccaa tcactttttc ctgttatgcc ataatttctg ggttatctgt ttttttatct 35460

ttgagacagg gtcttgctct gtcgcccagg ctggagtgca gtggtgcgat catggctcgc 35520

tgcagccttg atctcctgga ttcaagcaat tctcctgcct cagcctcctg agtagctggg 35580

attacaggcg ctcgccacca cgcccagcta atgattattt tttttagggc aaggactcgc 35640

tatgtggccc aggctagtct tgaactcctg ggctcaagtg atcaccctgg cccctcaaag 35700

tgctgggatt acaggtgtga gctacggcac ccagactgtt tttttaatct ttgactatct 35760

gctgagttac acatgatctg actctgtttg catttgctgt gatcagtggt cttttcacac 35820

atttacctga aaagcctcgt gtcacagcct catttgagat gagcgtgtct gtgagcatgc 35880

ccatgtgtgc tgcacaaatc gccagcatgg tccttgtcca ctttcacgtc tggcttttct 35940

cttctcttat ctcttcttac cctccagctg gtttgtgtat tagggatgtg atcctctgtc 36000

gcttgtggtg cagattttct cagcttctag tgtcttttaa cttgtgtgat gtacaaatgg 36060

tttaagtttt ttttaatgta acttaaatct tttacactta gtggcttttt gatttattgc 36120

atgttcagaa aggcctgccc cacccccaag attttttttt ttttttttta agccctgtat 36180

tttccttatg ggcgtccaca agtcggattt tcttttactt ccttgggctg gctgcacttc 36240

ttctgcggtg tggtggagat gggttgcccg ggccccagtg cccatctcga ggcactgttc 36300

tcactcggcg tcctgagttc tgcctgtgcc tctggttgtc tttcacgtag gcttctctgg 36360

gtggagtctg gatgcccctc cagcccctgc aggatgcttt gtctgctgga ccttcaggga 36420

aatggccttg gatgtctggc agccccagga tgtctgctgg gcccctcgat gtactggggt 36480

cttcggtgac ggtcactgca gcccagcact ttcaccaccc tcgttgtgcc tgttcccctc 36540

agaggctgcc cgcttgggcc tggggagggg gcccggtgct ctgggtgccc tggccttgct 36600

gtctccctag caaggtggct ggggcttggg gagcgctcct ctgcaaccct gtgctggggg 36660

cagctggctc ggccgggaag acacagctcc agatattttg ttcagaaaag aaactgcagt 36720

gtttattttc ttcagaaaaa aaacatttag aagatttttt ttaaagctct ttcgtttaga 36780

aggaatctag gtatgttatt gtttaagaaa aaaagtgttt gcaatgtatc agtcacccgt 36840

ttcattctga gcatgattta tgtgaggaaa tggttttttt aaaaattaag ctggagatat 36900

atccctgtac gatgcttctg tgaaaatgcg gcttttgtcc ggcgctgtta atgcaagttg 36960

taacagtgta atatgggagt cagtgtttac atgttacatt cctctgctgc agtcaaaata 37020

agcccggagg gtctacagta gcatttctgt attttatcag cttgggctgg ggctgagggg 37080

aggcctccac tcacacttgg aggggctgtt tctgccagat ttatttgcct tataaatatt 37140

ccccatgttt attttaactc gcctttaaaa tggagctgaa attaatatgg accccggggc 37200

tgctcccctg gcccctgagt gcctcccgta gtcgggtggg acatttctac ctggtcccat 37260

cctggaggcc aggtgcgcaa ctcacggtgc ctggcatcac agtgcccctc ctgcagtggg 37320

cactctgtcc cctggttggc tcctccacca aggaggctcc ctgtccccct ggccccaggc 37380

cctcggtggg ctctgcggcg tgcacgaggc ctccctcgtc cctctctact acaggcacag 37440

gctgccacca ggaactcctt tcagcgcacg tctgtgtggg gcccactgcc gggagctcca 37500

gatctggcga gtggtgccca cagtggggcc tgggacccag cctgcaaggt cgtaccatgc 37560

cacttttgcc ccagagaggc cactcaggca tcacagtgca cctcgggccc caagttggga 37620

gccccccttcc tcctttgttt gcatccaggt cttagggagc ccagggctgg acagatggca 37680

gaggcagggg gatcgtgtcc aagctcagtc aaggggagac agaattaaac agtgcacttc 37740

actaaacagc catatgctgg caaaatgggg ccatacctgt tgaccagagt gggctgagca 37800

cctggtttgt aacctaggag ggtgaggaag acgtgcagaa aactcagaaa actcctgaaa 37860

aagcagtgtg agcttgtggg tgggtgagca ggacccttga gtgcactggg gtggggcggg 37920

ggacgttctg gctgccccac tcccatagcc acgccctggc gcaagggact gtcctcccag 37980

tcttggtggg gaccctggga aggactccag gtcggctcct gctccgcctc tgccatgcag 38040

atgcccgcct tgtgccagcc cgtttgcctt ttctgctgtg ttgtgtcttt ccttctctgg 38100

aactgcaaga caggttgaag aagagaggag gtttctgggg aggtacagtg accctgggca 38160

ggcagtggag cccctctggg aacaatggcc actgcaggga ggcaggggtg cggaggggca 38220

cgtgacccca aatgactagg ctcagagggg gcatgcagcc ttgcaaacca aaccttccat 38280

tccacagagc aagccccegg caggtggagg aatgtggaag gcgaggatgg tgagcccaca 38340

ccggggagct cctttacaga ggcaggcact gggggtcggg atccaagtgc agccaagagg 38400

agcccgaggc ccagaggggc ctggcaatta agggggccca aggagctgag ctccaactcc 38460

agtcccacag ccgcacaatc ttcctgccac ttcctgacct ggagggctgc ctccaccggg 38520

aagcctccgc acccggcagg gacttgggcc ccgccagtgt gccttgtctc tgcctcttgc 38580

acacccacgg ccctgggctt cgtcttctcc ctcagtgcca ggttttggag ctcaggtgcc 38640

ctaaccccaa tatagggaat tgtggtccct ctattctagt cctccctctt gctgtgtctg 38700

taggtccgct ttctgcaaag cagggctctg agctggcaac actgggatgg ggccgagggc 38760

gtgaatgaga ccgcagcctc ctctcaggtc cgataacgga agtactgctt cccggaaaca 38820

accagggtct gcctgttcct gctgccagct ctgtagcccc tgcccaggtc cccgtggggg 38880

tttacagagc atggggccag cctctcctgc agctgccagg ctggctcagg ggctgaccag 38940

cccacacact gggccctggg ggaggggctg cagccagcca ggcctgtcca ccctctgagg 39000

ctgctccacc agcctgctgc tgtctgtcct cgcactcctg gggctccaag aggcagagtg 39060

tttttataac ttgggctggt ttaaggctgc ctgtcagtgt gtcatggaaa gagcttttat 39120

ttgaccctta ggaagtccgg aagccagcca ggtactgcga tcatttttct ctcttcctaa 39180

gcacaagagg gaagaagata aattttatct tgggaagaac cacagccatt tgggagaaaa 39240

tttaatttaa gtggcacatt ccgctggtgt gatgggatgt ccttgtgttc agcaataggg 39300

agaccagctg tcctcctggg gcttgttacc agactgcaaa gcccctgcca cctctctgtg 39360

tgcgtccctg tggctaagac atgacaaggt agggctttca gtggcatttg ttacagggcc 39420

agcacacaga ggctccggcc cagcatctgc ccctgagggg ttgggtgggg cggcggggtt 39480

ccagccaagc cggggaagga ggccttcgta gcacccccag ccctgtgctg cctccctggg 39540

gcatctgcag cttggcggcc catctggtat ttcctgggct ggcaggacct ccaaggggcc 39600

aggtgcctcc catgagactc actttgtgtg agtattccct ctggagagca ggccttcaag 39660

ctcaagaggc agatggattg ctctttaagc ctaggcttcg tgggcctgaa ttaatgcttt 39720

tgtttctgct tcctcccccc attccaattt tgaacaagaa agcctgccct gtgattaggc 39780

tcatcctgca gtagttatta tgcctctttg gaaaatgttt gcacaccaaa aagcacagag 39840

agctgagctg ctgggatcac atggaaaact gatttttatt tgttttgttt tgtttgtttt 39900

agagacaggg tctcactctg tttcccgggc tggagtgcag cttactgcag cctcaaactc 39960

ctaggctcaa gtgatcctcc ttcctcagcc tcccgagtag ccgggactac aggtgcatac 40020

caccatgccc cagtaatttt tttagtttgt agagatgggg acaagctgta ttgctcaggc 40080

tggtcttgaa ctcccgggct caagcagtct gtccaccttg gcctcccaga gtgctgggat 40140

tgcacacatg acccaccgca cccaacctct gggtctttta atgcagcggt aagttgagtg 40200

gttccagccc cctaccccca ggtcctgtga gctggtcccc tgacttctgt ggccagcacc 40260

tctgacttcc tgtggccagt catggcttga gttggttgct gcaccacgtt cctgtctgtc 40320

cctacccctg ctgggccgtg aatggcctga agaaggtgtc tgtaggtccc tgtctttggg 40380

tactctctat ccctgtgatc tggaggcttt ggtgtctgtc ttattttgct cgggcatttt 40440

tttactttag ctggtctgaa cggagttctc tgttggaatt ctcgtatcct gcattcttca 40500

aagggtaaac attcacataa tttgatcaat gcggggaggc gtacagtgaa aaccaatgct 40560

gtcaataatc cttgctcatg acaacaagaa gaggcccaaa atgaccctcc acacgagcga 40620

gacgtgagct tttgttgaga gatttcagcc gaacacatag ggtcaaggat gcccactcgg 40680

gaatatgccc actgcaccca cctcctccta gctgtaccct cagttgctga ttcagagctg 40740

gcagctgagg gatgggggca cctgtgactt tgaaggtggc ccttgaggcg ggagcttctc 40800

ctgtgccccg tgggtgccag gacaggaggc ccgggcactg ggggactccg agggagggcc 40860

tggagagtca cctgcccccg ctccaatcca ggcctggtgg gtcagcatcg tgtaggaggt 40920

ggggatgtac acgtcggtgg cagagacagc aaacatttgt ggcaaaatca ggagggtgtg 40980

ttgctgtatc cctagcaatg agctaggagg cccggagagc agcctggccc ccatcctgca 41040

gctgcagggc ccattcctgg agaagggttg gccttcaggg ccaggaaggc cctgacgtgc 41100

aggggcccag ctgcccacag aggggatgca ttggcctcac tctgccagtg cctgcacagc 41160

ccagcaggaa gcatctagcc catgcacagc cacctctggg agcaggggc aaagggcacc 41220

acacaatggg ctctcgccca gttacaccct tgcatttagg gcaggatatg caaagcagca 41280

gagttctgtt tatatttgca gtaactcata acttcatatc tttttttttt ttttgtgatg 41340

gagtcttgct ctgttgccca ggctggagtg cagtgacacg atcttggctc actgcaagct 41400

ccacctcctg ggtttacacc attctcctgt ctcggcctcc ccagcagctg ggactacagg 41460

cgcacatcgc catgctcagc taattttttg tatttttagt agagatgggg tttcaccatg 41520

ttagccagga tggtctcgat ctcctgacct cgtgatctgc ctgcttcggc ctcccaaagt 41580

gctgggatta caggcgtgag ccaccgcacc tggccataac ttcatatctt aaacaaaagc 41640

ttgtaccttt cactgcatat agcaagtcca aaaagagttt ggtttcgcca ttttggaagt 41700

gcacttccgt gtagagatgt gtatgtggtg cctatgtgtg tgtgcatgtg tggaagtgcg 41760

tgcataccgt ggtgcctgtt tcccaagtgt tgccggccct gagcggggct gggaatgcat 41820

ctctgcaatc ctgcttggct gggggtccca gggtccccct tcgctgtcct cacatgctga 41880

gtgaggcatg taaccacctt gtgctcagcc ggccaacaga gctctgtctg ctcaccctaa 41940

tcctgacagt ggtgcggttc tgtccccagc cacagtgagg aagctgcatg ccagatgctt 42000

cccgcagggc acctgcagaa tgattccaag ggatacaccg tgttttgaaa tggaattata 42060

tcattagtac ctgctgggat tagcaatgac attgggctta tccgtcctta catcacctcc 42120

tcttgttcaa agactgaagg gtaatgtggg agcccaccca cgtgcagctg ccccgctggg 42180

agttcttgtt cgtgttaggt tctgtgcccg tttgcatgtg tgtgcatgca tgtgtctaca 42240

tgagtgtgtg catatgtata tatgaatgaa tgtacccatg cgagtatgtg cgtgtgtgtg 42300

tacatttgtg cccgtgtaag agtatgtgca tgcaggtgtg tggcatgtgt gcacatacaa 42360

gtgtgcgtga gtacatgtgc atgcaaaaca catgtggagt gtggggttga acaatagagg 42420

gttttcacta caagagcaaa tatttccaat gactgctggt cgcagtgtcc tgtgctgcct 42480

cccttgtccc tggggtcctt cagtcccctc tctggggagc tggccctcct ggccctgccc 42540

ctagctgtga tagggttgga tgtgcctctg gcacatggaa gggcccagcc ttctgtggtc 42600

ttgagagtgc tttgctcaga tgatgtcttc ccttgcggtc tggtggcctc cccatcctgc 42660

caagcatctc ccagcttcca ccctagccca ggaggccccg gggggagaga aggaaagcca 42720

tgtgctgctc tggtggatgt ttctccatgc ctccgggtgc cttccagggg acaggtacca 42780

cttgtcactg acacacacgc ccttcaccac caggcgattt gctgattcac aacatgcttg 42840

acagtgtagc cttggaaagt gggctttgtc ccctggggca agctgtttga acagtaacct 42900

tggagcccac gagctgacca agggcctggg caaaggctgc ggggttctgc tctgactgca 42960

cttggtcaat aagggcctca gctttacatg tgctctctct ctgtttctct aacaggttgg 43020

tggtcggggc cagctgtatc ctttgcaagc gtgcaggtgg ccgtcctggg aactggacac 43080

tccttccctt gagtccctcc ctgccacccg cccttccggc aggctctttg ctgcctcaga 43140

tcggcactta ctgggcctct cggcgtggag gatctactgt gcctggggac agtttgaggc 43200

tgacctctgg ccagggattc ctggaggctc ttcctgagct gggacagctg gacatggcca 43260

tgaggcagct gggctgtcct ccctcaagag cagccccagc ctggacccat tgcttcagaa 43320

gggacaatta gacagggagg gtcaggagag gagctgcagg agggcctggg gtcaagggga 43380

catctgaggg agatgaaagt ccctggccgg gcgcggtggc tcatgcctgt agtcccagct 43440

acttgggagg ctgaggcagg ggaaccgctt gaacctggga ggcagacgtt gctgtgagct 43500

gagatcatgc cactgcactc cagcctaggc aacagaatga gactccatct caaaaaaaaa 43560

aaaaaaaaaa aaagccccaa gccgctggct gagagcacag gtggaagaaa gcagctgcct 43620

ggcatctccc gtggctgagg tcctccctga accagccact cctctccatc ccaactggaa 43680

tccaccagga tgcaggcctc gccctggagc cgggatggac agcacatggg agcagaggcc 43740

agaggccggc ctagtccgtg ggtgctgctt ggcggctcct tgctaacccc agcctgtgca 43800

cttgagatgt ggaggtgggg gtcttgagtt gggagcctcc tccagggcca gccatggatg 43860

ggtggggtgt gggctgtggc acagggccct ggggtgttgt cccatcattt ccaaaattgg 43920

gatctacagt gctccttagc tggcacccca cagatgtggc cctggagtgt gctggcttcc 43980

tcaccgggat tgggctggac accaccatca tgatgcgcag catccccctc cgcggcttcg 44040

accaggtagt gctggaggcc ccagctcccg cccctgtggc tctgaggcct tcctcccaca 44100

gccccctccc aggcaggtgc agtgcagtca tgggccacga gtgatgcctg aatctttaga 44160

gataaccctt gaattggatg aggaggttgt ccaggaaata tgcagacact cggccggagc 44220

gagggaggag ggtggcagtg gggcgcacag ctgcacagag gcctccagcc gaccacaggc 44280

acagtgaagg gaggctcagc ccagccttgt gccggcagag caggtcaccc tccgggtcgt 44340

cccattagta agaggccggt gtgtgctgca gaagtgttag ccccacctca gagacccccca 44400

actccctgct ctccccttgc ctgtcccctc tgagggagtg gggtgaaat aagctgtaat 44460

gtgtgagctg gggtgaggga cacagggcag cgctggctcc gagacagccc gtgggatctt 44520

ggcttctgga caggtgcgct gtgtacagtg gccttcatct gtgtctgggg tacacacact 44580

catgtggcca gcaggacgag ctcctagatt ggagcaatag agattttctt tttttagcta 44640

aaaactcttc aagggaaagt ctcctgttaa aatagaagtc tggagttgtc attgtggaga 44700

tgtcaaacac tccatgactg ccacgaagga ggccccttgc agggtgagct gtgcagatct 44760

gcaggccctg ctacaggtcc tcggccgacc ctcagctcac agagcctgag gaggtcacct 44820

gaggtgggag gagccaggcc tgtcatctcc agactcccag tgccaccccc aggctgactc 44880

agagacgtgt ccctccaccc gtgtggccat gtgttgttcc atcctaaagc tatggcacac 44940

tcccatcatg tccctcagtg caaacccacc gagccctgtg gtagggacgc agtgtctagg 45000

aaggcctcag agccttcaga tgtggggagg gtgagctggc agtggacgga ggaggaaagg 45060

ggcctgcaga atgggggccg tgcccaggca aggaggatgc acagtctagg cagccaggct 45120

gaggggcgag ggaccctgtg tcctggacag gctccatggt tctccctcag agcctgggat 45180

ggccatgccc tccaggcctc tggaggggtg caggagtgat gaacagcctg tgcggcagcc 45240

cctctgcagc acagccagcc ctgcccggcg ggccagactc ccacacacgc catctagagc 45300

cagctggcac acccgcacag gtgtcagagg atgttagcag tgactgtttt tctcctcttg 45360

aaaccagaag ctgtggccag cttcactgct ggtccgtgca agtccccatt gtcctgggaa 45420

cagggccccc atctgtagct ggacttgggg cgcacaggcc tcctgggccg ggagggagaa 45480

gcaggactct ctctgtccct gatctggacc tgagcctgct gtgcctacat ttctaggcgg 45540

gagcagagtc tcctacctac cctcctggct cctggatggc agaggggcat gttggccaca 45600

tgtggagggg tgggccagga gcaggggcca tcttgttgga ccagggcaca gctcctgggg 45660

gaaggcacgc ttctctgcca ttggttttag tgaagactaa gttttttgtt tttgtttttt 45720

aaagaaatgt gtagatgtac caggatttgt ttgtttttct tcttttttttc ttcttttttt 45780

tggtttgaga cagagtctca ctctgtcacc caggctggag tgcagtggca caatctcggc 45840

tcactgcaag ctccacctcc cgggttcacg ccattctcct gcctcagcct cccagttagc 45900

tggaactaca ggtacctgcc accacgcctg gctaattttt tgtgtttttt agtagagaca 45960

gtgtttcacc gtgttagcca ggatgatctc tatctcctga cctcgtgatc cgcccgcctc 46020

agcttctcaa agtgctggga tcacaggtgt gagccatcgc acccggcctt gcttattttt 46080

tcttttagag atagagtctc actctgtcgc ccaggctgga gtgcagtggt gtgatcacag 46140

ttcattgcag cctcaaactc ctgggcttga gggattctcc cacttcagcc tcccaaaagg 46200

ttgggattac aggtgtgacc gtatctggct gatgaagact tagttaatta gcatctagag 46260

aggagcccac aggctgagtg aatcctgacc atagggccca gggttgagga cttggagcca 46320

ggacaggggc tagacactcc acagagagca agaggacgta gagcaggggg cagggtgtct 46380

cacccccgtct tccgcacaga cccctctccg cccttcattg aggccttcga gagcagggca 46440

gggccagagc ctctctccca cggccacctg gtcttcatgg cctgtcttct tgtgctttgc 46500

agcaaatgtc ctccatggtc atagagcaca tggcatctca tggcacccgg ttcctgaggg 46560

gctgtgcccc ctcgcgggtc aggaggctcc ctgatggcca gctgcaggtc acctgggagg 46620

acagcaccac cggcaaggag gacacgggca cctttgacac cgtcctgtgg gccataggta 46680

agggcacgtc gagccacacg ctctgtctct ggtctccccg aggtgcatgg agaatctttg 46740

ccccacttcc tgtcacctcc cagggctccc ccatcctgct ggctgccagg cgggttggcc 46800

gctccccagt gcacctcgag agcaaccgtg aaggcctgtg gggcggcact cacactaggc 46860

tgtgcccatc ttgccatccc cagcaccttg catctctgcg tgtctcccca ccaccgtggg 46920

acatgctgga aaaaaccaga gaagagactg agacggcatc agccaggtgt cctcatcgag 46980

gatcaactag gcaatcatcc tcgccttccc tggcccttga gcaattgctt attaaggttt 47040

cagcacataa atcctacttg tcaccttcca ggtttaagtc ttggtatgtt tcttttcttt 47100

tttttttttt ttttgagatg gagtctcgct ctgtcgccca ggctggagtg cagtggcaac 47160

ctccgcctcc cgggttcaag cgattctcct gcctcagcct cctgggtagc tggggctaca 47220

ggcgcccacc actacacctg actaattttt gtatttttag tagagacggg gtttcaccat 47280

gtaggccagg atggtctcaa tctcctgacc ttgtgatcca cctgccttgg cctcccaaag 47340

tactgagatt acaggtgtga gccaccgcac ctggccagta tgtatttttt ctttagcata 47400

ttaaatgttt tgctgcttca ggctttccag ctaggttttt ttcttttttt gcgtgtgaaa 47460

cagggtctct ctctgttgcc taggaggagt gtggtggtgt gatcatagct cactgcagcc 47520

tccacctccc aggctcaatc aatcttccca tctcagcctt ctaagtgctg ggattacagg 47580

tttgagctac tgtgcccagc caagctagtt tgttgttttt aacataaaaa tgatatggat 47640

ttgaaagttt taaaaattat ggtgacatac acataacatc aaattcatca tcttaagcat 47700

tttaaagtat atagttcagt ggtgttaagt acgttcatat tgtacaagca gcatgaccat 47760

ccatctctag aacttttcca ttatctccaa actgagctct gtccccatta aacactcact 47820

ccctgctgcc cgggcactca cccttcactg tgtgtctcta tggatctgac tcctcgaggg 47880

acttcatgta agtagaatca tgcagtgttt gtccttttgt gactggctca tttctcttag 47940

cacaatgttt tcagggtgca tccatgttgc agcatttgtt agagttagca tttgtgtagc 48000

atgtgccagc atttccttcc tgttgaaggc tgagttgtat gtgtgtccat tatctgtcag 48060

tggacactta ggttgcttcc gtcttttggc tgttgtggat catgctgctg tgaacatggg 48120

tgtgcacaag ccgcctgttt tcagcacata aaaatgacac agagttttta aagttctgtc 48180

cagccttctc gtggctttca gtgtttccca gtgggtccct ttgggcctgg caggtcatca 48240

catgccagga gtggcatttt gcaggccttt ccagaagtca catttcgaag gcttccaaag 48300

acatcaccct tccagtgctg ggcagaggcc tgggcgtttc ccggccaggc gtgggctggc 48360

tacaacttgc tgggcacagt ctggtctcac cttcttgctc tctgtcaggt gtaggtccgg 48420

ttttgcatgg ggcagtggcc gagcaggtgt ggggagtgtg gacacagcca ggggatacct 48480

gtgcccaggc ctgggccggg gctgtctgac gggactggca aggggcagct ggagccaaca 48540

gggatcgccc agacactgct ctgtcccagg cctgctgcag aggaggccat tgcagatggg 48600

ccgccgcaag gagggctgcg ggttctcgtc agggtgggtg gcggcacaca cagcatgcct 48660

gctcagatgt cctacccacc tcggtgcctg gccgtctagt ttaggctcct agttttccca 48720

atcaccctgt caacttgcat gttgaaggcg ggagcaacct agtcagtgtt gttagttctc 48780

cgaggccttt ctgtgctgag cctggtccgt tgtcccctgc gagagcccaa gatcagagcc 48840

agggtccctc tcagatcacc tgcctccacc tccatcagct aaccgggctt gcgtggggct 48900

ggccaggggt cagtgtctcc tgggggaggg ctcccagagg cacaagctgt gtcatagggt 48960

gatgcacttc tgaagcagtc actcggaatg ggaaatcaga aaggaaacca gcctggagcg 49020

gctgaggctt cacttttgtg cagtggacac aagcgcagag gtgatgaccg gggaggacct 49080

gagatgcctg agcgcacgca gggctcttgg cccggaggtg atcagtcagc aagagcaatg 49140

ttctcagcca cgttgtaaaa gtagatttta agtaagttta ttatgataaa cactacgaag 49200

gattaggtaa cattttggac tctggagtgt actgatggtt ctcatcctaa actccacttt 49260

atttttcttt caaaatttat cctcctactg tcattcccaa ataaactcca ctctaaaggg 49320

gaggtggcga gtttctccct cccaccgcgc cctagccctc ctcctctgtc tgctgacacc 49380

tccgttcacc cacagcataa gggagctgtt gccattgaac cgaggggcag ccctcgacca 49440

agcccatagg gatgtagcag accaatgggc cgggggctgt gtccccggaa agccggggcg 49500

tcagcctggc aggcaacaag cgaggcccct ccctactcag cagagctgcc gtggcctgca 49560

cgcccatcct cccttgctag gagtctgttt tattttttg tactttcaaa atgagcatcc 49620

cccagacagt ctgcctggca tgattgatgc tgggggtgga agcttttcac agtccttggc 49680

ctctgaccct gcttcccgag ggcggcgcgg cttctgcgcg cctgctgggg gccgctcggg 49740

catgtgctga cttcgctgct gctcagagcc aggttttgta aacattcagg agccacttcc 49800

caggtcagca actgcgagcg ttttctgctg gtttgtacga taagccttta accaagcttt 49860

atttctactt gcaataaagg atgatccact ctgggaggtt aaaataaagc gccctagggg 49920

cggcacagct ggcaatgcca acatctccga ctctgcttcc tgtaccgggt gtgcactacg 49980

tccgcacaag ctgggctccg acaggaaggg gggatgcctc cgtgcccggt gcacacacac 50040

acagaagggg cccaggtgct gcacagagct cccgtgtggg gccgagtgct ggccctgccc 50100

gttgcccgtg tgcctggcgc ctggccgcag ctggcgagga ccatggacat tggcattgcg 50160

aagtgggccc tgcagtctgg aagcagagga ggaccagagc cccttcctcg acgcagcctt 50220

gatctccacg tggtggatta aacatattag cagttaaagc agttaattgc tgtgcagggg 50280

gcccccgctc attgtttgtc tctgaatcac ccgcccacac caggtgtctc agataataga 50340

ctgggaactt cagtgaggag gatttcctgt ctgcagatgt gccgattaca gcgctgagtg 50400

aagacagtca gccagcactg ctgcgggctc gaattcgccc gctgagttaa ccagttgtgg 50460

cctgaatccc tggctgtcta ggggagcagg gccaggctgg ggcatcctga gcaaacgcct 50520

cccagtgcag gggcttctaa gtgcagttaa gtttagattt ggttttaaag aagggacaga 50580

ctgcctctgg actgcagccc tgaaaaactg gaagttgaga tcttcatgtg ccccttttggc 50640

gtccccagcc cctctggatg gttctctgtc cctctcttga gggtgagatg ctgaaggctc 50700

tggcgtatcc ctccctgcac cacaggagtc ctgcccatgg ccagaagagc cagcagacac 50760

aggaggggac tcgctaaacc tgggggtccc acagccaggc agctgttgtg cctgagtgcc 50820

agggagggtg gccacaggtc tgcctggggc cttgtgatat ggcaggtgag acgagaccca 50880

gggaaggaat ccgtctggat cttaccccag ccactgaagc tatttcctct ttgggcttta 50940

tcttagataa ctcagagatg tccacctgtg tgtaaaccag catcagagcc tgaaacacaa 51000

catgcatgga gcatgggagc ttggcttccc ggcttcctct tggcaccaca cagaggccac 51060

ccccacagcc tgcccctgta tagtctcccc cacgtggcac gtgtgcctgg accacagcca 51120

gagagactgg ggtgcccagg agatgatgat gggggctggc ggtgcccagg aggggagctg 51180

ttgccagggt gggctgcagc cctgggctcc tctctccacc caggcactgt ttgctggcat 51240

gtacacggca ggcaactgtc tttgctcagc tgtgctcggc tgatggctcc cgtacaccgc 51300

agactcagct acacaagtcc ctgcggattg tccctctgtt gagtgccagg cagcaggaaa 51360

gggcagaaag caagaaccaa gctggggatg ggtgggagca tcctaagcct ggtgagaggg 51420

aaggggcggc tcctcctgga tgccctctgt gctggtgtgg gtgcagctgg ggcttagggg 51480

ccgcggtggg tgtggatgct gcccgggcga gggggctgcg gtgggcccat caggactgct 51540

ctcagggatg cgccttgctg cagctgcgtg aaaccaagtt tgtggctatg ctcttcccgg 51600

tggggatggg cccagcagtg accctggcat ccacatgcct ccatgctctc agggtggagt 51660

ggccatccct gagcaggtga cacggatcgg aggcccctct ttgatagtct aacacctttt 51720

tattttagtg caaatttagt ttgtaattaa tgacatcaag agagacccaa atctgcctcc 51780

attgtgagtt tgatattttt tgaagtgggg ccaagatgaa cattcatgga gctcttccta 51840

tgagcggtgg ctgcggcctt cccctccaca ccacgtggcc ccgggcgtgc gctgctgctc 51900

ctgagacagc actgttggct cagccctagc tcatggtggt gctaacacct cacagaggcc 51960

aggccagagt agcaggaggc agcgccctgt gcttacccac cagcctggtg ccccacgctt 52020

ggcttgaggg ctagttcaca cttatgtcca gagcccacag ctatctgaca ggccagccgg 52080

cacctgcata catggttgtc caactgggtc ttgttaccat gacaaattca gtacttaatg 52140

attagaactg agtggaaacc aattaaaaaa aaaagaacaa catggtgaaa agtcttctag 52200

agataggtca gcaccattta tgcatattac catgcaccct ctaatgtctg caggtgccct 52260

ggggcagcca acctgttaca attgcaatta tgttaacaat tgttaaaggg ccgggtgagg 52320

tggctcatgc ctgtaatccc aacactttgg aatgccaagg tgggaggatc atttgagccc 52380

aggagttcaa gaccagcctg agcaacagag tgaaacctct tttcaaataa ttaataataa 52440

taataataat aataattgtt ttttgtttgt ttttgagacg gtgtctagct ctgttgccca 52500

ggttggagtg caggagcgcg atctggctca ccgcaacctc cacctcccga attcaagcga 52560

ttctcctgcc tcagccttcc aagtagctgg gattacaggc acccgccacc gcgcccagct 52620

aatttttgta tttttagtag agacagggtt tcactgtgtt agccaggctg gtctcgaact 52680

cctgacgtca tgatccgccc gcctcagcct cccaaagtgt tgggattaca ggcgtgagcc 52740

actgcacccg gtctaataat aactgttaaa gcaataatga ccactcgcca cagagcacgc 52800

tccttcctgg gggtcctctg ggcctgagct gggggctgcg ccgtactcca aggctgactg 52860

tggggtctta cacatcataa tgcacatagg cagcttgagt aggaaaggcc cttatgctgc 52920

ctgaggggaa gctcacccca ccccccaagg agccgccctg ggtatgagac atccctggaa 52980

cggcctaagg ggtccctggt ggctgctggc agtcacagca agtggccaat caggtcccat 53040

tgaggcccag gggctcactc atttattcaa ctaacatggc gtctcgatgg gacctgaggc 53100

cagcagggca ggtgcgtccc cttccccctg gtgggctcat agctgcgggt aggggcccgg 53160

ggctcattga gaaggtgcga ttccagaaaa aaaaaaaaaa agaagataaa tattttaaaa 53220

taataagctt caagaatcta agtccagttc caaaggcata cgctcctctg tgcctggtcc 53280

aaggtgcctc actggggcaa gtggcaggcc aggccccgtg agggtggctg gctctggggg 53340

ccacatgcct catgagacag tcgccaggtg gcccacaggg cctgtgtgtg aagccgtgcc 53400

cgcctcgcat cgcccaccgg ccctggagcc tcccactccc acaccctcgt cctcaggcgc 53460

agtgcttggc cctggctgcc tctgtctgga tcacagccac tggctcaccc tgctgtactc 53520

ctgctggcac ttacctctca cctggggcgc tgcctccttg ctcttctctt gggaaaatgt 53580

gtcccaggcg ggcgtgaaat cagagggcat gcctgttctt cccatcatgg atgagagagg 53640

cgcatgatac tgcatgcggc tggccgtccc cgtgtccctg tgtccatcag aatagatggg 53700

agtgacccat ggtgactgtg tgggtggttt ttgggcttca gccttctctg gtccctcctt 53760

ggggccaggc tggctggaca agcatggtgg ctgccctcca tctcctgggc cattgacagc 53820

agctggtggg gctctcattg tgatcagagg agggctgccc tgcacggctg ttcctagcac 53880

tggccacaca tggagatggg ctgtcctgcg tcaggggtgc tgcactgctg ggcctggggc 53940

tggaggcagc tccgggctgc agagatgctc agcccagtgc ttcctgagtg tcagtgttgt 54000

gggccagcca cacatataca taggaagtga gcacgtccat ttgtacctgg aattactatt 54060

tttttggcag aaggacaaac tttgttgggc ccatcagccc caattctgaa ccaagtccag 54120

tgggcagcaa tgagacccac tttgctacca gcagagacat ctctgcagtg gcaggttcgc 54180

caacgtgcca gtggttgcca tctctgaagg gacgttcctt ggggcttgcg gccatactct 54240

gcaccatggt cagcagctct tctttgcctt atatgtagtg ggcatggcct gccttcagga 54300

tggctggtca gttcggccac ctctggctcc cacattgacc acagctctgt tggctgagat 54360

gatcctcttg gagggcagct tcatgtaagt cttcctggtt tccgggctag gggaggtgac 54420

tgcggtgtag ttctcaaagg cccaggctaa caagttccag tctcagcctc tcctccaggc 54480

agcgcttgat ggtacaggtg tggtgcctgc agggaggacg ctgccgacgt ccagctgggc 54540

cctcttgcta cagtacacaa acagaaattg gcccgtgtgg aggcccttgg tggtgatgga 54600

cagctctgtc tgctgcttaa actggtgtgg atcctggaga ccatctcagc aggggcatgt 54660

ccttcaaggt gcccttgatg tagctgtgcc gctcagctaa gtccacagca tgcaggccta 54720

tgagccctcc tgcttcatgt gcacaaggaa cacagagctg gtgcccttcc tctgcccacg 54780

aatcccgtgg cccacgactg ctcatgaata cagcctgaag agctgtacct ggaattttaa 54840

tgcccttgtc gtttgcagaa aatatgccag agtgtcatgt atccattacg taataagctc 54900

ttacaaagaa ggaacatggt gaagatgcct ataaatggat gagaaaaggc aggaactggt 54960

aatttacaaa agaggaaata cagttcataa acacgtggcc aaaaattact tcatcttact 55020

agtcatcaaa gaagtgcaaa ttaaagcagc atgacagaga gtgctgtgcg ccgcctgccc 55080

caggaaggaa cagagtctaa ggactgccct gcgcccagaa gcccccactc agctggccgc 55140

ccttcctttg agggtctgcc ccaggaactc aactgacaca tggcaagctg gtgccccagg 55200

tctccatcct ggtggtgtct aggaagcaga gccttgagag tgcctgtcct accccaggag 55260

gggccattca gcagcacttg gcaaatgctg cactgggcac tccagagcca aggcttcgag 55320

gacggagctg cagggagcac agagccgccc catggggagg cctgcagtgg gccacaggca 55380

cagctgggaa agctctgagc acaacatgca gggcgaaaga caccggtgca ggcacccttc 55440

tttccctaaa tggcctgaat agtgcacatt tatcctggca taggagggaa atggtcggga 55500

gctgaccagg cacaactgct ttatcctggc ataggaggga aatggtgggg agctgactgg 55560

gcacagccac ctgcatcctg ctgctcacag cattcgggcc ccagtgggtc cccaccttgg 55620

cacaagtcgt ccaacctcct gcagggctcc aggcctgagc cagtagggcc taggacgcct 55680

gattggctgt ccagcaccag cagggagatc tggcccttct ctggccactg cccagtcctt 55740

tgctcagcaa gacccatagt ggggctcagg gcctggtgtc ctgccatcag ggctggccac 55800

ggctagggac gtggccccac ccaagtggag ctggcctctt ccctgcaccc actcccagcc 55860

acgtcccacc agccagctt ctaaccccac tgtgccctcg gctgccctcc tcagggctga 55920

gcctgctgcc tgccacaggc cactcactct tcttttgctg ttcattcccg aagggctgtg 55980

gagtccctgc agggccaggc gtgtctgtgc cgcgcagccc actctcccct ggccatccag 56040

cctgtccagc tgtcatgcct ttcacattag tggctccatt acattcccac cgacttccca 56100

gagtgatcca cagagcatgt gcggaagagt cctggctttg gatgggccac ctatttccat 56160

gcctctttta tctcttgtga ctacttttaa atttatcttt atttccttcg ggacctgggg 56220

acagggtttg gtcagcacct gcaaggtctg tagttgccat agtgccatag ttaccatgag 56280

gaacatgatg agtgctcttg gcttcccagg accagccagg caagcgtgca gaggagagtg 56340

tgggtgcagc gtcagatgtt ctgttccggc acggagcagg caccaggaag tgctgggcct 56400

ggtgggctgg acaccaggtt ggagagggac cagacggctg agcgtgagcc cccggcctgc 56460

agggaccaca gcccctcctt cgtgccccag ccctgcccat ggggcccagc tccttcctct 56520

gatgtgggct ggcatcccct gtgtctgggc tgatccccga ccggtgccag ccctcccggc 56580

acccaattcc tgcggctgag cagagcacag aggctggagc cggcctcccg caagctggct 56640

tagccaagtt ggtatgtttt gactcctgcc ccgggacagc agctggagac ttaagggttc 56700

cctctcacgg gagcttcagc ttccagatgc ttaggtgggc gccacccgca taccggccag 56760

cagctggttt gtcccagcca cgatgagcag gggagctatg cttttgaggc aaaattgcct 56820

tcgccattgg tggatcatcc tgagcccctg ggagccgaga gcacctgggg ttgggaggga 56880

aaagctgctg tggccatccg ctggcctggc aaaatcacac ccatctgagt taggggagaa 56940

agggacctct gctggctgtt tggctatgaa gaggcccatg cggtgccctc tctccgggcc 57000

ccaggctgtg tggagactgg cagggggcag ctgtgctgac cccctggact ggccatcccc 57060

tgcccctctt ggcctttgca cccaagagca ggatcagctg agccagctgc cccctagaat 57120

gggcacggtg tagtttggac actgcccctt agctcagctc cccttggccc tgaggtctct 57180

aggctgggac tgtgtgccag gcagccacga ggggctgcag cacagagcag gtgggagacc 57240

cggctgctag ctctgctcac ctggccctct actagcgggt tacgggggct tgctttctcc 57300

taatggggag agaccttcaa gccctacctg ggcagagggc cagatcccag gacttgagca 57360

ttgttggggt acagtccagg gtgtggctgg ctcctcttca gcttgtccag atagggagga 57420

ggccattggg agccagcagg tgtcccttga aggaggcccc tctggactct tgaggcctgg 57480

gagctgatgg atctcactgc ctaatggtat caggctgtgg tgctgcagac agatgcaggg 57540

aggccaggca ggccaggtgc caacagctcc ccatgaaggg ctggtttctc cggatgaagt 57600

cagtaccaga gccactggca ctgtgctggt ggccctgcag cagggcctga ggcctgggca 57660

tgcggaagat tctggagtcc cgcgcttagc actctttgat gtcagggagc cccagcattg 57720

gcaagtgcct cttcctttcc cgcgtgccag gaaccagtct aaggccgact ccagtttcca 57780

ccggtggcac ccctgccttg tctcctgtgc cggctgtcat ctgaccagtg tccgtttcag 57840

acctgcctgc cacctcctgc agaggccagg agcccctcta cgctgctggt gcttcacatt 57900

tggccagttc taagtggaca ttcttttttc ttgagacagt ctcactctgt cgcccaggct 57960

gaagtgcagt ggtgtgatct tggctcactg caaccgacac ctcccgggtt caagcaattc 58020

tcatgcctca ccatcccaag tagctgggat tacgggtgca tgccaccaca cccagctatt 58080

tttgtatttt taatagagac agggtttctt tcttttcttt tctttctttt tttttttttg 58140

agacagagtt ttgctcttgt tgcgcaggct ggagcgcaat ggtgcaatct tagctcactg 58200

cagcctccac ctcctgggtt caagcgattc aacctcccga gtagctggga ctataggtgt 58260

gcaccactac gcctaggtaa ttttgtattt ttagtagaga tggggtttca ccatgttggc 58320

caggctggtt ttgaactcca gacctcaggt gatccacttg cctcggcctc ccaaagtgtt 58380

gggattatag gcatgagcca tcacgcctgg ctgagacagg gtttcatcat gttggccagc 58440

ctggtctcga actcatgacc tcaggtgatt tgcccacctc gtcctcctac agtgctggga 58500

ttacaggcag gagccactgc acccggtctc taagtggaca ttctgagaaa cagtttaaac 58560

acaaccgctc taggtcaaag ccactgaaga taacctttca gccccctctc tgtttccttc 58620

aggtcgagtc ccagacacca gaagtctgaa tttggagaag gctggggtag atactagccc 58680

cgacactcag aagatcctgg tggactcccg ggaagccacc tctgtgcccc acatctacgc 58740

cattggtgac gtggtggagg tacggcatgc gtcccgggac cagggcccct gcccctgcct 58800

gctccaccac ccctgctcgc tgggctccgg ctgctgccgt cctgtaggag agaaacgaca 58860

ctttctctga tgacagaggc tctgggccaa accccagggc cagcctgtct ggagttctgg 58920

ggcaacctgg acatgggcct cggctctctg cctttccttt cctttctgtg gtgggagtgt 58980

ggatggtcta aagacagctg caagcaccag cagcagacgc ctgctgggaa tggggcatgg 59040

gtcagctctg cacgcaggcc tcaacccctg gcaggtaggc tagaggcata ggcttagaaa 59100

tgccaccatg gcettggggc cgtcctgtcc ccacagggtt gagaggcagg tctagttcgg 59160

gcccacctgg ccccgccctc cccgcctcag tatcctctgg cttgcctctc tgggcatcac 59220

accggggcag gtcctttgct ctcagctgct gctgctgctg tgaagtggag accggtgcca 59280

gtctttcctg ggtgggggct ctggagccct ccttacacat ggccccagta agggactgtg 59340

gtggtcagtc ttgggatact gcaccctggc agcctcagga gtgctcggcc tgtcctgcat 59400

gtgtccagca cctgctgctg aaagtggctc tggagggtcg ctgagagctt ctttgtagcg 59460

agacctgtca gtgtctgcgt cctggggctg tggtaccaaa tcaccacaca cgggtggccc 59520

agaacaacag acatggatta tctcacgttt ggggggcagt agtctgaaat gaaagtgtca 59580

gctggggctg ggtgcagtgt ctcatgcttg taatcccagc actttgggac tccgaggcag 59640

gcagattact tgaggtcagg agttcaagac cagcctagcc aacatggtga aaccgtctct 59700

actaaaaaaa tacaaaaatt agccaggcgt ggtggcacgt atctgtaatc ccagctaccc 59760

gggaggctga ggcaggagaa tcatttgaac tcgggaggag gaaggcggag gttaccgtga 59820

gccaagattg tgccactgca ctccagcctg ggtgacagag cgagactcca tgtcagaaag 59880

tgtcagctgg gccaggctcc ctcggaagcc tctggggtag gatgccccag gcgtccctcg 59940

ggttgtggct atatcactcc tctctctgcc tcatcgtcat gtgacagtct cctgggtgtg 60000

tctctgggcc ctgatttgcc tcttacagtt tctctgcaat tggacttagg gtccacccta 60060

atccaggatg acttcatttc catccttacc ttaattgtat ctgcaaagat cttatttcca 60120

aataaggtca cactctgagg ctccaggcag accctgcagg gtcctgtagg gcccatctag 60180

ccgacccgat tgtgtggaca gagcatgtgg ctccatgtgc ctcagccacc ctgcagcccc 60240

agcttgctta gctggatggt tcagctgctc agtgatttct gcaagcgcag cctctgcctg 60300

tggaccatgt aggtgcagtg gtctctggtt gcagggtctg ttgaatcctg tgggcgctgc 60360

accctgagac agtgccatgt gcatctctgt gcacagcggg aagcctccct tctgtctggg 60420

aatctgagtt ttccctctgc aagtgtgtag ctcccaggct cctgtgttgg aaactggaac 60480

atttcaaacc gttttgccag aaatgcatgg cgcacacaaa ggcattcttt atttaaacat 60540

taaatccact ctgtcagaaa ggtcgttctg aatagtccaa agagttaaca cccaaccagt 60600

ggtcccttga gcggctggga agagcctgtg ggcggggcgg gggctattgt ttaatgagat 60660

gttgtgtgct gtgcgccgcc cgccgggatg tttccgtagt tagtcaggcc tgctggaatg 60720

cgcaggctgc gctctgagtt tatctccacg gatctctatg catttctgga atgccaaaca 60780

acatctgcat ttcctgctgc catggtgatt ggcagcccat cccagaggac ggtgctggaa 60840

cccccaaggc tgggcacacc ctgggacgga gggatctcca gcacagcgac attctgattt 60900

ggaatttgtt atggactgtg acgtgcagat caagctgcac gtcaggagca catggaacgc 60960

tttggggtcc ctttttagcc ggggattcca gtgaatgaaa acggtagcag ggctcttttt 61020

gagcttggtc atggggcagc cctccgaggt cagcaatgtg cagaaggggt ttggagagaa 61080

ggcttgagca tgtgagggtg gcacggccag cctcatcagc agcggactcc tttcccaggg 61140

cagagtggag ggcagcaaac aggagaatga gcaacctacc taggtccttg ggatgcccct 61200

caggaggacg agctctaggg ggaatgctgt acgtcaccat gctcggtgcc agtgttgggg 61260

attttctaaa gggagatgcc cactcggagg gcaaagggtg atggcatagg ggctgcatat 61320

aaccccggac atggtgctga cgtggccccc ctgtgccttc cccaaggggc ggcctgagct 61380

gacacccaca gcgatcatgg ccgggaggct cctggtgcag cggctcttcg gcgggtcctc 61440

agatctgatg gactacgaca atgtgagttc tctagcagga cgccacgtgc agcctaggac 61500

aggctgagtt cgaggctagc tcttctgggg tgaggggcct ttgtgcgctg tgtgtctgca 61560

tgtagatgtg ggcatgcgtg tgtgtgcgct tatgtgggtg tgttgtacac acgtgtgtgt 61620

gcatgcatgg gtgtgtggct cagttctgag agtgtggcgc aggcgtgggc aggtgggtcg 61680

cctactgtgc ttgcagcctg ctctgggcgc atctggtcct ggtctgtgat cgctgctctt 61740

tgtgtcacat gcagtgggga acttcggttt tcggggccca agggccctcc ctgactccag 61800

acctgctttc aggttcccac gaccgtcttc accccactgg agtatggctg tgtggggctg 61860

tccgaggagg aggcagtggc tcgccacggg caggagcatg ttgaggtgag gcctgggagc 61920

agcacagctg aggacagtgg cgactccacg acctcacccc atgctctggg cggaggcctt 61980

gtgagcaggg tgccaaggtc tctcctttcc aggggccctg agcagtggct gtgtgtagcg 62040

ggaagggaca cgttgggggc agcctcagaa gtgggggtgc ctgggctttg gcagccttgg 62100

ggtgactggg cttaggtgcc tctggtctct gccaccatgt gtcacagcag tgaggggaag 62160

gcccttggct gccttgttct gagggcaagg agaagccctg tggcccagaa gcccccagcc 62220

ccaccccagc catgctgcag gggtgcccag caccagcagg gtcaccacca cggtgccacc 62280

cgctcccctg ctgaggtcag ctgagcactg gccccactcc agcacacagc agcttgtccc 62340

tgaaagcacc agggccccga agatgcctct gtgctgtccc caccttgcag ggccatgaat 62400

ctcactctac ccagaaaagt ttctcccagg ggcctccgac ctctgcttcc acccccaccc 62460

cccccaccac cctgcacctc gtcttcctgg actgcccagg gtttctccca ggcgagagcc 62520

ccccgccccc cgccccccac cacatcctga tacccatcct cttccagctt cttccaggct 62580

caggcactca cccttgagga aaggggtttt catcgagcaa gcctgctttg ggggtctgct 62640

ctggcaggtg gctgtggctc cggggtggcc tggagggatg cgggggctct gaagcctgcc 62700

gggccgtggt agctggaagc cttcggtgcg gtttcatctt gctgccgctg cagcttgcag 62760

gcacccagag aaaccggagc ctgcagcctg ggcggtgggg cctgcggcct ttggggatga 62820

gacactgggc tccagggctg gccccttccc cctgcacccc agaactctcc atcaacagga 62880

cgggcctgac aggccagcct tccccgaggc actttatttg ctatttttgt ttgattcatc 62940

acaagtgtgc tgggacatat gttcctggca ttttataatg ttgttttatg tttcacaatg 63000

ttgaatggcc aaatactctg tttgttttct tttcaattct gtcttagtta gaacaatatt 63060

ctctgaagtt ccattaatta ataatggctg tacagcacat gtgagggccc cactcatttt 63120

ttacttggcc gcagccccgg acacgctgtc gagcccttca ggggtgctga aagcctttgt 63180

ccaacagctc cggctccggt gtggccacag cggccttgct ccagtgaggg ccaagcaaac 63240

accagggtgc ctgagggagc ccaggtggcc tcctgctcac ccattcttgc ctccaccatt 63300

tgttgtgggt gagctacgtc cacccaggtt tgctgtgcct ggcctggctg cttggattgc 63360

ctgatgcctg ctctgctgct gtggtttacg gggtcccag gcctgggtgg gacagggtct 63420

gtcctgcaga gcatggctcc agccactggc tgcctgcacg tgagagggcc tgcacacacc 63480

ccgtggccag ccaagccctc atccccatcc cagaccagac cttcaggcca gatggatggt 63540

tcctgcccgc catgaccctg ccttgcccat gcagcctgtg ggagctgcag gtgccacgag 63600

gtttgtttgt cgcagcactg atccagggtt ggtatcctgt cctcaggtct atcacgccca 63660

ttataaacca ctggagttca cggtggctgg acgagatgca tcccagtgtt atgtaaaggt 63720

gagcatccct gtggcccagg gtgctgagga tgagagggag ggtggcaaag agcctggcag 63780

ggtgaacacc cgaggactgg ccccacccat gcctcccagg cagggtgcag ggtggatgca 63840

gggtggacgc aacccagccc cctcctgggc tggtggctga gatgtgcggc tttcagatgg 63900

tgtgcctgag ggagcccca cagctggtgc tgggcctgca tttccttggc cccaacgcag 63960

gcgaagttac tcaaggattt gctctgggga tcaagtaagt cccgaggaat gcaggctgcg 64020

atgcgatgtg cagctgggta tccctcgagt gccggcagtc ctacagtgga gagctgctgc 64080

cactcttgcg gcatttatgt ggccttcgtg ggtttgcagc agagattcct caggcccctc 64140

agacagggcg ggttttaggg ggacaagagg cggttctgcc ccagccagac gctgttgagc 64200

cagccagagc caggtgaggt gtcccctgtg gccccgggtc gctgctaggg cccttgccca 64260

cctggccaag caccacgcca ccatgagcag agtgccagta cctggagagc caccccaggg 64320

ctgctgtgtg tcttctggag ctggggccat tgcctgggtc ttgggctcag gctgtgtgtt 64380

ttcggggtcc cggggtcaaa ggaagccatg gcaaaggctc tgggtgggtt gacaggggta 64440

gttggaggtt gggacattgt gcatggcctg agagaggtgg ctggctggat gtggggcctg 64500

ggaggtggtg gtgtggtgag gccaggatgc gctgtctgtg ggtaaagcag agagcagagg 64560

cctaaggcag gactgtgcct agcaggggag gatggaacag caggaagcca ggccagggca 64620

agccagggcg tggctgggtg ggaggtgggc tggacgagca ctaggcatct gctgggaggg 64680

aggtggatgg cacccacggg tgtgggcagg ggtcctggct gctggctggg ggtcagggga 64740

cagtgaggag accagcagcc tctgaggccc tcctcttccc gggggtggga tgctgtggac 64800

ccccctccct gtgctgaccc cgcctgcctc ccccatcccc catgtgcagg tgtggggctt 64860

cctatgcgca ggtgatgcgg accgtgggta tccatcccac atgctctgag gaggtagtca 64920

agctgcgcat ctccaagcgc tcaggcctgg accccacggt gacaggctgc tgagggtaag 64980

cgccatccct gcaggccagg gcacacggtg cgcccgccgc cagctcctcg gaggccagac 65040

ccaggtatgc aggtggggct ggctctgttt gaggacaggg catgtggcag gggtgcagga 65100

gccctggcca tgggctccct ccaggtgtac agcaaggcta cacctgccac gccacccaga 65160

atgggtgctc catcctgtaa ccaggccaca gcggtgacag gcagaagggg aagttcccag 65220

gggcccaaac ctccccgggg gaccccagac cccggcctgg ccacagctgc tccccacaca 65280

caggaggtat caagaaacgg ggctcatcct tagcagccta tcccaggtgg atgttggcgt 65340

ggaaggtgtc ccgggtgggc taaagtcggg catctcaagt tgctgcccca caggggggctg 65400

cagtcgggaa gctggcctcc caccgaggcc tcccaccaag gcctggctcc tgcagagttc 65460

agcgcccgct catttcccta tctcccagaa ggtggagacg ctgccctgtg atggccggcc 65520

ctgcccagcc tgccctgcac atctggttct gttccagctc tgcagagcca ggctgaccgg 65580

ggggcatgtt gttcatcgtg acctcttctg gggaggaaga aactggcaag ggctcctggc 65640

ctctgcccca gggatgcctg tgccagagcc ccctcgcctc gtggtctaat agtgcatcct 65700

ccggcctggg ctgcagggca ggggccgggg cagggtgggc cctcagcccg tgccatccag 65760

cccacctggg gcacccccat ggctgtcagc ccctcccagg gttggggtgt ttgggctcca 65820

tctctccccc ggggctgagg tggagaccag gcagagcagt gggctccccc caagatgcct 65880

gtggacaggc tctagggtct gcatggcgcc gaggggctct ggggaggcct ctctgggggt 65940

actcagggcc ccctgcttca ccgtggccgc ctcccccegg cggggccgcg ctcgataggg 66000

ataaacaaag ggcatcctga ggaaactctt atcagaacat tacacctccc agagctgttt 66060

tgttaggagc ctgctataaa tttttatcat ttcaaaatat ttttgtagca ccgcgtcgct 66120

ccctgtgacg catcgccctg gggtggggcc attctctgct gggtttattt ctcacccatc 66180

tcccttgggg gtccctgggt gtggcagtgg gagacatagc taggctgatg tgaggggtgg 66240

gtggctgacc tgtgctgacc ttcctgttgt tggcaggatg gctgcaggcc aggtttgggg 66300

ggcctcaacc ctctcctgga gcgcctgtga gatggtcagc gtggagcgca agtgctggac 66360

gggtggcccg tgtgccccac agggatggct caggggactg tccacctcac ccctgcacct 66420

ttcagccttt gccgccgggc accccccca ggctcctggt gccggatgat gacgacctgg 66480

gtggaaacct accctgtggg cacccatgtc cgagccccct ggcatttctg caatgcaaat 66540

aaagagggta ctttttctga agtgtg 66566

## Claims

1. A protein comprising the amino acid sequence of SEQ ID NO: 2 or 4.

2. A protein comprising the amino acid sequence of SEQ ID NO: 2 or 4 in which one or more amino acids are replaced, deleted, added, and/or inserted, having homology of 60% or higher to the amino acid sequence of SEQ ID NO: 2 or 4, and having a thioredoxin reductase activity.

3. A protein having a thioredoxin reductase activity, encoded by a DNA which hybridizes to the DNA comprising the nucleotide sequence of SEQ ID NO: 1 or 3.

4. A protein comprising the amino acid sequence of SEQ ID NO: 2 or 4 in which one or more amino acids are replaced, deleted, added, and/or inserted and having an XIAP-binding activity.

**5.** Aprotein encoded by a DNA which hybridizes to the DNA comprising the nucleotide sequence of SEQ ID NO: 1 or 3, and having an XIAP-binding activity.

**6.** An antibody biding to the protein of any one of claims 1 to 5.

**7.** A cDNA encoding the protein of any one of claims 1 to 5.

**8.** A cDNA comprising a protein coding region of the nucleotide sequence of SEQ ID NO: 1 or 3.

**9.** A vector into which the DNA of claim 7 or 8 has been inserted.

**10.** A transformant carrying the vector of claim 9.

**11.** A method for producing the protein of any one of claims 1 to 5, the method containing culturing the transformant of claim 10.

**12.** An antisense DNA against all or a part of the cDNA of claim 7.

**13.** An oligonucleotide comprising a strand of at least 15 nucleotides and hybridizing to the cDNA of claim 7.

**14.** A DNA encoding a protein with a thioredoxin reductase activity and comprising the first exon or the second exon, and the third to the nineteenth exons below:

the first exon, SEQ ID NO: 18;
the second exon, SEQ ID NO: 19;
the third exon, SEQ ID NO: 20;
the forth exon, SEQ ID NO: 21;
the fifth exon, SEQ ID NO: 22;
the sixth exon, SEQ ID NO: 23;
the seventh exon, SEQ ID NO: 24;
the eighth exon, SEQ ID NO: 25;
the ninth exon, SEQ ID NO: 26;
the tenth exon, SEQ ID NO: 27;
the eleventh exon, SEQ ID NO: 28;
the twelfth exon, SEQ ID NO: 29;
the thirteenth exon, SEQ ID NO: 30;
the fourteenth exon, SEQ ID NO: 31;
the fifteenth exon, SEQ ID NO: 32;
the sixteenth exon, SEQ ID NO: 33;
the seventeenth exon, SEQ ID NO: 34;
the eighteenth exon, SEQ ID NO: 35; and
the nineteenth exon, SEQ ID NO: 36.

**15.** The DNA of claim 14, described by SEQ ID NO: 37.

**16.** A DNA hybridizing to the nucleotide sequence of any one of SEQ ID NOs: 18 to 36 or a part thereof, which can hybridize to human chromosome 22q11.2.

**17.** A DNA which can hybridize to all or a part of a portion of the nucleotide sequence of SEQ ID NO: 37, the portion non-overlapping with the nucleotide sequences of SEQ ID NOs: 18 to 36.

**18.** A method for screening a compound having an activity of inhibiting a binding of XIAP with the binding factor, the method comprising the steps of :

(a) contacting simultaneously a candidate substance as a subject for screening, and XIAP with the protein of claim 2, or
(a)' contacting a candidate substance as a subject for screening with XIAP, and then, further contacting with the protein of claim 2,

(b) determining the amount of the protein of claim 2 which binds and/or does not bind to XIAP, and
(c) selecting a compound which inhibits binding of XIAP with the protein of claim 2.

**19.** A method for screening a compound having an activity of promoting or inhibiting an enzyme activity of thioredoxin reductase II, the method comprising the steps of:

(a) contacting a candidate substance as a subject for screening with the protein of any one of claims 1 to 3,
(b) observing the change in a thioredoxin reductase activity of the protein of any one of claims 1 to 3, and
(c) selecting a compound which promotes or inhibits an enzyme activity of thioredoxin reductase II.

Figure 1

```
  1'  MAVALRGLGWRFRWRTQAVAGGVRGAARGAAAGQRDYDLLVVGGGSGGLACAKEAAQLGR
                                           ****...******** ****** *.
  1"                                       MNGPEDLPKSYDYDLIIIGGGSGGLAAAKEAAQYGK
                                                     FAD-binding region (ADP)

 61'  KVAVVDYVEPSPQGTRWGLGGTCVNVGCIPKKLMHQAALLGGLIQDAPNYGWKVAQPVPH
      ** *.*.*.*.* ************************************  .**..****.*...* *
 37"  KVMVLDFVTPTPLGTRWGLGGTCVNVGCIPKKLMHQAALLGQALQDSRNYGWKVEETVKH

                   Reduction active center
121'  DWRKMAEAVQNHVKSLNWGHRVQLQDRKVKYFNIKASFVDEHTVCGVAKGGKEILLSADH
      ** .* ******. *****.**.*...** * *   . *.. * . .... *** . **.
 97"  DWDRMIEAVQNHIGSLNWGYRVALREKKVVYENAYGQFIGPHRIKATNNKGKEKIYSAES

181'  IIIATGGRPRYPTHIEGALEYGITSDDIFWLKESPGKTLVVGASYVALECAGFLTGIGLD
      ..****.**** . * *. ** *.***.* *  .*******************.****.
157"  FLIATGERPRYLG-IPGDKEYCISSDDLFSLPYCPGKTLVVGASYVALECAGFLAGIGLG
                                                   NADPH-binding domain
241'  TTIMMRSIPLRGFDQQMSSMVIEHMASHGTRFLRGCAPSRVRRL---PDGQLQVTWEDST
      .*.*.*** ******.*.... ***.**..*.*  .* .* ..   . *.*.*. ....
216"  VTVMVRSILLRGFDQDMANKIGEHMEEHGIKFIRQFVPIKVEQIEAGTPGRLRVVAQSTN

298'  TGKEDTGTFDTVLWAIGRVPDTRSLNLEKAGVDTSPDTQKILVDSREATSVPHIYAIGDV
      ...  .*...**. **** . **...**..**... .* ** *.. *.*.**.******.
276"  SEEIIEGEYNTVMLAIGRDACTRKIGLETVGVKINEKTGKIPVTDEEQTNVPYIYAIGDI
                                                  FAD-binding region (Flavin)

358'  VEGRPELTPTAIMAGRLLVQRLFGGSSDLMDYDNVPTTVFTPLEYGCVGLSEEEAVARHG
      .*.. ****.** *****.***..**.   **.************* *****.**.. *
336"  LEDKVELTPVAIQAGRLLAQRLYAGSTVKCDYENVPTTVFTPLEYGACGLSEEKAVEKFG

418'  QEHVEVYHAHYKPLEFTVAGRDASQCYVKMVCLREPPTAGAGPAFSLAPTQGEVTQGFAL
      .*..****... ***.*...**...**.*..*   .  .  .*    *.*..********
396"  EENIEVYHSYFWPLEWTIPSRDNNKCYAKIICNTKDNERVVG-FHVLGPNAGEVTQGFAA

478'  GIKCGASYAQVMRTVGIHPTCSEEVVKLRISKRSGLDPTVTGCSeCysG
      ..*** .  *. .*.****.*.* ..*...**** .  .** * *
455"  ALKCGLTKKQLDSTIGIHPVCAEVFTTLSVTKRSGASILQAGCSeCysG
```

Figure 2

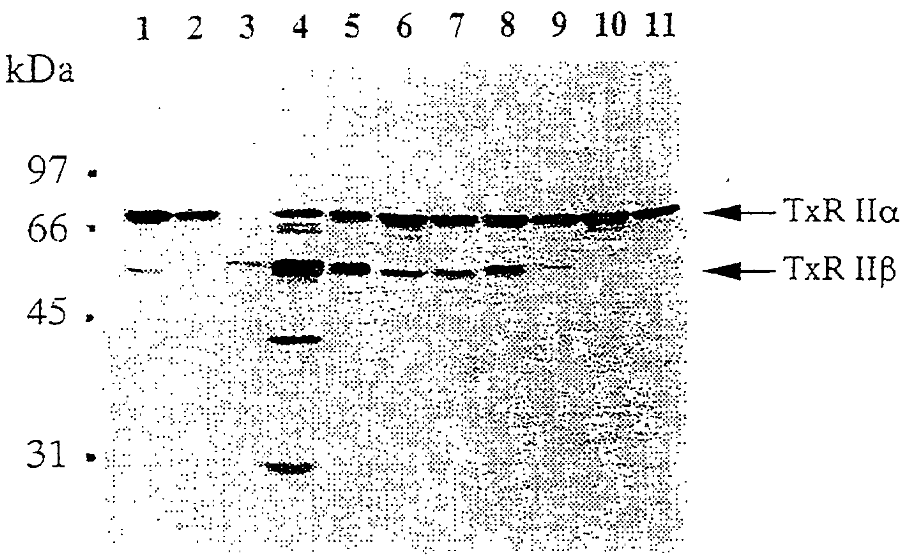


| | |
|---|---|
| 1 | Raji |
| 2 | Jurkat |
| 3 | HL60 |
| 4 | U937 |
| 5 | ZR75-1 |
| 6 | HepG 2 |
| 7 | HeLa |
| 8 | A431 |
| 9 | MRC 5 |
| 10 | NIH3T3 |
| 11 | Rat-1 |

Figure 3

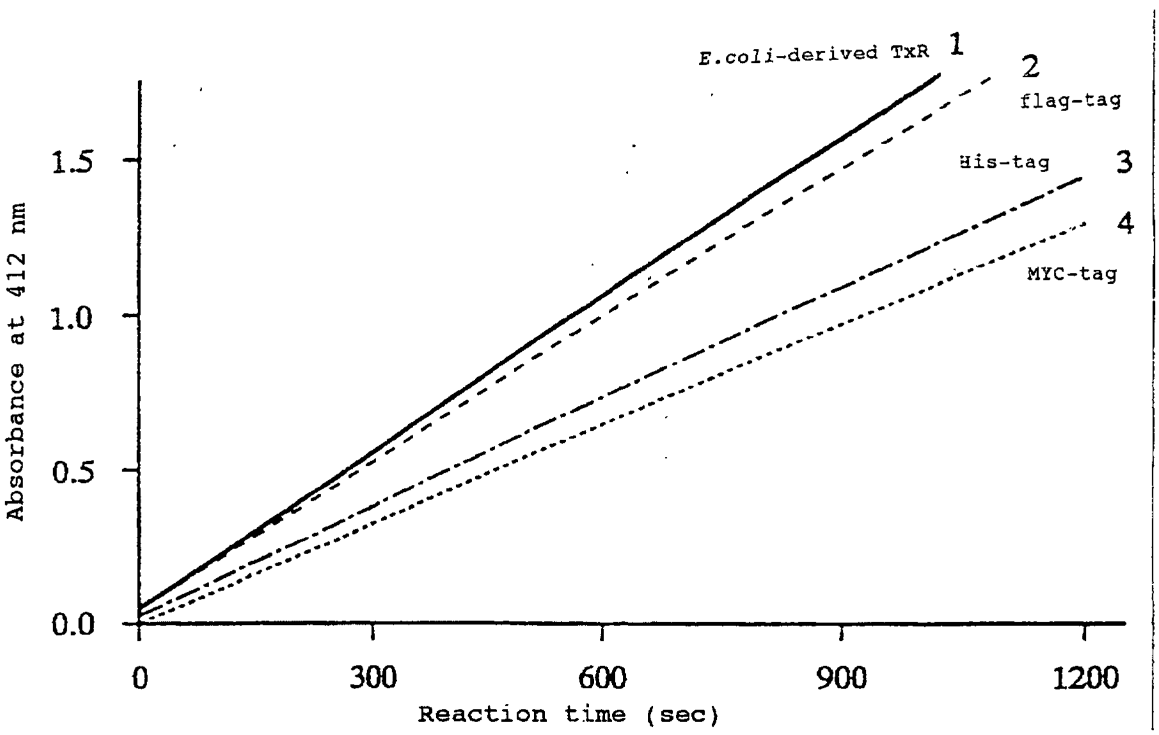
E.coli-derived TxR 1
2
flag-tag
His-tag
3
4
MYC-tag
1.5
1.0
0.5
0.0
Absorbance at 412 nm
0
300
600
900
1200
Reaction time (sec)

Figure 4

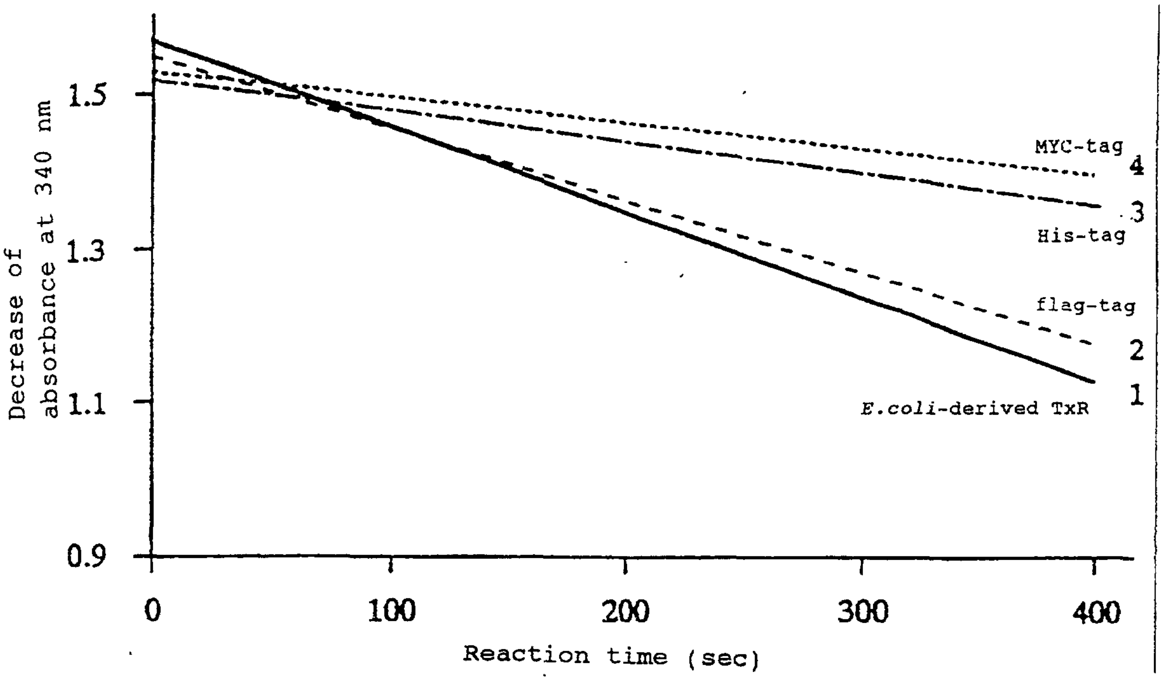
1.5
1.3
1.1
0.9
Decrease of absorbance at 340 nm
0
100
200
300
400
Reaction time (sec)
MYC-tag
4
3
His-tag
flag-tag
2
1
E.coli-derived TxR

Figure 5

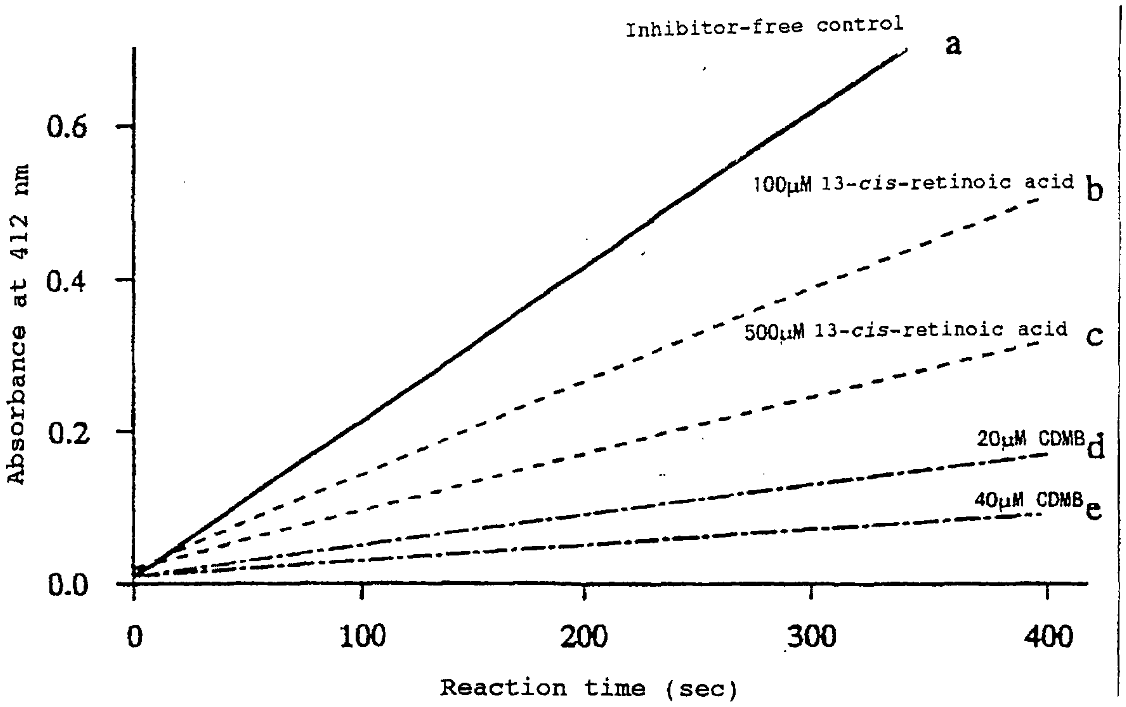
Inhibitor-free control
a
100μM 13-cis-retinoic acid
b
500μM 13-cis-retinoic acid
c
20μM CDMB
d
40μM CDMB
e
Absorbance at 412 nm
0.0
0.2
0.4
0.6
0
100
200
300
400
Reaction time (sec)

## INTERNATIONAL SEARCH REPORT

International application No.
PCT/JP99/05983

A. CLASSIFICATION OF SUBJECT MATTER
Int.Cl[7] C12N15/53, 9/02, 1/21, 1/19, 1/15, 5/10,
C07K16/40, C12Q1/26, 1/68,
G01N33/50, 33/15, 33/566
According to International Patent Classification (IPC) or to both national classification and IPC

B. FIELDS SEARCHED
Minimum documentation searched (classification system followed by classification symbols)
Int.Cl[7] C12N15/00-15/90

Documentation searched other than minimum documentation to the extent that such documents are included in the fields searched

Electronic data base consulted during the international search (name of data base and, where practicable, search terms used)
GENBANK/EMBL/DDBJ/GENESEQ
PIR/SWISSPROT/GENESEQ
BIOSIS/WPI

C. DOCUMENTS CONSIDERED TO BE RELEVANT

| Category* | Citation of document, with indication, where appropriate, of the relevant passages | Relevant to claim No. |
|---|---|---|
| P,X<br>P,A | J. Biol. Chem, 274(35), August 27 1999<br>Qui-AnSun et al., "Redox regulation of cell signaling by selenocysteine in mammalian thioredoxin reductase", p.24522-24530 | 1-17,19<br>18 |
| P,X<br>P,A | Eur. J. Biochem., 261(2), April 1999<br>Antonio Miranda-Vizuete et al., "Human mitochondorial thioredoxin reductase. cDNA cloning, expression and genomic organization", p.405-412 | 1-17,19<br>18 |
| P,X<br>P,A | FEBS Letters, 442(1), Jan. 8 1999<br>Pamela Y. et al., "Cloning,sequencing and functional expression of a novel human thioredoxin reductase", p.105-111 | 1-17,19<br>18 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>25 January, 2000 (25.01.00) | Date of mailing of the international search report<br>08 February, 2000 (08.02.00) |
| Name and mailing address of the ISA/<br>Japanese Patent Office<br>Facsimile No. | Authorized officer<br><br>Telephone No. |

Form PCT/ISA/210 (second sheet) (July 1992)

**INTERNATIONAL SEARCH REPORT**

International application No.
PCT/JP99/05983

C (Continuation). DOCUMENTS CONSIDERED TO BE RELEVANT

| Category* | Citation of document, with indication, where appropriate, of the relevant passages | Relevant to claim No. |
|---|---|---|
| P,X<br>P,A | Database Medline on PubMed, US National Library of Medicine (Bethesda, MD, USA), No. 10462449, Gorlatov SN., et al., "Human selenium-dependent thioredoxin reductase from HeLa cells: properties of forms with differingheparin affinities", Abstract, Arch. Biochem. Biophys, 369(1), p. 133-142, Sep.1 1999 | 1-17,19<br>18 |
| A | Proc. Natl. Acad. Sci. USA., 93(3), Feb.6 1996 Takashi Tamura et al., "A new selenoprotein from human lung adenocarcinoma cells: Purification, properties, and thioredoxinreductase activity", p. 1006-1011 | 1-19 |
| A | Proc. Natl. Acad. Sci. USA, 93(12), June 11 1996 Vadim N. Gladyshev et al., "Selenocysteine, identified as the penultimate C-terminal residue in human T-cell thioredoxin reductase, corresponds to TGA in the human placental gene", p. 6146-6151 | 1-19 |
| A | FEBS Letters, 373(1), Oct. 2 1995 Pamela Y. Gasdaska et al., "Cloning and sequencing of a human thioredoxin reductase", p. 5-9 | 1-19 |
| A | J. Biol. Chem, 273(15), Apr. 10 1998 Liangwei Zhong et al., "Rat and calf thioredoxin reductase are homologous to glutathione reductase with a carboxyll-terminal elongation containing a conserved catalytically active penultimate selenocysteine residue", p. 8581-8591 | 1-19 |
| A | GENOMICS, 37(2), Oct. 15, 1996 John R. Gasdaska et al., " thioredoxin reductase gene localization to chromosomal position 12q23-q24.1 and mRNA distribution in human tissue", p. 257-259 | 1-19 |

Form PCT/ISA/210 (continuation of second sheet) (July 1992)